# EUROPEAN PATENT APPLICATION

(11) **EP 2 305 797 A2**
(43) Date of publication of application: **06.04.2011**
(21) Application number: 10011413.1
(22) Date of filing: 22.01.2001
(51) Int. Cl.: C12N 9/02, C12N 15/82, A01H 5/10

(54) **Low-lipoxygenase 1 barley**

(30) Priority: 29.12.2000 US 751687; 29.12.2000 WO PCT/IB00/02045
(62) Divisional of application: 01902597.2
(71) Applicant: Carlsberg Research Laboratory, 2500 Copenhagen (DK); HEINEKEN TECHNICAL SERVICES B.V., 2380 BB Zoeterwoude (NL); Brasseries KRONENBOURG Société dite : S.A., 67037 Strasbourg Cedex 2 (FR)
(72) Inventor: Douma, Anna Christiana, 3707 SB Zeist (NL); Doderer, Albert, 2380 BB Zoeterwoude (NL); Cameron-Mills, Varena, 2500 Valby (DK); Skadhauge, Brigitte, 3460 Birkeroed (DK); Bech, Lene Molskov, 2765 Smorum (DK); Schmitt, Nathalie, 2584 ET Den Haag (NL); Heistek, Jolanda Carolina, 3132 VA Vlaardingen (NL); Van Mechelen, Joannes Reinier, 1013 HD Amsterdam (NL)
(74) Representative: Williams, Aylsa

(57) **Abstract**

Barley plants having reduced lipoxygenase-1 enzyme activity are provided, for example, barley plants expressing mutant LOX-1 protein. The barley plants of the invention are useful in the production of plant products such as malt and brewed beverages, particularly beer, having increased stability and reduced T2N potential.

SEQUENCE LISTING

<110> Douma, Anneke
      Doderer, Albert
      Cameron-Mills, Verena
      Skadhauge, Birgitte
      Bech, Lene

<120> LOW LIPOXYGENASE 1 BARLEY

<130> 11225.12WO01

<160> 18

<170> PatentIn version 3.0

<210> 1
<211> 143
<212> DNA
<213> Hordeum vulgare

<400> 1
ctaagccatc ggcaaccatg gatgaataaa gggcgttcgc cacgtacgaa acttgtcgag 60
agattggtgt agtgtgtgtc tgtgacagta ctatgtcagc agttgctctt taagccgaat 120
aaataaagca gatttgcttc caa 143

<210> 2
<211> 24
<212> DNA
<213> Hordeum vulgare

<400> 2
gaaaagcttg gaggtagacg ctgc 24

<210> 3

<211> 33

<212> DNA

<213> Hordeum vulgare

<400> 3
tataggatcc ttgttcttgg cctcctctcc tcg 33

<210> 4

<211> 22

<212> DNA

<213> Hordeum vulgare

<400> 4
agtgaaaaac agtgtgctgg tg 22

<210> 5

<211> 21

<212> DNA

<213> Hordeum vulgare

<400> 5
ggcttaaaga gcaactgctg a 21

<210> 6

<211> 23

<212> DNA

<213> Hordeum vulgare

<400> 6
caagatgcat atgctgctgg gag 23

<210> 7

<211> 29

<212> DNA

<213> Hordeum vulgare

<400>  7
cgatggttta aattagatgg agatgctgt                                          29

<210>  8

<211>  4663

<212>  DNA

<213>  Hordeum vulgare

<400>  8
cagccccatg catgcacatg cacatgcaca tgcacatgca gtgcagccaa gcaccgctcg      60

atgggcgatc acccgtcacg ggaccggagc gcgccatgcg aagcacgagg agggcacgtc     120

accgtccgcg cgcagcacgt ggagagcacg tcgccgtccg atccatctct ccaaagccga     180

gcgccacacc accgggaccg gacccggacc ggcctataaa ttgcccggac cgagctgcaa     240

gcagctcctc acacacactc acgcaacaca catccatctt cactgaaaag tgaaaaacag     300

tgtgctggtg ccattggttg gagcagtgaa agcgaggaga ggaggccaag aacaagatgc     360

tgctgggagg gctgatcgac accctcacgg gggcgaacaa gagcgcccgg ctcaagggca     420

cggtggtgct catgcgcaag aacgtgctgg acctcaacga cttcggcgcc accatcatcg     480

acggcatcgg cgagttcctc ggcaagggcg tcacctgcca gcttatcagc tccaccgccg     540

tcgaccaagg taatcactac cctcctccgg ccttctcctc tgtttacaag atatagtatt     600

tctttcgtgt gggccggcgg ccatggatgg atggatgtgt ctggatcggc taaagaagat     660

aggatagcta gccctggccg gtcgtcttta cctgagcatg ggcatatgcc atcgaaaaaa     720

gagacaacag catgcatgca tggtgcgcgc accagaccac gcagagcacc ggatgctcga     780

gacaaagcaa cacaacaagc aaggacgaca cgtcaaaagc aacacaacaa gcaaggacgg     840

cacgtcaaaa gcaacacaaa cctaaactaa agcacaaaga cgtaagagca agcacacaat     900

cagcaggcta taaacagttg tcatcaaaaa caacgctgga agagagagag aaggaaggaa     960

gtagtagcca tgaaaaatta aatcaccggg cgttgctctt tgcccaacaa ttaatcaagc    1020

agggtacgtg gcatgtatag ttcttgtaag taaactaagc atgtgatatg agaaggtacg    1080

tggtggtgca gacaacggcg gtcgcgggaa ggtgggcgcg gaggcggagc tggagcagtg    1140

ggtgacgagc ctgccgtcgc tgacgacggg ggagtccaag ttcggcctca ccttcgactg    1200

ggaggtggag aagctcgggg tgccgggcgc catcgtcgtc aacaactacc acagctccga    1260

gttcctgctt aaaaccatca ccctccacga cgtccccggc cgcagcggca acctcacctt    1320

cgtcgccaac tcatggatct accccgccgc caactaccga tacagccgcg tcttcttcgc    1380

caacgacacg cgtggatttt cctctacttt cctctccttt cattttcacc gccttcgtca    1440

ttcatggtcg atcattaagt cttgccagga caatagatga tgagctagga gtggttacca 1500
cttagcagta cgtacattat ttattccgtg ttggtagaaa aggatatggt ttggtgcaga 1560
tcgacacaag attgaatgaa agttgcaccg tggcaccgtg gcagcgtggt aggtgaaaat 1620
aactgttgca cggatccacc cacatgattg ttttcatgaa taaacttttt aaggatgtgt 1680
ctagccacat ctagatgcat gtcacataat tattgcatac caaaacgatt aaattaagca 1740
taaaaagaaa aggaaaaaaa tactcacata tctcgacgta agatcaatga tatagtattt 1800
agatatgcaa tatttatctt acatctaaac ctttcttcat tcttaaatat aagacatttg 1860
taagatttca ctatggacaa catacgaaac aaaatcagtg gatctctcta tgcattcatt 1920
atgtagtcta taataaaatc tttaaaagat cgtatatttt gcaacggagg gagtaaaaca 1980
taacttttta atagtaatgt tgcacggctc cacactcgca gacgtacctg ccgagccaga 2040
tgccggcggc gctgaagccg taccgcgacg acgagctccg gaacctgcgt ggcgacgacc 2100
agcagggccc gtaccaggag cacgaccgca tctaccgcta cgacgtctac aacgacctcg 2160
gcgagggccg ccccatcctc ggcggcaact ccgaccaccc ttacccgcgc cgcggccgca 2220
cggagcgcaa gcccaacgcc agcgacccga gcctggagag ccggctgtcg ctgctggagc 2280
agatctacgt gccgcgggac gagaagttcg gccacctcaa gacgtccgac ttcctgggct 2340
actccatcaa ggccatcacg cagggcatcc tgccggccgt gcgcacctac gtggacacca 2400
cccccggcga gttcgactcc ttccaggaca tcatcaacct ctatgagggc ggcatcaagc 2460
tgcccaaggt ggccgccctg gaggagctcc gtaagcagtt cccgctccag ctcatcaagg 2520
acctcctccc cgtcggcggc gactccctgc ttaagctccc cgtgccccac atcatccagg 2580
agaacaagca ggcgtggagg accgacgagg agttcgcacg ggaggtgctc gccggcgtca 2640
acccggtcat gatcacgcgt ctcacggtga gtcagcgatt atttgttcat tgtgtgtgta 2700
tggtgtccat ggtgagaaag tgcagatctt gatttgcgtt gggtcgcatg cacgcatgct 2760
gcatgcatgc aggagttccc gccaaaaagt agtctggacc ctagcaagtt tggtgaccac 2820
accagcacca tcacggcgga gcacatagag aagaacctcg agggcctcac ggtgcagcag 2880
gtaattggtc caagccatcg acatcaacta tgatttacct aggagtaatt ggtagctgta 2940
gataatttgg cttcgttgca attaatttga tgctggccga tcaagtgatc gtattgggtt 3000
tgaaatttgc aggcgctgga aagcaacagg ctgtacatcc ttgatcacca tgaccggttc 3060
atgccgttcc tgatcgacgt caacaacctg cccggcaact tcatctacgc cacgaggacc 3120
ctcttcttcc tgcgcggcga cggcaggctc acgccgctcg ccatcgagct gagcgagccc 3180
atcatccagg gcggccttac cacggccaag agcaaggttt acacgccggt gcccagcggc 3240
tccgtcgaag gctgggtgtg ggagctcgcc aaggcctacg tcgccgtcaa tgactccggg 3300
tggcaccagc tcgtcagcca ctggtacgtt ctccacggtc gatgtgattc agtcagtcga 3360

tgcacaacaa ctgatcgaaa tatgattgat tgaaacgcgc aggctgaaca ctcacgcggt 3420
gatggagccg ttcgtgatct cgacgaaccg gcaccttagc gtgacgcacc cggtgcacaa 3480
gctgctgagc ccgcactacc gcgacaccat gaccatcaac gcgctggcgc ggcagacgct 3540
catcaacgcc ggcggcatct tcgagatgac ggtgttcccg ggcaagttcg cgttggggat 3600
gtcggccgtg gtgtacaagg actggaagtt caccgagcag ggactgccgg acgatctcat 3660
caagaggtac gtacctggta aatgttatga atgtgtaaaa caaattgggc gtctcgctca 3720
ctgacaggaa cgtggtaaaa aaaatgcagg ggcatggcgg tggaggaccc gtcgagcccg 3780
tacaaggtgc ggttgctggt gtcggactac ccgtacgcgg cggacgggct ggcgatctgg 3840
cacgccattg agcagtacgt gagcgagtac ctggccatct actacccgaa cgacggcgtg 3900
ctgcagggcg atacggaggt gcaggcgtgg tggaaggaga cgcgcgaggt cgggcacggc 3960
gacctcaagg acgccccatg gtggcccaag atgcaaagtg tgccggagct ggccaaggcg 4020
tgcaccacca tcatctggat cgggtcggcg ctgcatgcgg cagtcaactt cgggcagtac 4080
ccctacgcgg ggttcctccc gaaccggccg acggtgagcc ggcgccgcat gccggagccc 4140
ggcacggagg agtacgcgga gctggagcgc gacccggagc gggccttcat ccacaccatc 4200
acgagccaga tccagaccat catcggcgtg tcgctgctgg aggtgctgtc gaagcactcc 4260
tccgacgagc tgtacctcgg gcagcgggac acgccggagt ggacctcgga cccaaaggcc 4320
ctggaggtgt tcaagcggtt cagcgaccgg ctggtggaga tcgagagcaa ggtggtgggc 4380
atgaaccatg acccggagct caagaaccgc aacggcccgg ctaagtttcc ctacatgctg 4440
ctctacccca acacctccga ccacaagggc gccgctgccg ggcttaccgc caagggcatc 4500
cccaacagca tctccatcta atctaagcca tcggcaacca tggatgaata aagggcgttc 4560
gccacgtacg aaacttgtcg agagattggt gtagtgtgtg tctgtgacag tactatgtca 4620
gcagttgctc tttaagccga ataaataaag cagatttgct tcc 4663

<210> 9

<211> 862

<212> PRT

<213> Hordeum vulgare


<400> 9
   Met Leu Leu Gly Gly Leu Ile Asp Thr Leu Thr Gly Ala Asn Lys Ser
   1 5 10 15
   Ala Arg Leu Lys Gly Thr Val Val Leu Met Arg Lys Asn Val Leu Asp
   20 25 30
   Leu Asn Asp Phe Gly Ala Thr Ile Ile Asp Gly Ile Gly Glu Phe Leu
           35                  40                  45
   Gly Lys Gly Val Thr Cys Gln Leu Ile Ser Ser Thr Ala Val Asp Gln
       50                  55                  60
   Asp Asn Gly Gly Arg Gly Lys Val Gly Ala Glu Ala Glu Leu Glu Gln
   65                  70                  75                  80
   Trp Val Thr Ser Leu Pro Ser Leu Thr Thr Gly Glu Ser Lys Phe Gly
                   85                  90                  95
   Leu Thr Phe Asp Trp Glu Val Glu Lys Leu Gly Val Pro Gly Ala Ile
               100                 105                 110
   Val Val Asn Asn Tyr His Ser Ser Glu Phe Leu Leu Lys Thr Ile Thr
           115                 120                 125
   Leu His Asp Val Pro Gly Arg Ser Gly Asn Leu Thr Phe Val Ala Asn
       130                 135                 140
   Ser Trp Ile Tyr Pro Ala Ala Asn Tyr Arg Tyr Ser Arg Val Phe Phe
   145                 150                 155                 160
   Ala Asn Asp Thr Tyr Leu Pro Ser Gln Met Pro Ala Ala Leu Lys Pro
                   165                 170                 175
   Tyr Arg Asp Asp Glu Leu Arg Asn Leu Arg Gly Asp Asp Gln Gln Gly
               180                 185                 190
   Pro Tyr Gln Glu His Asp Arg Ile Tyr Arg Tyr Asp Val Tyr Asn Asp
           195                 200                 205
   Leu Gly Glu Gly Arg Pro Ile Leu Gly Gly Asn Ser Asp His Pro Tyr
       210                 215                 220
   Pro Arg Arg Gly Arg Thr Glu Arg Lys Pro Asn Ala Ser Asp Pro Ser
   225                 230                 235                 240
   Leu Glu Ser Arg Leu Ser Leu Leu Glu Gln Ile Tyr Val Pro Arg Asp
                   245                 250                 255
   Glu Lys Phe Gly His Leu Lys Thr Ser Asp Phe Leu Gly Tyr Ser Ile
               260                 265                 270
   Lys Ala Ile Thr Gln Gly Ile Leu Pro Ala Val Arg Thr Tyr Val Asp
           275                 280                 285
   Thr Thr Pro Gly Glu Phe Asp Ser Phe Gln Asp Ile Ile Asn Leu Tyr
       290                 295                 300
   Glu Gly Gly Ile Lys Leu Pro Lys Val Ala Ala Leu Glu Glu Leu Arg
   305                 310                 315                 320
   Lys Gln Phe Pro Leu Gln Leu Ile Lys Asp Leu Leu Pro Val Gly Gly
                   325                 330                 335
   Asp Ser Leu Leu Lys Leu Pro Val Pro His Ile Ile Gln Glu Asn Lys
               340                 345                 350
   Gln Ala Trp Arg Thr Asp Glu Glu Phe Ala Arg Glu Val Leu Ala Gly
           355                 360                 365
   Val Asn Pro Val Met Ile Thr Arg Leu Thr Glu Phe Pro Pro Lys Ser
   370                 375                 380
   Ser Leu Asp Pro Ser Lys Phe Gly Asp His Thr Ser Thr Ile Thr Ala
   385                 390                 395                 400
   Glu His Ile Glu Lys Asn Leu Glu Gly Leu Thr Val Gln Gln Ala Leu
                   405                 410                 415
   Glu Ser Asn Arg Leu Tyr Ile Leu Asp His His Asp Arg Phe Met Pro
               420                 425                 430
   Phe Leu Ile Asp Val Asn Asn Leu Pro Gly Asn Phe Ile Tyr Ala Thr
           435                 440                 445
   Arg Thr Leu Phe Phe Leu Arg Gly Asp Gly Arg Leu Thr Pro Leu Ala
       450                 455                 460
   Ile Glu Leu Ser Glu Pro Ile Ile Gln Gly Gly Leu Thr Thr Ala Lys
   465                 470                 475                 480
   Ser Lys Val Tyr Thr Pro Val Pro Ser Gly Ser Val Glu Gly Trp Val
                   485                 490                 495
   Trp Glu Leu Ala Lys Ala Tyr Val Ala Val Asn Asp Ser Gly Trp His
               500                 505                 510
   Gln Leu Val Ser His Trp Leu Asn Thr His Ala Val Met Glu Pro Phe
           515                 520                 525
   Val Ile Ser Thr Asn Arg His Leu Ser Val Thr His Pro Val His Lys
       530                 535                 540
   Leu Leu Ser Pro His Tyr Arg Asp Thr Met Thr Ile Asn Ala Leu Ala
   545                 550                 555                 560
   Arg Gln Thr Leu Ile Asn Ala Gly Gly Ile Phe Glu Met Thr Val Phe
                   565                 570                 575
   Pro Gly Lys Phe Ala Leu Gly Met Ser Ala Val Val Tyr Lys Asp Trp
               580                 585                 590
   Lys Phe Thr Glu Gln Gly Leu Pro Asp Asp Leu Ile Lys Arg Gly Met
           595                 600                 605
   Ala Val Glu Asp Pro Ser Ser Pro Tyr Lys Val Arg Leu Leu Val Ser
       610                 615                 620
   Asp Tyr Pro Tyr Ala Ala Asp Gly Leu Ala Ile Trp His Ala Ile Glu
   625                 630                 635                 640
   Gln Tyr Val Ser Glu Tyr Leu Ala Ile Tyr Tyr Pro Asn Asp Gly Val
                   645                 650                 655
   Leu Gln Gly Asp Thr Glu Val Gln Ala Trp Trp Lys Glu Thr Arg Glu
               660                 665                 670
   Val Gly His Gly Asp Leu Lys Asp Ala Pro Trp Trp Pro Lys Met Gln
           675                 680                 685
   Ser Val Pro Glu Leu Ala Lys Ala Cys Thr Thr Ile Ile Trp Ile Gly
       690                 695                 700
   Ser Ala Leu His Ala Ala Val Asn Phe Gly Gln Tyr Pro Tyr Ala Gly
   705                 710                 715                 720
   Phe Leu Pro Asn Arg Pro Thr Val Ser Arg Arg Arg Met Pro Glu Pro
                   725                     730                     735
   Gly Thr Glu Glu Tyr Ala Glu Leu Glu Arg Asp Pro Glu Arg Ala Phe
               740                     745                     750
   Ile His Thr Ile Thr Ser Gln Ile Gln Thr Ile Ile Gly Val Ser Leu
           755                     760                     765
   Leu Glu Val Leu Ser Lys His Ser Ser Asp Glu Leu Tyr Leu Gly Gln
       770                     775                     780
   Arg Asp Thr Pro Glu Trp Thr Ser Asp Pro Lys Ala Leu Glu Val Phe
   785                     790                     795                     800
   Lys Arg Phe Ser Asp Arg Leu Val Glu Ile Glu Ser Lys Val Val Gly
                   805                     810                     815
   Met Asn His Asp Pro Glu Leu Lys Asn Arg Asn Gly Pro Ala Lys Phe
               820                     825                     830
   Pro Tyr Met Leu Leu Tyr Pro Asn Thr Ser Asp His Lys Gly Ala Ala
           835                     840                     845
   Ala Gly Leu Thr Ala Lys Gly Ile Pro Asn Ser Ile Ser Ile
       850                     855                     860

<210>  10

<211>  2818

<212>  DNA

<213>  Hordeum vulgare


<400>  10
agtgaaaaac agtgtgctgg tgccattggt tggagcagtg aaagcgagga gaggaggcca      60

agaacaagat gctgctggga gggctgatcg acaccctcac gggggcgaac aagagcgccc     120

ggctcaaggg cacggtggtg ctcatgcgca agaacgtgct ggacctcaac gacttcggcg     180

ccaccatcat cgacggcatc ggcgagttcc tcggcaaggg cgtcacctgc cagcttatca     240

gctccaccgc cgtcgaccaa gacaacggcg gtcgcgggaa ggtgggcgcg gaggcggagc     300

tggagcagtg ggtgacgagc ctgccgtcgc tgacgacggg ggagtccaag ttcggcctca     360

ccttcgactg ggaggtggag aagctcgggg tgccgggcgc catcgtcgtc aacaactacc     420

acagctccga gttcctgctt aaaaccatca ccctccacga cgtccccggc cgcagcggca     480

acctcacctt cgtcgccaac tcatggatct accccgccgc caactaccga tacagccgcg     540

tcttcttcgc caacgacacg tacctgccga gccagatgcc ggcggcgctg aagccgtacc     600

gcgacgacga gctccggaac ctgcgtggcg acgaccagca gggcccgtac caggagcacg     660

accgcatcta ccgctacgac gtctacaacg acctcggcga gggccgcccc atcctcggcg     720

gcaactccga ccacccttac ccgcgccgcg gccgcacgga gcgcaagccc aacgccagcg     780

acccgagcct ggagagccgg ctgtcgctgc tggagcagat ctacgtgccg cgggacgaga 840
agttcggcca cctcaagacg tccgacttcc tgggctactc catcaaggcc atcacgcagg 900
gcatcctgcc ggccgtgcgc acctacgtgg acaccacccc cggcgagttc gactccttcc 960
aggacatcat caacctctat gagggcggca tcaagctgcc caaggtggcc gccctggagg 1020
agctccgtaa gcagttcccg ctccagctca tcaaggacct cctccccgtc ggcggcgact 1080
ccctgcttaa gctccccgtg ccccacatca tccaggagaa caagcaggcg tggaggaccg 1140
acgaggagtt cgcacgggag gtgctcgccg gcgtcaaccc ggtcatgatc acgcgtctca 1200
cggagttccc gccaaaaagt agtctggacc ctagcaagtt tggtgaccac accagcacca 1260
tcacggcgga gcacatagag aagaacctcg agggcctcac ggtgcagcag gcgctggaaa 1320
gcaacaggct gtacatcctt gatcaccatg accggttcat gccgttcctg atcgacgtca 1380
acaacctgcc cggcaacttc atctacgcca cgaggaccct cttcttcctg cgcggcgacg 1440
gcaggctcac gccgctcgcc atcgagctga gcgagcccat catccagggc ggccttacca 1500
cggccaagag caaggtttac acgccggtgc ccagcggctc cgtcgaaggc tgggtgtggg 1560
agctcgccaa ggcctacgtc gccgtcaatg actccgggtg gcaccagctc gtcagccact 1620
ggctgaacac tcacgcggtg atggagccgt tcgtgatctc gacgaaccgg caccttagcg 1680
tgacgcaccc ggtgcacaag ctgctgagcc cgcactaccg cgacaccatg accatcaacg 1740
cgctggcgcg gcagacgctc atcaacgccg gcggcatctt cgagatgacg gtgttcccgg 1800
gcaagttcgc gttggggatg tcggccgtgg tgtacaagga ctggaagttc accgagcagg 1860
gactgccgga cgatctcatc aagaggggca tggcggtgga ggacccgtcg agcccgtaca 1920
aggtgcggtt gctggtgtcg gactacccgt acgcggcgga cgggctggcg atctggcacg 1980
ccattgagca gtacgtgagc gagtacctgg ccatctacta cccgaacgac ggcgtgctgc 2040
agggcgatac ggaggtgcag gcgtggtgga aggagacgcg cgaggtcggg cacggcgacc 2100
tcaaggacgc cccatggtgg cccaagatgc aaagtgtgcc ggagctggcc aaggcgtgca 2160
ccaccatcat ctggatcggg tcggcgctgc atgcggcagt caacttcggg cagtacccct 2220
acgcggggtt cctcccgaac cggccgacgg tgagccggcg ccgcatgccg gagcccggca 2280
cggaggagta cgcggagctg gagcgcgacc cggagcgggc cttcatccac accatcacga 2340
gccagatcca gaccatcatc ggcgtgtcgc tgctggaggt gctgtcgaag cactcctccg 2400
acgagctgta cctcgggcag cgggacacgc cggagtggac ctcggaccca aaggccctgg 2460
aggtgttcaa gcggttcagc gaccggctgg tggagatcga gagcaaggtg gtgggcatga 2520
accatgaccc ggagctcaag aaccgcaacg gcccggctaa gtttccctac atgctgctct 2580
accccaacac ctccgaccac aagggcgccg ctgccgggct taccgccaag ggcatcccca 2640
acagcatctc catctaatct aagccatcgg caaccatgga tgaataaagg gcgttcgcca 2700

cgtacgaaac ttgtcgagag attggtgtag tgtgtgtctg tgacagtact atgtcagcag 2760
ttgctcttta agccgaataa ataaagcaga tttgcttcca aaaaaaaaaa aaaaaaaa 2818

<210> 11
<211> 4663
<212> DNA
<213> Hordeum vulgare

<220>
<221> variation
<222> (2346)..(2348)
<223> "n" is a, c, t, or g encoding an acidic, basic, or polar amino ac
i

<400> 11
   cagccccatg catgcacatg cacatgcaca tgcacatgca gtgcagccaa gcaccgctcg 60
   atgggcgatc acccgtcacg ggaccggagc gcgccatgcg aagcacgagg agggcacgtc 120
   accgtccgcg cgcagcacgt ggagagcacg tcgccgtccg atccatctct ccaaagccga 180
   gcgccacacc accgggaccg gacccggacc ggcctataaa ttgcccggac cgagctgcaa 240
   gcagctcctc acacacactc acgcaacaca catccatctt cactgaaaag tgaaaaacag 300
   tgtgctggtg ccattggttg gagcagtgaa agcgaggaga ggaggccaag aacaagatgc 360
   tgctgggagg gctgatcgac accctcacgg gggcgaacaa gagcgcccgg ctcaagggca 420
   cggtggtgct catgcgcaag aacgtgctgg acctcaacga cttcggcgcc accatcatcg 480
   acggcatcgg cgagttcctc ggcaagggtg tcacctgcca gcttatcagc tccaccgccg 540
   tcgaccaagg taatcactac cctcctccgg ccttctcctc tgtttacaag atatagtatt 600
   tctttcgtgt gggccggcgg ccatggatgg atggatgtgt ctggatcggc taaagaagat 660
   aggatagcta gccctggccg gtcgtcttta cctgagcatg ggcatatgcc atcgaaaaaa 720
   gagacaacag catgcatgca tggtgcgcgc accagaccac gcagagcacc ggatgctcga 780
   gacaaagcaa cacaacaagc aaggacgaca cgtcaaaagc aacacaacaa gcaaggacgg 840
   cacgtcaaaa gcaacacaaa cctaaactaa agcacaaaga cgtaagagca agcacacaat 900
   cagcaggcta taaacagttg tcatcaaaaa caacgctgga agagagagag aaggaaggaa 960
   gtagtagcca tgaaaaatta aatcaccggg cgttgctctt tgcccaacaa ttaatcaagc 1020
   agggtacgtg gcatgtatag ttcttgtaag taaactaagc atgtgatatg agaaggtacg 1080
   tggtggtgca gacaacggcg gtcgcgggaa ggtgggcgcg gaggcggagc tggagcagtg 1140
   ggtgacgagc ctgccgtcgc tgacgacggg ggagtccaag ttcggcctca ccttcgactg 1200
   ggaggtggag aagctcgggg tgccgggcgc catcgtcgtc aacaactacc acagctccga 1260
   gttcctgctt aaaaccatca ccctccacga cgtccccggc cgcagcggca acctcacctt 1320
   cgtcgccaac tcatggatct accccgccgc caactaccga tacagccgcg tcttcttcgc 1380
   caacgacgtg cgtggatttt cctctacttt cctctccttt cattttcacc gccttcgtca 1440
   ttcatggtcg atcattaagt cttgccagga caatagatga tgagctagga gtggttacca 1500
   cttagcagta cgtacattat ttattccgtg ttggtagaaa aggatatggt ttggtgcaga 1560
   tcgacacaag attgaatgaa agttgcaccg tggcaccgtg gcagcgtggt aggtgaaaat 1620
   aactgttgca cggatccacc cacatgattg ttttcatgaa taaacttttt aaggatgtgt 1680
   ctagccacat ctagatgcat gtcacataat tattgcatac caaaacgatt aaattaagca 1740
   taaaaagaaa aggaaaaaaa tactcacata tctcgacgta agatcaatga tatagtattt 1800
   agatatgcaa tatttatctt acatctaaac ctttcttcat tcttaaatat aagacatttg 1860
   taagatttca ctatggacaa catacgaaac aaaatcagtg gatctctcta tgcattcatt 1920
   atgtagtcta taataaaatc tttaaaagat cgtatatttt gcaacggagg gagtaaaaca 1980
   taacttttta atagtaatgt tgcacggctc cacactcgca gacgtacctg ccgagccaga 2040
   tgccggcggc gctgaagccg taccgcgacg acgagctccg gaacctgcgt ggcgacgacc 2100
   agcagggccc gtaccaggag cacgaccgca tctaccgcta cgacgtctac aacgacctcg 2160
   gcgagggccg ccccatcctc ggcggcaact ccgaccaccc ttacccgcgc cgcggccgca 2220
   cggagcgcaa gcccaacgcc agcgacccga gcctggagag ccggctgtcg ctgctggagc 2280
   agatctacgt gccgcgggac gagaagttcg gccacctcaa gacgtccgac ttcctgggct 2340
   actccatcaa ggccatcacg cagggcatcc tgccggccgt gcgcacctac gtggacacca 2400
   cccccggcga gttcgactcc ttccaggaca tcatcaacct ctatgagggc ggcatcaagc 2460
   tgcccaaggt ggccgccctg gaggagctcc gtaagcagtt cccgctccag ctcatcaagg 2520
   acctcctccc cgtcggcggc gactccctgc ttaagctccc cgtgccccac atcatccagg 2580
   agaacaagca ggcgtggagg accgacgagg agttcgcacg ggaggtgctc gccnnngtca 2640
   acccggtcat gatcacgcgt ctcacggtga gtcagcgatt atttgttcat tgtgtgtgta 2700
   tggtgtccat ggtgagaaag tgcagatctt gatttgcgtt gggtcgcatg cacgcatgct 2760
   gcatgcatgc aggagttccc gccaaaaagt agtctggacc ctagcaagtt tggtgaccac 2820
   accagcacca tcacggcgga gcacatagag aagaacctcg agggcctcac ggtgcagcag 2880
   gtaattggtc caagccatcg acatcaacta tgatttacct aggagtaatt ggtagctgta 2940
   gataatttgg cttcgttgca attaatttga tgctggccga tcaagtgatc gtattgggtt 3000
   tgaaatttgc aggcgctgga aagcaacagg ctgtacatcc ttgatcacca tgaccggttc 3060
   atgccgttcc tgatcgacgt caacaacctg cccggcaact tcatctacgc cacgaggacc 3120
   ctcttcttcc tgcgcggcga cggcaggctc acgccgctcg ccatcgagct gagcgagccc 3180
   atcatccagg gcggccttac cacggccaag agcaaggttt acacgccggt gcccagcggc 3240
   tccgtcgaag gctgggtgtg ggagctcgcc aaggcctacg tcgccgtcaa tgactccggg 3300
   tggcaccagc tcgtcagcca ctggtacgtt ctccacggtc gatgtgattc agtcagtcga 3360
   tgcacaacaa ctgatcgaaa tatgattgat tgaaacgcgc aggctgaaca ctcacgcggt 3420
   gatggagccg ttcgtgatct cgacgaaccg gcaccttagc gtgacgcacc cggtgcacaa 3480
   gctgctgagc ccgcactacc gcgacaccat gaccatcaac gcgctggcgc ggcagacgct 3540
   catcaacgcc ggcggcatct tcgagatgac ggtgttcccg ggcaagttcg cgttggggat 3600
   gtcggccgtg gtgtacaagg actggaagtt caccgagcag ggactgccgg acgatctcat 3660
   caagaggtac gtacctggta aatgttatga atgtgtaaaa caaattgggc gtctcgctca 3720
   ctgacaggaa cgtggtaaaa aaaatgcagg ggcatggcgg tggaggaccc gtcgagcccg 3780
   tacaaggtgc ggttgctggt gtcggactac ccgtacgcgg cggacgggct ggcgatctgg 3840
   cacgccattg agcagtacgt gagcgagtac ctggccatct actacccgaa cgacggcgtg 3900
   ctgcagggcg atacggaggt gcaggcgtgg tggaaggaga cgcgcgaggt cgggcacggc 3960
   gacctcaagg acgccccatg gtggcccaag atgcaaagtg tgccggagct ggccaaggcg 4020
   tgcaccacca tcatctggat cgggtcggcg ctgcatgcgg cagtcaactt cgggcagtac 4080
   ccctacgcgg ggttcctccc gaaccggccg acggtgagcc ggcgccgcat gccggagccc 4140
   ggcacggagg agtacgcgga gctggagcgc gacccggagc gggccttcat ccacaccatc 4200
   acgagccaga tccagaccat catcggcgtg tcgctgctgg aggtgctgtc gaagcactcc 4260
   tccgacgagc tgtacctcgg gcagcgggac acgccggagt ggacctcgga cccaaaggcc 4320
   ctggaggtgt tcaagcggtt cagcgaccgg ctggtggaga tcgagagcaa ggtggtgggc 4380
   atgaaccatg acccggagct caagaaccgc aacggcccgg ctaagtttcc ctacatgctg 4440
   ctctacccca acacctccga ccacaagggc gccgctgccg ggcttaccgc caagggcatc 4500
   cccaacagca tctccatcta atctaagcca tcggcaacca tggatgaata aagggcgttc 4560
   gccacgtacg aaacttgtcg agagattggt gtagtgtgtg tctgtgacag tactatgtca 4620
   gcagttgctc tttaagccga ataaataaag cagatttgct tcc 4663

<210> 12
<211> 862
<212> PRT
<213> Hordeum vulgare

<220>

<221>  variation

<222>  (368)..(368)

<223>  "Xaa" is an acidic, basic, or polar amino acid


<400>  12

Met Leu Leu Gly Gly Leu Ile Asp Thr Leu Thr Gly Ala Asn Lys Ser
1               5                   10                  15

Ala Arg Leu Lys Gly Thr Val Val Leu Met Arg Lys Asn Val Leu Asp
            20                  25                  30

Leu Asn Asp Phe Gly Ala Thr Ile Ile Asp Gly Ile Gly Glu Phe Leu
        35                  40                  45

Gly Lys Gly Val Thr Cys Gln Leu Ile Ser Ser Thr Ala Val Asp Gln
    50                  55                  60

Asp Asn Gly Gly Arg Gly Lys Val Gly Ala Glu Ala Glu Leu Glu Gln
65                  70                  75                  80

Trp Val Thr Ser Leu Pro Ser Leu Thr Thr Gly Glu Ser Lys Phe Gly
                85                  90                  95

Leu Thr Phe Asp Trp Glu Val Glu Lys Leu Gly Val Pro Gly Ala Ile
            100                 105                 110

Val Val Asn Asn Tyr His Ser Ser Glu Phe Leu Leu Lys Thr Ile Thr
        115                 120                 125

Leu His Asp Val Pro Gly Arg Ser Gly Asn Leu Thr Phe Val Ala Asn
    130                 135                 140

Ser Trp Ile Tyr Pro Ala Ala Asn Tyr Arg Tyr Ser Arg Val Phe Phe
145                 150                 155                 160

Ala Asn Asp Thr Tyr Leu Pro Ser Gln Met Pro Ala Ala Leu Lys Pro
                165                 170                 175

Tyr Arg Asp Asp Glu Leu Arg Asn Leu Arg Gly Asp Asp Gln Gln Gly
            180                 185                 190

Pro Tyr Gln Glu His Asp Arg Ile Tyr Arg Tyr Asp Val Tyr Asn Asp
        195                 200                 205

Leu Gly Glu Gly Arg Pro Ile Leu Gly Gly Asn Ser Asp His Pro Tyr
    210                 215                 220

Pro Arg Arg Gly Arg Thr Glu Arg Lys Pro Asn Ala Ser Asp Pro Ser
225                 230                 235                 240

Leu Glu Ser Arg Leu Ser Leu Leu Glu Gln Ile Tyr Val Pro Arg Asp
                245                 250                 255

Glu Lys Phe Gly His Leu Lys Thr Ser Asp Phe Leu Gly Tyr Ser Ile

            260                 265                 270

Lys Ala Ile Thr Gln Gly Ile Leu Pro Ala Val Arg Thr Tyr Val Asp
        275                 280                 285

Thr Thr Pro Gly Glu Phe Asp Ser Phe Gln Asp Ile Ile Asn Leu Tyr
    290                 295                 300

Glu Gly Gly Ile Lys Leu Pro Lys Val Ala Ala Leu Glu Glu Leu Arg
305                 310                 315                 320

Lys Gln Phe Pro Leu Gln Leu Ile Lys Asp Leu Leu Pro Val Gly Gly
                325                 330                 335

Asp Ser Leu Leu Lys Leu Pro Val Pro His Ile Ile Gln Glu Asn Lys
            340                 345                 350

Gln Ala Trp Arg Thr Asp Glu Glu Phe Ala Arg Glu Val Leu Ala Xaa
        355                 360                 365

Val Asn Pro Val Met Ile Thr Arg Leu Thr Glu Phe Pro Pro Lys Ser
    370                 375                 380

Ser Leu Asp Pro Ser Lys Phe Gly Asp His Thr Ser Thr Ile Thr Ala
385                 390                 395                 400

Glu His Ile Glu Lys Asn Leu Glu Gly Leu Thr Val Gln Gln Ala Leu
                405                 410                 415

Glu Ser Asn Arg Leu Tyr Ile Leu Asp His His Asp Arg Phe Met Pro
            420                 425                 430

Phe Leu Ile Asp Val Asn Asn Leu Pro Gly Asn Phe Ile Tyr Ala Thr
        435                 440                 445

Arg Thr Leu Phe Phe Leu Arg Gly Asp Gly Arg Leu Thr Pro Leu Ala
    450                 455                 460

Ile Glu Leu Ser Glu Pro Ile Ile Gln Gly Gly Leu Thr Thr Ala Lys
465                 470                 475                 480

Ser Lys Val Tyr Thr Pro Val Pro Ser Gly Ser Val Glu Gly Trp Val
                485                 490                 495

Trp Glu Leu Ala Lys Ala Tyr Val Ala Val Asn Asp Ser Gly Trp His
            500                 505                 510

Gln Leu Val Ser His Trp Leu Asn Thr His Ala Val Met Glu Pro Phe
        515                 520                 525

Val Ile Ser Thr Asn Arg His Leu Ser Val Thr His Pro Val His Lys
    530                 535                 540

Leu Leu Ser Pro His Tyr Arg Asp Thr Met Thr Ile Asn Ala Leu Ala
545                 550                 555                 560

Arg Gln Thr Leu Ile Asn Ala Gly Gly Ile Phe Glu Met Thr Val Phe
                565                 570                 575

Pro Gly Lys Phe Ala Leu Gly Met Ser Ala Val Val Tyr Lys Asp Trp
            580                 585                 590

Lys Phe Thr Glu Gln Gly Leu Pro Asp Asp Leu Ile Lys Arg Gly Met
        595                 600                 605

Ala Val Glu Asp Pro Ser Ser Pro Tyr Lys Val Arg Leu Leu Val Ser
    610                 615                 620

Asp Tyr Pro Tyr Ala Ala Asp Gly Leu Ala Ile Trp His Ala Ile Glu
625                 630                 635                 640

Gln Tyr Val Ser Glu Tyr Leu Ala Ile Tyr Tyr Pro Asn Asp Gly Val
                645                 650                 655

Leu Gln Gly Asp Thr Glu Val Gln Ala Trp Trp Lys Glu Thr Arg Glu
            660                 665                 670

Val Gly His Gly Asp Leu Lys Asp Ala Pro Trp Trp Pro Lys Met Gln
        675                 680                 685

Ser Val Pro Glu Leu Ala Lys Ala Cys Thr Thr Ile Ile Trp Ile Gly
    690                 695                 700

Ser Ala Leu His Ala Ala Val Asn Phe Gly Gln Tyr Pro Tyr Ala Gly
705                 710                 715                 720

Phe Leu Pro Asn Arg Pro Thr Val Ser Arg Arg Arg Met Pro Glu Pro
                725                 730                 735

Gly Thr Glu Glu Tyr Ala Glu Leu Glu Arg Asp Pro Glu Arg Ala Phe
            740                 745                 750

Ile His Thr Ile Thr Ser Gln Ile Gln Thr Ile Ile Gly Val Ser Leu
        755                 760                 765

Leu Glu Val Leu Ser Lys His Ser Ser Asp Glu Leu Tyr Leu Gly Gln
    770                 775                 780

Arg Asp Thr Pro Glu Trp Thr Ser Asp Pro Lys Ala Leu Glu Val Phe
785                 790                 795                 800

Lys Arg Phe Ser Asp Arg Leu Val Glu Ile Glu Ser Lys Val Val Gly
                805                 810                 815

Met Asn His Asp Pro Glu Leu Lys Asn Arg Asn Gly Pro Ala Lys Phe
            820                 825                 830

Pro Tyr Met Leu Leu Tyr Pro Asn Thr Ser Asp His Lys Gly Ala Ala
        835                 840                 845

Ala Gly Leu Thr Ala Lys Gly Ile Pro Asn Ser Ile Ser Ile
    850                 855                 860

<210>  13

<211>  20

<212>  DNA

<213>  Hordeum vulgare


<400>  13
cgctacgacg tctacaacga                                                  20


<210>  14

<211>  20

<212>  DNA

<213>  Hordeum vulgare


<400>  14
cagactactt tttggcggga                                                    20


<210>  15

<211>  839

<212>  PRT

<213>  Glycine max


<400>  15

Met Phe Ser Ala Gly His Lys Ile Lys Gly Thr Val Val Leu Met Pro
1               5                   10                  15

Lys Asn Glu Leu Glu Val Asn Pro Asp Gly Ser Ala Val Asp Asn Leu
            20                  25                  30

Asn Ala Phe Leu Gly Arg Ser Val Ser Leu Gln Leu Ile Ser Ala Thr
        35                  40                  45

Lys Ala Asp Ala His Gly Lys Gly Lys Val Gly Lys Asp Thr Phe Leu
    50                  55                  60

Glu Gly Ile Asn Thr Ser Leu Pro Thr Leu Gly Ala Gly Glu Ser Ala
65                  70                  75                  80

Phe Asn Ile His Phe Glu Trp Asp Gly Ser Met Gly Ile Pro Gly Ala
                85                  90                  95

Phe Tyr Ile Lys Asn Tyr Met Gln Val Glu Phe Phe Leu Lys Ser Leu
            100                 105                 110

Thr Leu Glu Ala Ile Ser Asn Gln Gly Thr Ile Arg Phe Val Cys Asn
        115                 120                 125

Ser Trp Val Tyr Asn Thr Lys Leu Tyr Lys Ser Val Arg Ile Phe Phe
    130                 135                 140

Ala Asn His Thr Tyr Val Pro Ser Glu Thr Pro Ala Pro Leu Val Ser
145                 150                 155                 160

Tyr Arg Glu Glu Glu Leu Lys Ser Leu Arg Gly Asn Gly Thr Gly Glu
                165                 170                 175

Arg Lys Glu Tyr Asp Arg Ile Tyr Asp Tyr Asp Val Tyr Asn Asp Leu
            180                 185                 190

Gly Asn Pro Asp Lys Ser Glu Lys Leu Ala Arg Pro Val Leu Gly Gly
        195                 200                 205

Ser Ser Thr Phe Pro Tyr Pro Arg Arg Gly Arg Thr Gly Arg Gly Pro
    210                 215                 220

Thr Val Thr Asp Pro Asn Thr Glu Lys Gln Gly Glu Val Phe Tyr Val
225                 230                 235                 240

Pro Arg Asp Glu Asn Leu Gly His Leu Lys Ser Lys Asp Ala Leu Glu
                245                 250                 255

Ile Gly Thr Lys Ser Leu Ser Gln Ile Val Gln Pro Ala Phe Glu Ser
            260                 265                 270

Ala Phe Asp Leu Lys Ser Thr Pro Ile Glu Phe His Ser Phe Gln Asp
        275                 280                 285

Val His Asp Leu Tyr Glu Gly Gly Ile Lys Leu Pro Arg Asp Val Ile
    290                 295                 300

Ser Thr Ile Ile Pro Leu Pro Val Ile Lys Glu Leu Tyr Arg Thr Asp
305                 310                 315                 320

Gly Gln His Ile Leu Lys Phe Pro Gln Pro His Val Val Gln Val Ser
                325                 330                 335

Gln Ser Ala Trp Met Thr Asp Glu Glu Phe Ala Arg Glu Met Ile Ala
            340                 345                 350

Gly Val Asn Pro Cys Val Ile Arg Gly Leu Glu Glu Phe Pro Pro Lys
        355                 360                 365

Ser Asn Leu Asp Pro Ala Ile Tyr Gly Asp Gln Ser Ser Lys Ile Thr
    370                 375                 380

Ala Asp Ser Leu Asp Leu Asp Gly Tyr Thr Met Asp Glu Ala Leu Gly
385                 390                 395                 400

Ser Arg Arg Leu Phe Met Leu Asp Tyr His Asp Ile Phe Met Pro Tyr
                405                 410                 415

Val Arg Gln Ile Asn Gln Leu Asn Ser Ala Lys Thr Tyr Ala Thr Arg
            420                 425                 430

Thr Ile Leu Phe Leu Arg Glu Asp Gly Thr Leu Lys Pro Val Ala Ile
        435                 440                 445

Glu Leu Ser Leu Pro His Ser Ala Gly Asp Leu Ser Ala Ala Val Ser
    450                 455                 460

Gln Val Val Leu Pro Ala Lys Glu Gly Val Glu Ser Thr Ile Trp Leu
465                 470                 475                 480

Leu Ala Lys Ala Tyr Val Ile Val Asn Asp Ser Cys Tyr His Gln Leu
                485                 490                 495

Met Ser His Trp Leu Asn Thr His Ala Ala Met Glu Pro Phe Val Ile
            500                 505                 510

Ala Thr His Arg His Leu Ser Val Leu His Pro Ile Tyr Lys Leu Leu
        515                 520                 525

Thr Pro His Tyr Arg Asn Asn Met Asn Ile Asn Ala Leu Ala Arg Gln
    530                 535                 540

Ser Leu Ile Asn Ala Asn Gly Ile Ile Glu Thr Thr Phe Leu Pro Ser

545                 550                 555                 560

Lys Tyr Ser Val Glu Met Ser Ser Ala Val Tyr Lys Asn Trp Val Phe
                565                 570                 575

Thr Asp Gln Ala Leu Pro Ala Asp Leu Ile Lys Arg Gly Val Ala Ile
            580                 585                 590

Lys Asp Pro Ser Thr Pro His Gly Val Arg Leu Leu Ile Glu Asp Tyr
        595                 600                 605

Pro Tyr Ala Ala Asp Gly Leu Glu Ile Trp Ala Ala Ile Lys Thr Trp
    610                 615                 620

Val Gln Glu Tyr Val Pro Leu Tyr Tyr Ala Arg Asp Asp Asp Val Lys
625                 630                 635                 640

Asn Asp Ser Glu Leu Gln His Trp Trp Lys Glu Ala Val Glu Lys Gly
                645                 650                 655

His Gly Asp Leu Lys Asp Lys Pro Trp Trp Pro Lys Leu Gln Thr Leu
            660                 665                 670

Glu Asp Leu Val Glu Val Cys Leu Ile Ile Ile Trp Ile Ala Ser Ala
        675                 680                 685

Leu His Ala Ala Val Asn Phe Gly Gln Tyr Pro Tyr Gly Gly Leu Ile
    690                 695                 700

Met Asn Arg Pro Thr Ala Ser Arg Arg Leu Leu Pro Glu Lys Gly Thr
705                 710                 715                 720

Pro Glu Tyr Glu Glu Met Ile Asn Asn His Glu Lys Ala Tyr Leu Arg
                725                 730                 735

Thr Ile Thr Ser Lys Leu Pro Thr Leu Ile Ser Leu Ser Val Ile Glu
            740                 745                 750

Ile Leu Ser Thr His Ala Ser Asp Glu Val Tyr Leu Gly Gln Arg Asp
        755                 760                 765

Asn Pro His Trp Thr Ser Asp Ser Lys Ala Leu Gln Ala Phe Gln Lys
    770                 775                 780

Phe Gly Asn Lys Leu Lys Glu Ile Glu Glu Lys Leu Val Arg Arg Asn
785                 790                 795                 800

Asn Asp Pro Ser Leu Gln Gly Asn Arg Leu Gly Pro Val Gln Leu Pro
                805                 810                 815

Tyr Thr Leu Leu Tyr Pro Ser Ser Glu Glu Gly Leu Thr Phe Arg Gly
            820                 825                 830

Ile Pro Asn Ser Ile Ser Ile
        835

<210>  16

<211>  865

<212>  PRT

<213>  Glycine max

<400>  16

Met Phe Ser Val Pro Gly Val Ser Gly Ile Leu Asn Arg Gly Gly Gly
1               5                   10                  15

His Lys Ile Lys Gly Thr Val Val Leu Met Arg Lys Asn Val Leu Asp
            20                  25                  30

Phe Asn Ser Val Ala Asp Leu Thr Lys Gly Asn Val Gly Gly Leu Ile
        35                  40                  45

Gly Thr Gly Leu Asn Val Val Gly Ser Thr Leu Asp Asn Leu Thr Ala
    50                  55                  60

Phe Leu Gly Arg Ser Val Ala Leu Gln Leu Ile Ser Ala Thr Lys Pro
65                  70                  75                  80

Leu Ala Asn Gly Lys Gly Lys Val Gly Lys Asp Thr Phe Leu Glu Gly
                85                  90                  95

Ile Ile Val Ser Leu Pro Thr Leu Gly Ala Gly Glu Ser Ala Phe Asn
            100                 105                 110

Ile Gln Phe Glu Trp Asp Glu Ser Met Gly Ile Pro Gly Ala Phe Tyr
        115                 120                 125

Ile Lys Asn Tyr Met Gln Val Glu Phe Tyr Leu Lys Ser Leu Thr Leu
    130                 135                 140

Glu Asp Val Pro Asn Gln Gly Thr Ile Arg Phe Val Cys Asn Ser Trp
145                 150                 155                 160

Val Tyr Asn Thr Lys Leu Tyr Lys Ser Val Arg Ile Phe Phe Ala Asn
                165                 170                 175

His Thr Tyr Val Pro Ser Glu Thr Pro Ala Ala Leu Val Gly Tyr Arg
            180                 185                 190

Glu Glu Glu Leu Lys Asn Leu Arg Gly Asp Gly Lys Gly Glu Arg Lys
        195                 200                 205

Glu His Asp Arg Ile Tyr Asp Tyr Asp Val Tyr Asn Asp Leu Gly Asn
    210                 215                 220

Pro Asp His Gly Glu Asn Phe Ala Arg Pro Ile Leu Gly Gly Ser Ser
225                 230                 235                 240

Thr His Pro Tyr Pro Arg Arg Gly Arg Thr Gly Arg Tyr Pro Thr Arg
                245                 250                 255

Lys Asp Gln Asn Ser Glu Lys Pro Gly Glu Val Tyr Val Pro Arg Asp
            260                 265                 270

Glu Asn Phe Gly His Leu Lys Ser Ser Asp Phe Leu Ala Tyr Gly Ile
        275                 280                 285

Lys Ser Leu Ser Gln Tyr Val Leu Pro Ala Phe Glu Ser Val Phe Asp
    290                 295                 300

Leu Asn Phe Thr Pro Asn Glu Phe Asp Ser Phe Gln Asp Val Arg Asp
305                 310                 315                 320

Leu His Glu Gly Gly Ile Lys Leu Pro Thr Glu Val Ile Ser Thr Ile
                325                 330                 335

Met Pro Leu Pro Val Val Lys Glu Leu Phe Arg Thr Asp Gly Glu Gln
            340                 345                 350

Val Leu Lys Phe Pro Pro Pro His Val Ile Gln Val Ser Lys Ser Ala
        355                 360                 365

Trp Met Thr Asp Glu Glu Phe Ala Arg Glu Met Val Ala Gly Val Asn
    370                 375                 380

Pro Cys Val Ile Arg Gly Leu Gln Glu Phe Pro Pro Lys Ser Asn Leu
385                 390                 395                 400

Asp Pro Thr Ile Tyr Gly Glu Gln Thr Ser Lys Ile Thr Ala Asp Ala
                405                 410                 415

Leu Asp Leu Asp Gly Tyr Thr Val Asp Glu Ala Leu Ala Ser Arg Arg
            420                 425                 430

Leu Phe Met Leu Asp Tyr His Asp Val Phe Met Pro Tyr Ile Arg Arg
        435                 440                 445

Ile Asn Gln Thr Tyr Ala Lys Ala Tyr Ala Thr Arg Thr Ile Leu Phe
    450                 455                 460

Leu Arg Glu Asn Gly Thr Leu Lys Pro Val Ala Ile Glu Leu Ser Leu
465                 470                 475                 480

Pro His Pro Ala Gly Asp Leu Ser Gly Ala Val Ser Gln Val Ile Leu
                485                 490                 495

Pro Ala Lys Glu Gly Val Glu Ser Thr Ile Trp Leu Leu Ala Lys Ala
            500                 505                 510

Tyr Val Val Val Asn Asp Ser Cys Tyr His Gln Leu Met Ser His Trp
        515                 520                 525

Leu Asn Thr His Ala Val Ile Glu Pro Phe Ile Ile Ala Thr Asn Arg
    530                 535                 540

His Leu Ser Ala Leu His Pro Ile Tyr Lys Leu Leu Thr Pro His Tyr
545                 550                 555                 560

Arg Asp Thr Met Asn Ile Asn Ala Leu Ala Arg Gln Ser Leu Ile Asn
                565                 570                 575

Ala Asp Gly Ile Ile Glu Lys Ser Phe Leu Pro Ser Lys His Ser Val
            580                 585                 590

Glu Met Ser Ser Ala Val Tyr Lys Asn Trp Val Phe Thr Asp Gln Ala
        595                 600                 605

Leu Pro Ala Asp Leu Ile Lys Arg Gly Val Ala Ile Lys Asp Pro Ser
    610                 615                 620

Ala Pro His Gly Leu Arg Leu Leu Ile Glu Asp Tyr Pro Tyr Ala Val
625                 630                 635                 640

Asp Gly Leu Glu Ile Trp Ala Ala Ile Lys Thr Trp Val Gln Glu Tyr
                645                 650                 655

Val Ser Leu Tyr Tyr Ala Arg Asp Asp Asp Val Lys Pro Asp Ser Glu

660                 665                 670

Leu Gln Gln Trp Trp Lys Glu Ala Val Glu Lys Gly His Gly Asp Leu
        675                 680                 685

Lys Asp Lys Pro Trp Trp Pro Lys Leu Gln Thr Ile Glu Glu Leu Val
    690                 695                 700

Glu Ile Cys Thr Ile Ile Ile Trp Thr Ala Ser Ala Leu His Ala Ala
705                 710                 715                 720

Val Asn Phe Gly Gln Tyr Pro Tyr Gly Gly Phe Ile Leu Asn Arg Pro
                725                 730                 735

Thr Ser Ser Arg Arg Leu Leu Pro Glu Lys Gly Thr Pro Glu Tyr Glu
            740                 745                 750

Glu Met Val Lys Ser His Gln Lys Ala Tyr Leu Arg Thr Ile Thr Ser
        755                 760                 765

Lys Phe Gln Thr Leu Val Asp Leu Ser Val Ile Glu Ile Leu Ser Arg
    770                 775                 780

His Ala Ser Asp Glu Val Tyr Leu Gly Gln Arg Asp Asn Pro His Trp
785                 790                 795                 800

Thr Ser Asp Ser Lys Ala Leu Gln Ala Phe Gln Lys Phe Gly Asn Lys
                805                 810                 815

Leu Lys Glu Ile Glu Glu Lys Leu Ala Arg Lys Asn Asn Asp Gln Ser
            820                 825                 830

Leu Ser Asn Arg Leu Gly Pro Val Gln Leu Pro Tyr Thr Leu Leu His
        835                 840                 845

Pro Asn Ser Glu Gly Leu Thr Cys Arg Gly Ile Pro Asn Ser Ile Ser
    850                 855                 860

Ile
865

<210>  17

<211>  857

<212>  PRT

<213>  Glycine max

<400>  17

Met Leu Gly Gly Leu Leu His Arg Gly His Lys Ile Lys Gly Thr Val
1               5                   10                  15

Val Leu Met Arg Lys Asn Val Leu Asp Val Asn Ser Val Thr Ser Val
            20                  25                  30

Gly Gly Ile Ile Gly Gln Gly Leu Asp Leu Val Gly Ser Thr Leu Asp
        35                  40                  45

Thr Leu Thr Ala Phe Leu Gly Arg Ser Val Ser Leu Gln Leu Ile Ser
    50                  55                  60

Ala Thr Lys Ala Asp Ala Asn Gly Lys Gly Lys Leu Gly Lys Ala Thr
65                  70                  75                  80

Phe Leu Glu Gly Ile Ile Thr Ser Leu Pro Thr Leu Gly Ala Gly Gln
                85                  90                  95

Ser Ala Phe Lys Ile Asn Phe Glu Trp Asp Asp Gly Ser Gly Ile Pro
            100                 105                 110

Gly Ala Phe Tyr Ile Lys Asn Phe Met Gln Thr Glu Phe Phe Leu Val
        115                 120                 125

Ser Leu Thr Leu Glu Asp Ile Pro Asn His Gly Ser Ile His Phe Val
    130                 135                 140

Cys Asn Ser Trp Ile Tyr Asn Ala Lys Leu Phe Lys Ser Asp Arg Ile
145                 150                 155                 160

Phe Phe Ala Asn Gln Thr Tyr Leu Pro Ser Glu Thr Pro Ala Pro Leu
                165                 170                 175

Val Lys Tyr Arg Glu Glu Glu Leu His Asn Leu Arg Gly Asp Gly Thr
            180                 185                 190

Gly Glu Arg Lys Glu Trp Glu Arg Ile Tyr Asp Tyr Asp Val Tyr Asn
        195                 200                 205

Asp Leu Gly Asp Pro Asp Lys Gly Glu Asn His Ala Arg Pro Val Leu
    210                 215                 220

Gly Gly Asn Asp Thr Phe Pro Tyr Pro Arg Arg Gly Arg Thr Gly Arg
225                 230                 235                 240

Lys Pro Thr Arg Lys Asp Pro Asn Ser Glu Ser Arg Ser Asn Asp Val
                245                 250                 255

Tyr Leu Pro Arg Asp Glu Ala Phe Gly His Leu Lys Ser Ser Asp Phe
            260                 265                 270

Leu Thr Tyr Gly Leu Lys Ser Val Ser Gln Asn Val Leu Pro Leu Leu
        275                 280                 285

Gln Ser Ala Phe Asp Leu Asn Phe Thr Pro Arg Glu Phe Asp Ser Phe
    290                 295                 300

Asp Glu Val His Gly Leu Tyr Ser Gly Gly Ile Lys Leu Pro Thr Asp
305                 310                 315                 320

Ile Ile Ser Lys Ile Ser Pro Leu Pro Val Leu Lys Glu Ile Phe Arg
                325                 330                 335

Thr Asp Gly Glu Gln Ala Leu Lys Phe Pro Pro Pro Lys Val Ile Gln
            340                 345                 350

Val Ser Lys Ser Ala Trp Met Thr Asp Glu Glu Phe Ala Arg Glu Met
        355                 360                 365

Leu Ala Gly Val Asn Pro Asn Leu Ile Arg Cys Leu Lys Asp Phe Pro
    370                 375                 380

Pro Arg Ser Lys Leu Asp Ser Gln Val Tyr Gly Asp His Thr Ser Gln
385                 390                 395                 400

Ile Thr Lys Glu His Leu Glu Pro Asn Leu Glu Gly Leu Thr Val Asp
                405                 410                 415

Glu Ala Ile Gln Asn Lys Arg Leu Phe Leu Leu Asp His His Asp Pro
            420                 425                 430

Ile Met Pro Tyr Leu Arg Arg Ile Asn Ala Thr Ser Thr Lys Ala Tyr
        435                 440                 445

Ala Thr Arg Thr Ile Leu Phe Leu Lys Asn Asp Gly Thr Leu Arg Pro
    450                 455                 460

Leu Ala Ile Glu Leu Ser Leu Pro His Pro Gln Gly Asp Gln Ser Gly
465                 470                 475                 480

Ala Phe Ser Gln Val Phe Leu Pro Ala Asp Glu Gly Val Glu Ser Ser
                485                 490                 495

Ile Trp Leu Leu Ala Lys Ala Tyr Val Val Val Asn Asp Ser Cys Tyr
            500                 505                 510

His Gln Leu Val Ser His Trp Leu Asn Thr His Ala Val Val Glu Pro
        515                 520                 525

Phe Ile Ile Ala Thr Asn Arg His Leu Ser Val Val His Pro Ile Tyr
    530                 535                 540

Lys Leu Leu His Pro His Tyr Arg Asp Thr Met Asn Ile Asn Gly Leu
545                 550                 555                 560

Ala Arg Leu Ser Leu Val Asn Asp Gly Gly Val Ile Glu Gln Thr Phe
                565                 570                 575

Leu Trp Gly Arg Tyr Ser Val Glu Met Ser Ala Val Val Tyr Lys Asp
            580                 585                 590

Trp Val Phe Thr Asp Gln Ala Leu Pro Ala Asp Leu Ile Lys Arg Gly
        595                 600                 605

Met Ala Ile Glu Asp Pro Ser Cys Pro His Gly Ile Arg Leu Val Ile
    610                 615                 620

Glu Asp Tyr Pro Tyr Thr Val Asp Gly Leu Glu Ile Trp Asp Ala Ile
625                 630                 635                 640

Lys Thr Trp Val His Glu Tyr Val Phe Leu Tyr Tyr Lys Ser Asp Asp
                645                 650                 655

Thr Leu Arg Glu Asp Pro Glu Leu Gln Ala Cys Trp Lys Glu Leu Val
            660                 665                 670

Glu Val Gly His Gly Asp Lys Lys Asn Glu Pro Trp Trp Pro Lys Met
        675                 680                 685

Gln Thr Arg Glu Glu Leu Val Glu Ala Cys Ala Ile Ile Ile Trp Thr
    690                 695                 700

Ala Ser Ala Leu His Ala Ala Val Asn Phe Gly Gln Tyr Pro Tyr Gly
705                 710                 715                 720

Gly Leu Ile Leu Asn Arg Pro Thr Leu Ser Arg Arg Phe Met Pro Glu
                725                 730                 735

Lys Gly Ser Ala Glu Tyr Glu Glu Leu Arg Lys Asn Pro Gln Lys Ala

740                 745                 750

Tyr Leu Lys Thr Ile Thr Pro Lys Phe Gln Thr Leu Ile Asp Leu Ser
        755                 760                 765

Val Ile Glu Ile Leu Ser Arg His Ala Ser Asp Glu Val Tyr Leu Gly
    770                 775                 780

Glu Arg Asp Asn Pro Asn Trp Thr Ser Asp Thr Arg Ala Leu Glu Ala
785                 790                 795                 800

Phe Lys Arg Phe Gly Asn Lys Leu Ala Gln Ile Glu Asn Lys Leu Ser
                805                 810                 815

Glu Arg Asn Asn Asp Glu Lys Leu Arg Asn Arg Cys Gly Pro Val Gln
            820                 825                 830

Met Pro Tyr Thr Leu Leu Leu Pro Ser Ser Lys Glu Gly Leu Thr Phe
        835                 840                 845

Arg Gly Ile Pro Asn Ser Ile Ser Ile
    850                 855

<210>  18

<211>  864

<212>  PRT

<213>  Hordeum vulgare


<400>  18

Met Leu Gly Val Gly Gly Ile Val Ser Asp Leu Thr Gly Gly Ile Arg
1               5                   10                  15

Gly Ala His Leu Lys Gly Ser Val Val Leu Met Arg Lys Asn Ala Leu
            20                  25                  30

Asp Phe Asn Asp Phe Gly Ala His Val Met Asp Gly Val Thr Glu Leu
        35                  40                  45

Leu Gly Arg Gly Val Thr Cys Gln Leu Ile Ser Ser Thr Asn Val Asp
    50                  55                  60

His Asn Asn Gly Gly Arg Gly Lys Val Gly Ala Glu Ala Asn Leu Glu
65                  70                  75                  80

Gln Trp Leu Leu Pro Thr Asn Leu Pro Phe Ile Thr Thr Gly Glu Asn
                85                  90                  95

Lys Phe Ala Val Thr Phe Asp Trp Ser Val Asp Lys Leu Gly Val Pro
            100                 105                 110

Gly Ala Ile Ile Val Lys Asn Asn His Ala Ser Glu Phe Phe Leu Lys
        115                 120                 125

Thr Ile Thr Leu Asp Asn Val Pro Gly Arg Gly Thr Ile Val Phe Val
    130                 135                 140

Ala Asn Ser Trp Val Tyr Pro Gln Ala Lys Tyr Arg Tyr Asn Arg Val
145                 150                 155                 160

Phe Phe Ala Asn Asp Thr Tyr Leu Pro His Gln Met Pro Ala Ala Leu
                165                 170                 175

Lys Pro Tyr Arg Asp Asp Glu Leu Arg Asn Leu Arg Gly Asp Asp Gln
            180                 185                 190

Gln Gly Pro Tyr Leu Asp His Asp Arg Val Tyr Arg Tyr Asp Val Tyr
        195                 200                 205

Asn Asp Leu Gly Asp Ser Arg Asp Val Leu Gly Gly Ser Lys Asp Leu
    210                 215                 220

Pro Tyr Pro Arg Arg Cys Arg Thr Gly Arg Lys Pro Ser Asp Ser Lys
225                 230                 235                 240

Pro Asp His Glu Ser Arg Leu Leu Leu Leu Val Gln Asn Val Tyr Val
                245                 250                 255

Leu Arg Asp Glu Leu Phe Gly His Leu Lys Gln Ser Asp Leu Leu Gly
            260                 265                 270

Tyr Thr Leu Lys Gly Trp Leu Asp Gly Ile Ile Leu Ala Ile Arg Thr
        275                 280                 285

Tyr Val Asp Leu Ser Pro Gly Glu Phe Asp Ser Phe Ala Asp Ile Leu
    290                 295                 300

Lys Leu Tyr Glu Gly Gly Ile Lys Leu Pro Asn Ile Pro Ala Leu Glu
305                 310                 315                 320

Glu Val Arg Lys Arg Phe Pro Leu Gln Leu Val Lys Asp Leu Ile Pro
                325                 330                 335

Lys Gly Gly Asp Phe Leu Leu Lys Leu Pro Lys Pro Glu Ile Ile Lys
            340                 345                 350

Val Asp Gln Lys Ala Trp Met Thr Asp Glu Glu Phe Ala Arg Glu Met
        355                 360                 365

Leu Ala Gly Val Asn Pro Met Met Ile Lys Arg Leu Thr Glu Phe Pro
    370                 375                 380

Pro Lys Ser Thr Leu Asp Pro Ser Lys Tyr Gly Asp His Thr Ser Thr
385                 390                 395                 400

Met Thr Glu Glu His Val Ala Lys Ser Leu Glu Gly Leu Thr Val Gln
                405                 410                 415

Gln Ala Leu Ala Gly Asn Arg Leu Tyr Ile Val Asp Gln His Asp Asn
            420                 425                 430

Leu Met Pro Phe Leu Ile Asp Ile Asn Asn Leu Asp Ala Ser Phe Val
        435                 440                 445

Tyr Ala Thr Arg Thr Leu Leu Phe Leu Arg Gly Asp Gly Thr Leu Ala
    450                 455                 460

Pro Val Ala Ile Glu Leu Ser Ser Pro Leu Ile Gln Gly Glu Leu Thr
465                 470                 475                 480

Thr Ala Lys Ser Ala Val Tyr Thr Pro Gln His Ala Gly Val Glu Gly
                485                 490                 495

Trp Ile Trp Gln Leu Ala Lys Ala Tyr Ala Ser Val Asn Asp Tyr Gly
            500                 505                 510

Trp His Gln Leu Ile Ser His Trp Leu Asn Thr His Ala Val Met Glu
        515                 520                 525

Pro Phe Val Ile Ala Thr Asn Arg Gln Leu Ser Val Thr His Pro Val
    530                 535                 540

Tyr Lys Leu Leu His Pro His Tyr Arg Asp Thr Met Asn Ile Asn Ala
545                 550                 555                 560

Arg Ala Arg Gly Leu Leu Ile Asn Ala Gly Gly Val Ile Glu Met Thr
                565                 570                 575

Val Phe Pro His Lys His Ala Met Pro Met Ser Ser Met Val Tyr Lys
            580                 585                 590

His Trp Asn Phe Thr Glu Gln Ala Leu Pro Ala Asp Leu Ile Lys Arg
        595                 600                 605

Gly Met Ala Val Glu Asp Ala Ser Ser Pro His Lys Val Arg Leu Leu
    610                 615                 620

Ile Lys Asp Tyr Pro Tyr Ala Thr Asp Gly Leu Ala Val Trp Asp Ala
625                 630                 635                 640

Ile Glu Gln Trp Val Ser Asp Tyr Leu Thr Ile Tyr Tyr Pro Asn Asp
                645                 650                 655

Gly Val Leu Gln Gly Asp Val Glu Leu Gln Ala Trp Trp Lys Glu Val
            660                 665                 670

Arg Glu Val Gly His Gly Asp Leu Lys Asp Ala Ala Trp Trp Pro Lys
        675                 680                 685

Met Gln Thr Val Ala Glu Leu Ile Lys Ala Cys Ala Thr Ile Ile Trp
    690                 695                 700

Thr Gly Ser Ala Leu His Ala Ala Val Asn Phe Gly Gln Tyr Pro Tyr
705                 710                 715                 720

Ser Gly Tyr His Pro Asn Lys Pro Ser Ala Ser Arg Arg Pro Met Pro
                725                 730                 735

Val Gln Gly Ser Glu Glu Tyr Ala Glu Leu Glu Arg Asp Pro Glu Lys
            740                 745                 750

Ala Phe Ile Arg Thr Ile Thr Ser Gln Phe His Ala Leu Val Gly Ile
        755                 760                 765

Ser Leu Met Glu Ile Leu Ser Lys His Ser Ser Asp Glu Val Tyr Leu
    770                 775                 780

Gly Gln His Asp Thr Pro Ala Trp Thr Ser Asp Ala Lys Ala Leu Glu
785                 790                 795                 800

Ala Phe Lys Arg Phe Gly Ala Lys Leu Glu Gly Ile Glu Lys Gln Val
                805                 810                 815

Val Ala Met Asn Ser Asp Pro Gln Leu Lys Asn Arg Thr Gly Pro Ala
            820                 825                 830

Lys Phe Pro Tyr Met Leu Leu Tyr Pro Asn Thr Ser Asp His Thr Gly

        835                 840                 845
Gln Ala Glu Gly Leu Thr Ala Arg Gly Ile Pro Asn Ser Ile Ser Ile
    850                 855                 860

## Description

This application is being filed as a PCT International patent application in the name of Carlsberg Research Laboratory, a Denmark corporation, on 22 January 2001, designating all countries.

### FIELD OF THE INVENTION

This invention is in the field of plant biotechnology. More specifically, the invention relates to a mutant barley lipoxygenase 1 gene (*lox*-1) that encodes an enzyme with severely reduced 9-hydroperoxy-octadecanoic acid forming activity. The invention also relates to the use of barley cultivars homozygous for *lox*-1 in brewing processes to reduce the formation of off-flavors in brewed products, such as beer, during storage.

### BACKGROUND OF THE INVENTION

Lipoxygenases are a family of enzymes (EC 1.13.11.12) that catalyze the dioxidation of free and esterified poly-unsaturated fatty acids containing a 1(Z), 4(Z)-pentadiene configuration. The products of lipoxygenase-catalyzed reactions have long been suspected as major culprits for the appearance of stale flavors in plant grain/seed and grain/seed derived food products (Robinson et al., 1995, Food Chem., 54: 33-43). Lipoxygenases have been implicated in the production of volatile hexanal aldehydes generated during soybean processing, which have an undesirable aroma, limiting the use of soybean proteins in food products. Three lipoxygenase isozymes expressed in soybean seed are believed to contribute to lipid oxidation and hexanal formation. Soybean mutants lacking one or more of these isozymes have been generated with the aim of reducing hexanal formation and improving their flavor stability. The success of this approach has been evaluated by Hildebrand et al., 1990, J. Agric. Food Chem. 38: 1934-1936. Mutants lacking soybean lipoxygenase 3 produced higher hexanal levels, suggesting that this isozyme diverts 13-hydroxyperoxyoctadecanoids, produced by lipid oxidation, towards non-volatile products. The field performance of triple-null soybean lines, lacking all three seed lipoxygenases, has shown that these enzymes are not essential for normal agronomic and seed characteristics (Narvel et al., 1998, Crop Sci. 38: 926-928).

Lipoxygenases have also been implicated in the generation of off-flavors in rice, which can occur during grain storage. The release of free fatty acids can be detected in stored grain, which is indicative of the metabolism of the triglycerides reserves. The rice variety Daw Dam was found to accumulate lower levels of pentanals and hexanals giving a better flavor stability on storage (Susuki et al., 1999, J. Agric. Food Chem., 47: 1119-1124). This desirable phenotype was attributed to the absence of rice lipoxygenase-3, which oxidises unsaturated lipid acyl chains to form 9-hydroxyperoxyoctadecanoic positional isomers.

It is recognised that the lipoxygenase pathway is complex with many branches and its role in numerous aspects of plant growth and physiology are not fully understood. Modifications of the lipoxygenase pathway which alter 9-hydroperoxidation activity in seed crops are proposed to regulate their susceptibility to mycotoxin contamination by Aspergillus spp. (WO 9726364), which is consistent with the involvement of this pathway in plant pathogen resistance, but is not related to the aims of the invention herein described.

Among the many aroma volatiles which contribute to the flavor of beer, the higher unsaturated aldehydes with a 6-12 carbon chain have particularly low organoleptic flavor thresholds (Meilgaard 1975, MBAA Tech. Quart. 12: 151-168). Trans-2-nonenal, which is a member of this group, has both an extremely low flavor threshold of 0.11 ppb and contributes an unpleasant straw-like, "cardboard" flavor to the beer. The characteristic off-flavor caused by trans-2-nonenal is a common characteristic of beers stored for 1-3 months or more and is particularly detrimental to the flavor of lager beer, which is brewed with light malts and has a delicate flavor.

Sulfite has long been known to improve the flavor stability of beer, not only by binding oxygen and acting as an anti-oxidant, but also by forming volatile bisulfite addition compounds with aldehydes and ketones present in the beer. The two major sources of sulfite in beer are sulfite produced by yeast during fermentation via the sulfur assimilation pathway and sulfite added to the beer prior to packaging. Fermentation conditions that enhance yeast sulfite production and secretion will allow the formation of sulfite-carbonyl adducts from carbonyls present in the wort and prevent their further metabolism by the yeast (Dufour 1991, Proc.Eur. Brew. Conv. Congr., Lisbon, pp. 209-216). In this manner carbonyls such as acetaldehyde and diacetyl may be transferred to the beer. The ability of sulfite to prevent the appearance of the carbonyl compound *trans*-2-nonenal during beer aging has been demonstrated by brewing beer with a yeast strain blocked in the sulfur assimilation pathway (Johannesen et al., 1999, Proc.Eur. Brew. Conv. Congr., Nice, pp. 655-662). Following bottling, the beer was subjected to forced aging by storing it at 37°C for 7 days, after which trans-2-nonenal levels were found to be well above the taste-threshold. If 10 ppm sulfite was added to the low-sulfite beer just prior to bottling, the appearance of *trans-2-*nonenal during forced aging was significantly reduced. The reaction between sulfite and carbonyl compounds is reversible and under thermodynamic and kinetic control. The apparent equilibrium constants for bisulfite compounds ranges from 10⁻⁶ M for carbonyl compounds such as acetaldehyde, hexanal, and decanal, to 10⁻³ for diacetyl and pyruvate (Dufour 1991, *supra).* During beer storage, gas exchange through the packaging will allow oxygen into the beer and sulfite will be lost, such that weaker bisulfite adducts will dissociate, allowing free carbonyls to appear in the beer. While sulfite unquestionably enhances the flavor-stability of beer, particularly in the short-term, its retention in packaged beer is strongly dependent on gas exchange through the packaging and temperature. In a finished beer the natural levels of sulfite produced during fermentation are variable and the addition of sulfite prior to bottling is not a universally accepted practice. For these reasons sulfite alone does not provide a reliable method to enhance the long-term flavor-stability of beer under the different beer storage conditions used around the globe.

It is generally accepted that the trans-2-nonenal found in beer results from the oxidation of polyunsaturated fatty acids derived from barley grain lipids, where the 18-carbon chain fatty acid, linoleic acid [classified as an 18:2,n-6 polyunsaturated fatty acid (Broun, Gettner and Sommerville 1999, Annu. Rev. Nutr. 19: 197-216)] is the most abundant. However, there is little agreement in the literature as to the mechanism whereby *trans*-2-nonenal is formed. The presence of enzymatic pathways leading to *trans*-2-nonenal formation from poly-unsaturated fatty acids has been proposed, but the individual enzymatic steps have never been demonstrated experimentally in barley grain or during the malting process (Gardner 1988, Adv. Cereal Sci. Technol. 9: 161-215). The concept of using anti-sense or co-suppression gene technology to reduce lipoxygenase-1 levels in barley grain, and thereby control 9-hydroperoxidation and reduce aldehyde and alcohol levels in the finished barley grain, has been proposed as a means to control off-flavor formation, but results of such an approach are not reported (McElroy and Jacobsen, 1995, Bio/Technology 13: 245-249).

A forcing test has been developed as a method for assessing the *trans*-2-nonenal potential of a beer, where *trans*-2-nonenal formation in wort or beer is induced by subjecting samples to elevated temperatures at reduced pH, (100°C, at pH 4.0 for 2 hours). Attempts to correlate the *trans*-2-nonenal potential in wort and finished beer with the total level of lipoxygenase activity in the kilned malt have indicated that lipoxygenase may contribute to the appearance of *trans*-2-nonenal in aged beer (Drost et al., 1990, J. Am. Soc. Brew. Chem. 48: 124-131). The conclusions that can be drawn from this study, however, are severely limited by the fact that the lipoxygenase activity in the barley malt was regulated at the end of the malting process by the degree of enzyme inactivation during kiln drying. Thus, only the effect of the residual malt lipoxygenase activity on the *trans*-2-nonenal potential in the derived wort and finished beer was examined. The study failed to evaluate the lipoxygenases that catalyse the first step in the lipoxygenase enzymatic pathway in the barley grain during development and malting, and their role as determinants of *trans*-2-nonenal levels found in beer. Indeed, the absence of barley cultivars deficient in one or more lipoxygenase isoenzyme has made it impossible to provide convincing evidence for the role of the lipoxygenase pathway in barley malt in controlling the formation of *trans*-2-nonenal. Such experiments are needed to evaluate the contribution of enzymatic, as compared to auto-oxidative/chemical pathways, to the formation of *trans*-2-nonenal in beer. The brewing process involves a high temperature step of wort boiling where these non-enzymatic reactions are proposed to occur (Noël et al., 1999, J. Agric. Food Chem. 47: 4323-4326).

### SUMMARY OF THE INVENTION

This invention provides a barley cultivar having greatly reduced lipoxygenase-1 activity. In one embodiment, the barley plants of the invention contain a mutant *lox*-1 gene expressing greatly reduced levels of the isoenzyme lipoxygenase-1. In an alternative embodiment, the barley plants contain a heterologous nucleic acid sequence expressing an antisense sequence to the wild-type *lox*-1, thereby reducing the enzyme's activity.

As shown herein, malt and wort produced from the reduced lipoxygenase barley of the invention, for example, from barley cultivars homozygous for a mutant *lox*-1 gene, are useful to produce beer with significantly enhanced flavor stability and reduced *trans*-2-nonenal levels, particularly under conditions known to promote the appearance of T2N. The invention demonstrates a correlation between the activity of barley malt lipoxygenase-1 to produce 9-hydroxyperoxy-octadecadienoic acids (9-HPOD), and the presence of *trans*-2-nonenal in beer. The invention further demonstrates that the use of barley homozygous for the mutant *lox*-1 gene in the brewing process improves the flavor stability of the beer, both during storage and on exposure to elevated storage temperatures. These properties enhance the quality of the beer, and are useful to extend its shelf-life and reduce the need to cool beer during transport and storage.

The invention provides barley plants and portions thereof having reduced lipoxygenase-1 activity, including barley plants expressing mutant LOX-1 protein as described herein, as well as methods for producing such barley plants, plant portions, products of the plants, and particularly malt and beer products produced from the barley plants of the invention.

### BRIEF DESCRIPTION OF THE DRAWINGS

Figure 1 is a graph showing the effect of the inhibitor nordihydroguaiaretic acid (NDGA) on immuno-affinity purified lipoxygenase land 2 activity from embryos of 3 day germinated barley grain.
Figure 2 is a graph showing the fresh weight of developing grain of Line G and cv Vintage from 5 days after flowering to full-maturity (FM). Each determination is the mean single grain weight from 6 spikes.
Figure 3 is a graph showing the dry weight of developing grain of Line G and cv Vintage from 5 days after flowering to full-maturity (FM). Each determination is the mean single grain weight from 3 samples of 5 grain.
Figure 4 is a graph showing total lipoxygenase activity in developing grain of Line G and cv Vintage from 5 days after flowering to full-maturity (FM).
Figure 5 is a graph showing 9- and 13-HPOD products of linoleic acid oxidation by lipoxygenase activity in developing grain of Line G.
Figure 6 is a graph showing total lipoxygenase activity in embryos of germinating grain of Line G and cv Vintage expressed as µ mol/min/10 embryos (U/10 embryos).
Figure 7 is a graph showing 9- HPOD and 13-HPOD products of linoleic acid oxidation by lipoxygenase activity in embryos of germinating grain of Line G and cv Vintage, showing levels of 9- HPOD and 13-HPOD.
Figure 8 is a Western blot showing immunodetection of lipoxygenase 1 in embryos of developing grain of Line G and cv Vintage [wt] from 5 days after flowering to full-maturity (FM).
Figure 9 is a Western blot showing immunodetection of lipoxygenase 1 in embryos of grain of Line G and cv Vintage [wild-type] germinated for 0 - 6 days.
Figure 10 is a Northern blot probed with the 3' non-transcribed region of the *lox*-1 cDNA and showing lipoxygenase 1 transcripts detected in developing grain of Line G and cv Vintage [wild-type] from 5 days after flowering to full-maturity (FM).
Figure 11 is a Northern blot probed with the 3' non-transcribed region of the *lox*-1 cDNA and showing lipoxygenase 1 transcripts detected in embryos of grain of Line G and cv Vintage [wt] germinated for 0 - 6 days.
Figures 12A-12G are a nucleotide sequence alignment of the promoter and transcribed region of the *lox*-1 wild-type cv Vintage allele (WT) and the Line G allele (LG). The transcription start site (+1), ATG start codon (+69) and translation stop codon (+4231) in the gene sequences are underlined. Nucleotide mutations identified in the Line G allele are shown in bold italics and indicated by an asterisk.
Figure 13 is a schematic presentation of the *lox*-1 gene of cv Vintage (wild-type) and the mutant *lox*-1 gene of Line G. The transcript from +1 to +4375 is composed of 7 exons (stippled boxes) and 6 introns (white boxes). Two mutations in the *lox*-1 gene are indicated.
Figure 14 is a schematic drawing of gene cassettes for transient expression of the wild-type *lox*-1 cDNA and *lox*-1 gene and the mutant *lox*-1 gene from Line G. The lipoxygenase coding sequences were cloned between the constitutive maize ubiquitin promoter with intron 1 (*Ubi-1)* and the *nos* terminator.
Figure 15 is a bar graph showing Lipoxygenase 1 activity in barley aleurone protoplasts transfected with gene cassettes containing the wild-type *lox*-1 cDNA; the mutant *lox*-1 gene from Line G; WT *lox*-1 gene; and a control GUS reporter gene. Lipoxygenase activity in extracts of transfected protoplasts was assayed in microtiter plates by the oxidation of KI and quantitated spectrophotometrically. Lipoxygenase 1 activity was expressed as units per µg protein in the extract and is shown as the mean of 3 measurements from 2 replicate assays.
Figure 16 is a sequence alignment demonstrating that a RFLP between the wild-type and mutant *lox*-1 gene is due to a point mutation at nucleotide 2347, creating an additional *Aat*II restriction site.
Figure 17 is a schematic presentation of the *lox*-1 PCR fragments amplified and cleaved in the polymerase chain reaction - cleavage amplified polymorphic site (PCR-CAPS) assay. The positions of PCR primers are indicated by arrows and the *Aat*II sites are shown above the gene (sequence position). The exon and intron regions within the PCR product are distinguished by stippled and white boxes respectively, and the sizes of the *Aat*II digestion fragments are given.
Figure 18 is an electrophoretic agarose gel showing *lox*-1 PCR fragments (652 bp) amplified in the first step of the PCR-CAPS assay from Line G and cv Vintage genomic DNA.
Figure 19 is an electrophoretic agarose gel showing RFLP detected by PCR-CAPS in the wild-type and mutant *lox*-1 gene. The *Aat*II digestion fragments of the mutant gene include a unique 313 bp restriction fragment, indicated by an asterisk.

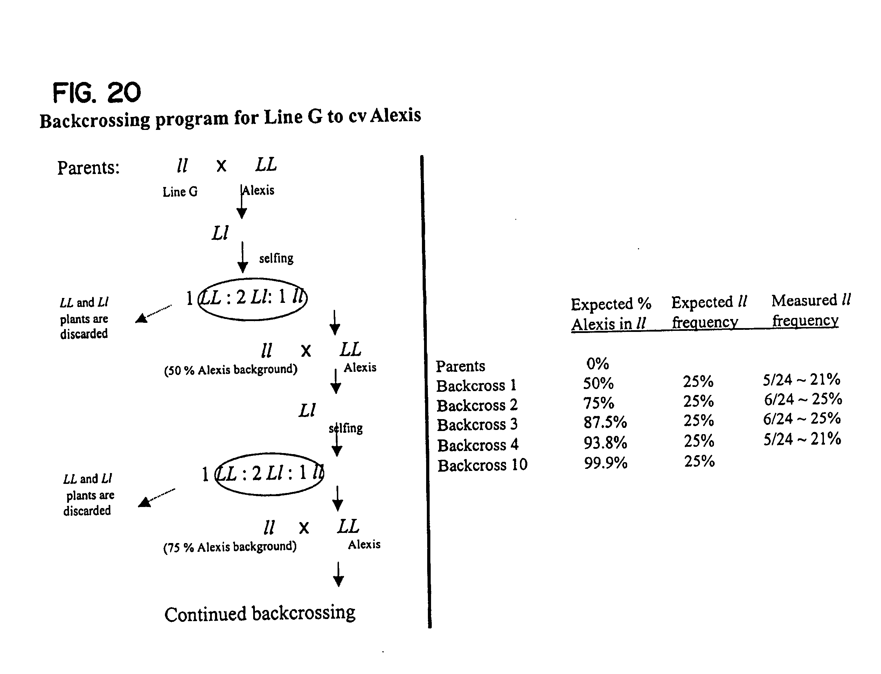

Figure 20 is a table showing a back-crossing program for the single recessive gene pair *ll* (low lipoxygenase trait) of Line G to cv Alexis. The *LL* genotype are plants expressing wild-type lipoxygenase activity (dominant allele), the *ll* genotype are plants expressing the low-lipoxygenase (recessive allele). *Ll* are heterozygous plants containing both the wild-type and the low-lipoxygenase allele. Since the low-lipoxygenase trait is a recessive trait, *Ll* plants show wild-type lipoxygenase activity. After each round of back-crossing (including self-pollination), the *ll* progeny is expected to represent 25% of the progeny. The observed frequencies of low-lipoxygenase activity are indicated. The calculated percentage of the cv Alexis genetic background having the homozygous low-lipoxygenase allele is indicated as % Alexis.
Figure 21 is an electrophoretic agarose gel showing PCR-CAPS detection of the mutant *lox*-1 gene in *ll* progeny of the Line G - Alexis back-cross program. PCR-CAPS assay on genomic DNA of Line G (Lane 2), cv Vintage (Lane 3),*ll* progeny of 3^{rd} (Lane 4) and 4^{th} back-cross (Lanes 5 - 9). DNA ladder (Lane 1). Control, backcrossed high lox line (lane 10).
Figures 22A-22B are a comparative alignment of amino acid sequences of soybean lipoxygenases LOX-1 (Gml), LOX-2 (Gm2), LOX-3 (Gm3), and barley lipoxygenases LOX-1 (Hvl) and LOX-2 (Hv2).

### DETAILED DESCRIPTION OF THE INVENTION

In accordance with the subject invention, plant materials, plant products, and methods are provided for producing a beverage, such as beer, the beverage having a reduced content of the off-flavor compound *trans*-2-nonenal, such that the flavor stability of the beverage, e.g., beer, during storage and on exposure to elevated temperatures is improved, relative to a control beverage. More particularly, the invention provides barley varieties whose developing and germinating grain produce greatly reduced activity levels of the enzyme lipoxygenase-1, denoted LOX-1, which, for example, when used in a beer brewing process, results in a beer having reduced *trans*-2-nonenal levels, as compared with a control barley variety.

The methods used to generate, characterize, and validate a barley variety having greatly reduced LOX-1 activity, and use of this type of barley for the production of flavor-stable beer are described below.

### 1. Definitions

As used herein, the following terms have the indicated defmitions:
"Plant portion" means a plant or specific part of a plant, such as the stem, leaves, roots, flowers, seeds, grains, fruits, or buds.
"LOX-1" means lipoxygenase-1 protein; "*lox*-1" means the gene encoding LOX-1.
"Mutant barley *lox*-1" means a mutagenized barley gene encoding a mutant lipoxygenase 1 polypeptide.
"Non-mutated control" means a plant, nucleic acid, gene, polypeptide, plant portion, or plant product containing wild type gene or protein.
"Heterologous" means a non-native sequence, e.g., a sequence derived from another species, or a recombinantly engineered or synthetic sequence that differs from the native sequence.
"Plant product" means a product resulting from the processing of a plant or plant portion, and includes, for example, malt and wort.
"Acidic amino acid" means aspartic or glutamic acid.
"Basic amino acid" means histidine, lysine, or arginine.
"Polar amino acid" means threonine, serine, tyrosine, tryptophan, asparagine, or glutamine.
"Organoleptic properties" means properties appealing to the olfactory and taste senses that are analysed, for example, by a trained taste panel.
"Brewed product" means a product prepared by mashing, boiling, and fermenting, e.g., beer.
"Reduced trans-2-nonenal" means less than about 50%, as compared with wild-type (control) conditions.

### 2. Lipoxygenase Activity

Lipoxygenase enzymes catalyze the oxidation of polyunsaturated fatty acids. In barley, the isoenzymes LOX-1 and LOX-2 are known. LOX-1 primarily catalyzes 9-hydroperoxidation, whereas LOX-2 primarily catalyzes 13-hydroperoxidation of polyunsaturated octadecanoic fatty acids. The data shown in the Examples below demonstrates a correlation between barley LOX-1 9-hydroperoxidation activity and the presence of *trans*-2-nonenal in beer. Accordingly, barley having reduced LOX-1 activity is useful to produce beer having a reduced *trans*-2-nonenal level and/or potential as compared with a control.

### 3. Production of low lipoxygenase barley

A variety of known genetic approaches can be used to produce the plants of the invention, that is, to reduce the level of lipoxygenase 1 enzyme activity expressed in a barley plant in a stable, inheritable manner. These approaches include, but are not restricted to antisense technology and mutagenesis, such as chemical and radiation induced mutagenesis, as well as site-directed mutagenesis.

**Barley transformation.** Barley can be transformed with various nucleic acid molecules designed to manipulate *lox*-1 gene expression or alter the architecture of the *lox*-1 gene. Various methods, for example, *Agrobacterium tumofaciens*-mediated transfer (Tingay et al, 1997, Plant J., 11: 1369-1376), particle bombardment (Wan and Lemaux, 1994, Plant Physiol., 104: 37-48, or polyethylene glycol (PEG)-mediated DNA uptake (Funatsuki and Kihara, 1995, Theor. Appl. Genet., 91:707-712), can be used to successfully introduce nucleic acids into a barley cell, for example into a protoplast, callus, or an embryo.

Various promoters can be used to drive expression of the gene of interest. For expression of *lox*-1-containing vectors, including antisense sequences, the native *lox-1* promoter region can be used. The promoter sequence of *lox*-1 is contained in nucleotides 2602 - 3511, which includes the 5' UTR of EMBL accession no. U83904. Alternatively, promoters that drive expression of the gene of interest constitutively, for example the *Ubi.1* maize ubiquitin promoter, can be used (Wan and Lemaux, *Supra;* Kjærulff et al., in P. Mathis, Ed., 1995, Photosynthesis: from Light to Biosphere, Vol. II, 151-154). Expression vectors can also contain a transcription termination region, for example, the 3' terminator of the nopaline synthase gene (3'*-nos)* (Bevan, et al, 1983, Nucl. Acids Res., 11: 369-385) has been fused to genes expressed in transgenic barley (Wan and Lemaux, *Supra;* Funatsuki and Kihara, *Supra).*

Expression vectors can also contain a gene that allows for selection of transformed cells when the vector has been successfully integrated in the cell. These genes can encode antibiotic or herbicide resistance genes, for example the neomycin phosphotransferase (*npt*) or the phosphinothricin acetyl transferase (*bar*) gene. When expressed, such resistance genes allow for growth of the transformed cell in neomycin - or bialaphos-containing media, respectively (See, for example, Wan and Lemaux, *Supra;* Funatsuki and Kihara, *Supra;* Kjærulff et al., in P. Mathis, *Supra).*

Following transformation, cells can be grown in selective media for a period of time and then cultured to allow for the formation of shoots, followed by root systems, and then plantlets. A successful barley transformation procedure was developed by Funatsuki and Kihara, (Supra), where transformation of barley protoplasts by PEG with neomycin phosphotransferase-containing expression vectors and subsequent selection in neomycin yielded fertile plants containing the transgene. The transgene was shown to integrate into the genome and most of the transgenic plants expressed the protein encoded by the transgene. These transgenic plants also were able to transmit and express the transgene following crosses.

It is understood that a variety of transformation methods, expression vectors, promoters, selectable markers, and the like are known and useful for transformation of barley.

**Barley mutagenesis.** The *lox*-1 gene can be targeted for site-specific mutagenesis using chimeric RNA/DNA oligonucleotides. These chimeric RNA/DNA oligonucleotides have been shown to successfully introduce mutations in plant cells (Zhu et al., 1999, Proc. Natl. Acad. Sci. 96: 8768-8773; and Beetham et al., 1999, Proc. Natl. Acad. Sci. 96: 8774-8778) and mammalian cells (Yoon et al., 1999, Proc. Natl. Acad. Sci. 93:2071-2076) at desired locations. The chimeric RNA/DNA oligonucleotides can be transformed into the barley protoplasts or cells of interest in a variety of ways, for example using the PEG-mediated or particle bombardment-mediated transformation methods described above. The individual protoplasts or cells can then regenerated by tissue culture to whole fertile plants, and the mutational event can be confirmed and followed, for example using a PCR-based approach as detailed in the Examples below.

This site-directed mutagenesis method can be applied to mutate specific residues in the *lox*-1 gene. The *lox*-1 gene can be mutated at one or more nucleotide position in the promoter region to downregulate or abolish *lox*-1 transcription. Specific mutagenesis can also be applied to introduce changes in the *lox*-1 coding region that, for example, reduce the enzyme's activity. Such mutations include, but are not limited to, insertions, deletions, and substitutions resulting in a frameshift, truncation of the LOX-1 protein, and/or alteration of the neutral and hydrophobic nature of the enzyme's substrate cavity.

**Antisense expression.** Reduction in *lox*-1 expression can also be accomplished by expression of a *lox*-1 antisense construct in the barley cells. Methods for the expression of antisense constructs in barley to reduce the expression of a targeted protein have been reported, for example, in Gilpin, M.J. et al., 1998, In: Photosynthesis: Mechanisms and Effects, G. Garab, ed., Vol. IV, 2983-2986; Kjærulff et al., 1995, In: Photosynthesis: from Light to Biosphere, P. Mathis, Ed., Vol. II, 151-154.

Barley cells can be transformed with an expression construct containing an antisense nucleic acid sequence. The expression construct produces an antisense RNA molecule capable of specifically binding to at least a portion of the mRNA produced from the wild type *lox*-1 gene, through complimentary base pairing, and capable of disrupting the splicing of the pre-mRNA or translation of this mRNA. A constitutive or tissue/temporal specific promoter, for example, the barley *lox*-1 promoter described above, can drive expression of the antisense nucleic acid sequence.

**Chemical mutagenesis.** The chemical mutagen sodium azide (NaN₃) has commonly been used for barley mutagenesis and is known to induce stable mutations in the DNA (deoxyribonucleic acid) sequence of the barley genome (Olsen et al., 1993, Proc. Natl. Acad. Sci. USA, 90: 8043-8047). Other chemical mutagens, for example, ethyl methanesulfonate (EMS), azidoglycerol (AG, 3-azido-1,2-propanediol), methyl nitrosourea (MNU), and maleic hydrazide (MH) can also be used to induce DNA mutations (Rank, J. et al., 1997, Mutat. Res. 390:121-7), as can UV irradiation.

As shown in the Examples below, the grain of the barley cultivars (cv) Vintage and Caruso were treated with sodium azide and propagated by self-fertilization through to the 3^{rd} generation (M3).

### 4. Identification and Selection of Low Lipoxygenase Barley

Identification and selection of barley plants having reduced lipoxygenase isoenzyme activity in the grain can be achieved, for example, by analysis of lipoxygenase activity. Enzymatic assays can be used to determine the activity of the two major lipoxygenases known to be present in either mature or germinating grain, LOX-1 and LOX-2. Such assays should distinguish LOX-1 activity from that of LOX-2.

One selective assay of LOX-1 and LOX-2 is based on the oxidation of a poly-unsaturated fatty acid by lipoxygenase and the spectrophotometric detection of the hydroperoxide product of such oxidation. The specificity of this assay for LOX-1 takes advantage of the comparative insensitivity of LOX-1 to an inhibitor, for example, NDGA, relative to LOX-2.

Selective assay can also be achieved using immunoprecipitation to selectively remove LOX-1 or LOX-2 from the assay. Specific anti-LOX-1 and anti-LOX-2 antibodies, for example, monoclonal antibodies, can be prepared from purified LOX-1 or LOX-2 as described in Holtman et. al, 1996, *Supra.*

These assay methods can be adapted for microtiter plate assay procedures, or other known repetitive, high throughput assay formats, allowing the rapid screening of many samples. These assays can be validated for screening leaf tips of germinating grain in a non-destructive manner, such that seedlings selected in the screen can be further propagated.

The loss of LOX-1 activity in putative mutants can be confirmed by assay of enzymatic activity. For example, grain extracts can be incubated with linoleic acid and the oxidation products of linoleic acid analyzed, for example, by reverse phase HPLC. The relative amounts of 9-HPOD and 13-HPOD formed from linoleic acid provides a measure of LOX-1 activity, whose major product is 9-HPOD.

As shown in the Examples below, approximately 20,000 grain of the M3 generation of mutagenized cv Vintage and cv Caruso were screened for LOX-1 and LOX-2 activity by oxidation assay in the presence of inhibitor and also by immunoprecipitation assays. Using these screening methods, a mutant in cv Vintage was found having a major reduction in LOX-1 activity, and was denoted Line G. The mutant phenotype was inherited in the M4 and M5 generations.

Seed produced from the Line G barley was deposited on January 4, 2001, with the National Collections of Industrial, Food and Marine Bacteria (NCIMB), 23 St. Machar Drive, Aberdeen, AB243RY, Scotland, UK, under the terms of the Budapest Treaty, as Accession Number: NCIMB 41078.

### 5. Genetic Sequences

A precise description of the genotypic alteration that accounts for the low-lipoxygenase phenotype in barley plants of the invention is useful for identifying plants having this genetic alteration and for crossing this genetic character into other barley cultivars in a breeding program. A variety of known molecular and biochemical methods can be used to determine the genetic basis for the low lipoxygenase phenotype.

It is generally recognized that both cis-acting and trans-acting genetic sequences can determine the expression of a given gene in the genome and the activity of the gene product. Control points in gene expression include the regulation of the timing, tissue-specificity and rate of gene transcription, the stability of the transcript and the rate of transcript translation. Both the level of gene expression and the stability and specific activity of the encoded enzyme will determine the level of enzyme activity detected in a tissue.

Alterations in a plant gene sequence can be determined by DNA sequencing of known relevant parts of the genome, while Northern analysis provides a tool to monitor stable transcript levels in a given plant tissue. Enzyme expressed in plant tissue can be evaluated by extracting the enzyme from the tissue and measuring the enzymatic activity.

As shown in the Examples below, the identity of the genetic changes that determine the low-lipoxygenase phenotype of the Line G mutant induced in cv Vintage were determined in the following manner. The structural gene encoding the LOX-1 protein, both in the parent cv Vintage and in the Line G, was amplified by the polymerase chain reaction (PCR), and the upstream promoter sequences, which regulate expression of the gene, as well as the entire coding sequence, comprising intron and exon sequences, were sequenced.

Comparison of the nucleotide sequences of the *lox*-1 gene from Line G and from wild-type cv Vintage revealed 2 nucleotide substitutions in 2 exons, of which one (at position + 2347) led to a non-conservative amino acid substitution (Glycine³⁶⁸→ Aspartate) in the expressed protein.

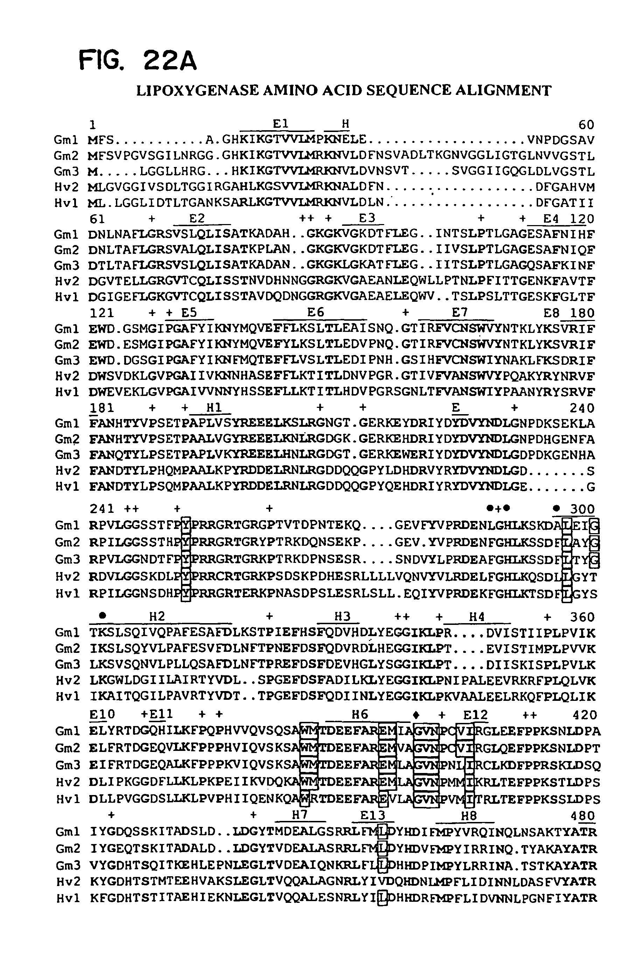

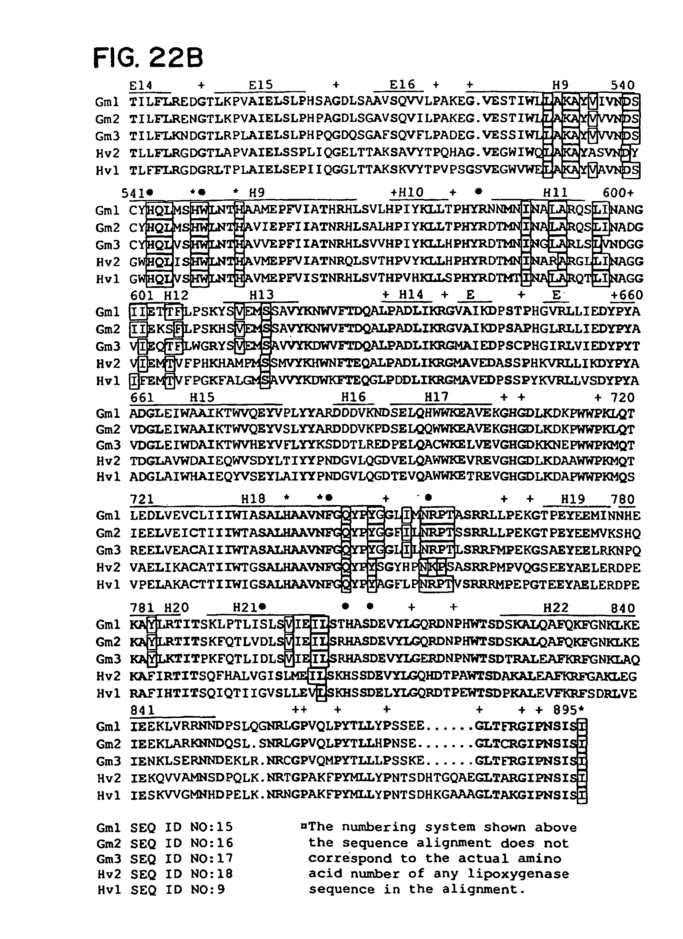

Figure 22 shows an alignment of soybean (Gm: *Glycine max L)* lipoxygenases LOX1 (Acc. No. P08170), LOX2 (Acc. No. P08170), LOX3 (Acc. No. AAB41272) and barley (hv: *Hordeum vulgare)* lipoxygenases LOX1 (Acc.No. P29114) and LOX2 (Acc. No. AAB70865.1). Conserved amino acid residues and conservative substitutions of charged residues are shown in bold. Secondary structure assignments for LOX3 of soybean *Glycine max,* where H=alpha helices and E=Beta strands, are shown above the alignment, and residues relevant to enzyme function (identified by an asterix or filled circle) are shown, as described in Skrzypczak-Jankun et.al., 1997, Proteins 29:15-31.

Amino acid residues that participate in non-heme iron binding or essential for catalysis (*) in soybean LOX3 include: H₅₁₈, H₅₂₃, H₇₀₉ [3 N atoms]; N₇₁₃. I₈₅₇. The equivalent residues in barley LOX 1 are H₅₁₇, H5₂₂, H₇₀₈, N₇₁₂. and I₈₆₂. Residues in soybean LOX3 with a predicted role in catalysis(•) are: H₂₆₆, H₅₁₃, H₇₇₆, F₂₆₄, F₂₇₂, F₇₁₄, W₅₁₉, R₅₅₂,R₇₂₆, D₇₆₆, D₇₇₉, K₂₇₈. The equivalent residues in barley LOX1 are: H₂₆₁, H₅₁₂, H₇₇₅, F₂₅₉, F₂₆₇, F₇₁₃, W₅₁₈, R₅₅₁, R₇₂₅, D₇₇₈, and K₂₇₃.

Proline P₈₆, _{109, 167**,** 171**,** 223, 234, 291, 311, 342, 343, 345, 371,381, 382, 486, 541, 548, 600, 616, 627, 685, 726, 734, 788, 829,833, 839, 857,} and glycine (G_{49, 67, 68, 70, 91, 107, 137, 187, 192, 210, 217, 218, 260, 306, 307,} 336$ _{392, 409, 458, 474, 490, 569, 607, 674, 676, 720, 736, 783, 828, 850,} 8ss) residues (+) located in loops and helix-capping positions in protein secondary structures, may facilitate sharp turns and folding of the peptide backbone.

Alignment of related plant lipoxygenases indicated that the Glycine-368 in barley LOX-1 is strongly conserved. Furthermore, this residue, which corresponds to Glycine-353 in soybean LOX-1, is one of 35 highly-conserved residues out a total of 58 residues that line the substrate cavity II of the enzyme, as seen from its crystal structure. These conserved residues are highlighted (boxes) in the alignment of plant lipoxygenase sequences shown in Figure 22 (Minor et.al., 1996, Biochemistry 35:10687-10701), and include the following barley LOX-1 residues: Y₂₂₄, L₁₆₈, W₃₅₅, E₃₆₄, G₃₆₈, V₃₆₉, N₃₇₀, I₃₇₄, L₄₂₄, L₄₉₉, K₅₀₁, A₅₀₂,V₅₀₄, D₅₀₈, S₅₀₉, H₅₁₂, Q₅₁₃, L₅₁₄, H₅₁₇, W₅₁₈, H₅₂₂, I₅₅₆, L₅₅₉, A₅₆₀, L₅₆₄, I₅₆₅, I₅₇₀, T₅₇₄, S₅₈₅, Q₇₁₅, Y₇₁₈, N₇₂₄, R₇₂₅, P₇₂₆, T₇₂₇, L₇₇₂, and I₈₆₂, All but 7 of the 35 conserved residues are neutral or hydrophobic residues. The substitution of a charged residue at position Glycine - 368 in barley or at another conserved neutral or hydrophobic residue lining the substrate cavity II, is likely is likely to disturb the structural and functional properties of the enzyme. The G→D₃₆₈ mutation in barley Line G LOX1( ◆)is located between alpha-helix H6 and beta-strand E12.

As shown in Figure 22, the lipoxygenase family of enzymes shares a high degree of sequence conservation, which is reflected in their conserved secondary structure, determined for several members of the plant lipoxygenase family including soybean LOX1 and LOX3 (Skrzypczak-Jankun *et al.,* 1997, *supra*). Barley LOX1 shares 56.9% sequence identity and 67.8% sequence similarity with soybean LOX3. Several amino acid residues in the soybean LOX3 isoenzyme have been identified as ligands for the non-heme iron, or are suggested to be essential for its activity (denoted by * ●). In view of the high sequence conservation between the barley LOX1 and the soybean LOX3, it is reasonable to predict that residues in the barley LOX1 sequence that are homologous to those identified as important for the function of LOX3 may also be essential for enzymatic activity. Thus, non-conservative amino acid substitutions at any of these positions, including substitutions of those residues in barley LOX1 corresponding to the 35 highly conserved residues of soybean LOX3 that line the substrate cavity and in other positions essential for enzyme activity, are likely to reduce lipoxygenase activity.

The amino acid residues proline and glycine are known to facilitate turns in a peptide backbone when they are located between secondary structural elements, which allow a protein to assume a folded tertiary structure. Proline and glycine residues are also common in helix capping motifs (Parker and Hefford, 1997, Protein Eng., 10: 487-496, http://www.expasy.ch). The single non-conservative substitution in Line G LOX1, where a glycine located between two predicted structural elements was replaced by aspartate, led to a significant loss of enzyme activity. It is thus predicted that mutation in the *LOX*-1 gene causing a non-conservative amino acid substitutions at one or more of the proline or glycine residues in the barley LOX1, located in regions outside the structural elements, may similarly prevent folding of the native protein and consequently reduce the activity of the encoded enzyme.

Thus, in one embodiment, a useful mutant barley plant of the invention having reduced lipoxygenase 1 activity contains a mutated nucleic acid sequence that alters the neutral or hydrophobic nature of the substrate cavity of the enzyme by insertion of one or more acidic, basic, or polar amino acids. For example, a useful nucleic acid sequence [SEQ ID NO: 11 encodes a barley LOX-1 protein [SEQ ID NO: 12] having a substitution at amino acid 368 from Glycine to Xaa, where Xaa is an acidic, basic, or polar amino acid. One specific amino acid sequence of the barley mutant LOX-1 of the invention is that where Xaa is aspartic acid, e.g., Line G.

As shown in the Examples below, the genotypic changes in Line G had no detectable influence on *lox*-1 gene expression, but the LOX-1 activity detected in mature and germinating grain of Line G were approximately 9% of that detected in grain of the parent line, cv Vintage. In order to provide direct evidence that the amino acid mutation in LOX-1 of Line G was responsible for the low-LOX-1 phenotype, the coding sequence of Line G *lox*-1 and cv Vintage *lox*-1 were expressed transiently in protoplasts from barley aleurone, and the activity of the mutant LOX-1 enzyme was shown to be strongly reduced in comparison to the wild-type LOX-1 enzyme.

### 6. Transfer between breeding lines

The detection of alterations in genetic character of the barley plants of the invention genotype is useful to identify the presence of a specific genetic character in a barley line, and to facilitate the transfer of this character between breeding lines in a breeding program. A variety of molecular tools are available for the detection of alterations in genomic sequence. Such methods include, but are not restricted to, detection of restriction fragment length polymorphisms (Gebhardt and Salamini 1992, Int. Rev. Cytology., 135: 201-237) and quantitative PCR based detection methods such as amplification using fluorescent primers, e.g. the TaqMan primer probe systems (Ibraham et al.,1998, Anal. Chem 70, 2013-2017). The choice of detection method will depend on the specific genetic character but should preferably be rapid and provide clearly interpretable data.

As shown in the Examples below, a PCR-Cleavage Amplified Polymorphic Site assay (PCR-CAPS) was provided for the detection of the mutant lipoxygenase-1 gene of Line G. The nucleotide substitution in the *lox*-1 gene in Line G at position + 2347 introduced an additional site of recognition by the *Aat*II restriction endonuclease that can be detected by the PCR-CAPS assay. Suitable detection methods for *lox*-1 are not restricted to this assay, but can equally well be based on TaqMan technology, and other known detection methods.

Also shown in the Examples below, the PCR-CAPS assay was applied to 4 generations of breeding material from a back-cross program, where the low-lipoxygenase phenotype in Line G was systematically back-crossed into cv Alexis. Inheritance of the low-lipoxygenase phentoype was shown to follow the inheritance of the *lox*-1 gene, and the phenotype was identified as recessive and only seen in lines homozygous for the *lox*-1 gene.

Accordingly, plant progeny of the invention includes breeding lines, for example, derived in a back-crossing program, that contain mutant *lox*-1 and express a low lipoxygenase phenotype.

### 7. Brewing

The barley plants of the invention, including plant parts, plant progeny, grain, and plant products such as malt and wort, having low lipoxygenase 1 activity, are demonstrated herein to be useful for the manufacture of a beverage having reduced levels of free *trans*-2-nonenal over a measured period of time, or under conditions of elevated storage temperature, as compared to a beverage produced from a wild-type control barley variety. For the purpose of these comparisons the sulfite content of the beer is controlled to 5ppm or below, since it is recognised that higher sulfite levels at the time of bottling will temporarily delay the appearance of free *trans*-2-nonenal. For example, beer brewed from malt derived from the mutated barley Line G described herein, possessed stabilized organoleptic properties over a measured period of time as compared with beer brewed from malt derived from a control, non-mutated barley.

Brewing trials and evaluation of bottled beer provide the best method for evaluating the influence of different ingredients on the quality and stability of the finished beer. In order to test the influence of different barley malts, sufficient barley grain is needed to perform the malting and brewing trials on a pilot scale and semi-industrial scale. During the period of barley propagation, the field performance of the barley line can be evaluated. The malting properties of a barley line can be evaluated during pilot or industrial scale malting, and should preferably lie within national malting quality recommendations eg. the European Brewing Convention recommendations for malting quality (Analytica-EBC/European Brewing Convention, 1998, Publ. Hans Carl Getränke-Fachverlag, Nürnberg, Germany). Following pilot or semi-industrial scale brewing, the beer is packaged in brown bottles and cooled to 5°C for optimal storage. At this stage the fresh beer can be analysed by trained taste panels able to detect specific beer flavors, including the off-flavor compound *trans*-2-nonenal. Additionally, the beer is chemically analysed for major flavor components including *trans*-2-nonenal. These methods of beer quality analysis are then repeated on the beer following various storage conditions known to reveal the long-term storage stability of the beer, for example, forced aging treatments.

As shown in the Examples below, Line G barley was propagated in the field over several seasons in order to malt 10 tons of this line in an industrial malthouse. The control barley varieties cv Vintage and cv Nevada, both having the wild-type LOX-1 phenotype, were malted under similar conditions. The kilned malt from Line G and the control barley cultivars lay within the specifications required for the semi-industrial brewing trials.

Brewing trials were performed on a 30-hl scale and evaluation of the freshly bottled beers revealed that beers brewed from malt of both Line G and the control cultivars had a *trans*-2-nonenal content below the taste-threshold and were deemed satisfactory by a taste-panel. Two forced-aging treatments, either storage at 37°C for 7 days or storage for 6 to 12 weeks at 30°C, were used to evaluate the flavor-stability of the beer. The flavor-stability of beer brewed from Line G malt were found to be superior to that of control malt, both with respect to taste panel evaluation as well as the level of free trans-2-nonenal, and the improvement was found to be statistically significant.

### EXAMPLES

The present invention is further defined in the Examples below. It should be understood that the Examples, while indicating preferred embodiments, are given by way of illustration only.

### Example 1

### Screening and Selection of Lipoxygenase Isoenzyme Mutants from Mutagenised Barley

### 1. Barley mutagenesis

Grains of barley, *Hordeum vulgare* cv Vintage and cv Caruso, were mutagenised with sodium azide according to a published procedure (Kleinhofs et al., 1978 Mutation Research 51: 29-35). The mutagenesis introduces point mutations in the genomic DNA that, for example, may result in amino acid changes in encoded proteins. The mutated M1 grains were propagated in the greenhouse through two generations, and the M3 grain collected for screening. The observed frequency of single gene trait mutants in the M2 generation, according to Kleinhofs *et al.,* 1978, *supra,* are 1.0-2.7 mutants per 10,000 grain from the M2 generation. Since most gene mutations are recessive and only detectable in the homozygous state, the mutagenized population was screened at the M3 generation where the expected proportion of homozygous mutant grain would be higher. A mutation frequency of 0.9 - 2.3 per 10,000 grain was expected in the mutagenized material at M3.

### 2. A non-destructive assay of lipoxygenase 1 (LOX-1) and lipoxygenase-2 (LOX-2) activity in M3 mutagenized grain

A rapid screening procedure for detection of mutant barley grain with reduced LOX-1 activity was developed with the following criteria: The screening procedure should not prevent propagation of the grain/seedling; the selected grain/seedling tissue should express quantifiable levels of lipoxygenase activity; the assay should distinguish LOX-1 activity from that of LOX-2; and the assay procedure should encompass multiple samples.

The levels of total lipoxygenase activity in different tissues of the germinating grain, namely the shoot, root, and scutellum tissue of embryo and the endosperm were assayed as follows: Extracts of barley seedling tissue were prepared by homogenising the tissue in ice-cold 20 mM Tris-HCI, pH 7.5, containing 2 mM NaN₃ and 0.5 mM phenylmethylsulfonyl fluoride (PMSF), followed by removal of insoluble material by centrifugation at 1000 *g* for 10 minutes. Lipoxygenase activity in 100 µl extract was assayed at 25°C, by addition of 2.9 ml of 20 mM linoleic acid substrate, prepared by dispersing 35 µl linoleic acid (free acid, L-1376, Sigma, USA) in 5 ml H₂O containing 1% Tween 20. The reaction was followed spectrophotometrically, where the rate of increase in absorbance at 234 nm (A₂₃₄nm), due to the formation of conjugated diene in the hydroperoxide product, is proportional to the enzyme activity present. One unit of lipoxygenase activity is defined as Δ A₂₃₄ = 0.001 per minute in a 3-ml reaction, equivalent to the oxidation of 0.12 µmole linoleic acid.

The leaf tissue of grain germinated for 4 days in the dark had the highest detected levels of lipoxygenase activity (Holtman et al., 1996, Plant Physiology 111: 569-576). Leaf tips from 4-day seedlings were thus selected for the non-destructive lipoxygenase screening assay. The pH optimum of total barley lipoxygenase activity was tested between pH 4.5 and pH 9.0 and found to be pH 6.5. Hence a 25 mM HEPES buffer (pH 6.5) containing 0.2 M boric acid was selected for the screening assay.

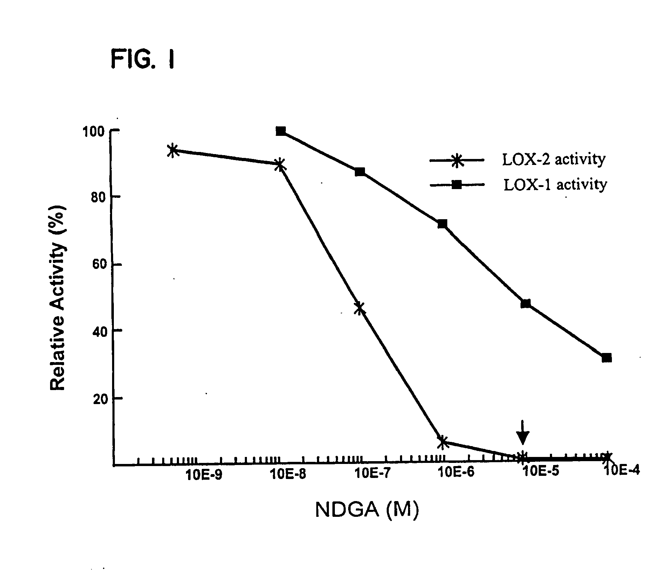

Since both LOX-1 and LOX-2 enzymes were immuno-detected in shoots of 4-day seedlings (Holtman *et al.,* 1996, *supra),* a LOX-1 and LOX-2 specific assay was used. The lipoxygenase inhibitor nordihydroguaiaretic acid (NDGA), identified by Eskin et al., 1977, Crit. Rev. Food, Science and Nutrition 9: 1-40, was found to be a selective inhibitor of barley lipoxygenases. NDGA at 1x10⁻⁵ M strongly inhibited purified barley LOX-2, while LOX-1 retained 47 % activity **(****Figure 1****)**. The selectivity of this inhibitor was tested in the leaf tip assay, by determining the ratio of 9-hydroperoxyoctadecanoid (9-HPOD) to 13-hydroperoxyoctadecanoid (13-HPOD), which result from linoleic acid oxidation by LOX-1 and LOX-2, respectively. In the lipoxygenase assay of cv Vintage leaf tips, the proportion of 13-HPOD formed fell from 24.5% to 9.5% on addition of 1-10⁻⁵ M NDGA.

A selective assay for LOX-2 activity in leaf tip extracts was based on the use of LOX-1-specific monoclonal antibody (5D2) (Holtman *et al.,* 1996, *supra)* to immunoprecipitate LOX-1 present in the extracts. The residual lipoxygenase activity detected in the extracts after LOX-1 precipitation provided a measure of LOX-2 activity. The efficiency of this immunoprecipitation (described below) was evaluated by quantifying the residual LOX-1 and LOX-2 in the extract supernatant by ELISA assay, using specific monoclonal antibodies against LOX-1 (denoted 5D2) and LOX-2 (denoted 5.8) (Holtman *et al.,* 1996, *supra).* LOX-1 immunoprecipitation from extracts of cv Vintage leaf tips removed 85% of (LOX-1) protein and 15% of LOX-2 protein.

Immunoprecipitation was performed in a V-bottom 96-well plate by adding 5 µl 5D2-coated Dynabeads (Dynal) and 75 µl buffer [20 mM Tris-HCI pH 7.5, 1% v/v Bovine Calf Serum (HyClone)] to 20 µl of each leaf tip extract. The plate was incubated on a titerplate shaker (MTS4, IKA, Labor Technik) for 1 hour at 4°C. The immunoprecipitate was pelleted by centrifugation at 4°C in a Sigma 302-K centrifuge for 10 minutes at 2000 rpm. The supernatant (70 µl) from each sample was assayed for lipoxygenase activity in a flat bottom 96 well plate, as described below, but with addition of 100 µl assay buffer (25 mM HEPES, 0.2 M boric acid, pH 6.5).

The LOX-1 and LOX-2 assays were adapted for a high-throughput screening method. Leaf tips (1 cm) from eight 4 day-germinated grains were individually homogenised in 150 µl ice-cold buffer (20 mM Tris-HCl, pH 7.5) for 2 x 30 seconds in a multi-well homogeniser (Berg et al., 1992, Electrophoresis 13: 76-81). After centrifugation for 15 minutes at 3000 rpm, 40 µl of the supernatant of each extract was transferred to a flat bottom 96 well plate. To each well, 170 µl buffer (25 mM HEPES, 0.2 M boric acid pH 6.5, 1·10⁻⁵ M NDGA) and 10 µl substrate (20 mM linoleic acid) were added and then incubated for 20 minutes at 25°C. The reaction was terminated by the addition of 20 µl saturated potassium iodide solution (KI) and incubated for a further 8 minutes at 25°C. The redox reaction between hydroperoxydienes and KI yields I₂, which was monitored by its extinction maximum at 350 nm in a microplate reader (Multiskan MCC/340).

### 3. Identification of potential lipoxygenase 1 mutants in the M3 and M4 grain of mutagenised barley

Grain of the M3 generation of cv Vintage and cv Caruso was stored at 45°C for 6.5 days to break dormancy, ensuring a 95% germination frequency. M3 grain of cv Vintage (9318) and cv Caruso (9633) was germinated and screened for lines whose LOX-1 activity was 15% or less of wild-type grain. The putative mutant lines (50 cv Vintage and 42 cv Caruso lines) were propagated to the M4 generation, harvested, and the germinated grain re-screened. The mutant LOX-1 phenotype was confirmed in one cv Vintage line and six cv Caruso lines, after measuring the lipoxygenase activity in extracts of 5 leaf-tips from each line. When the LOX-1 and LOX-2 activities in germinating embryos of these 7 putative mutants were examined, only the cv Vintage mutant (denoted Line G) showed a major reduction in LOX-1. In mature quiescent grain, lipoxygenase activity present in the embryo is almost exclusively LOX-1 activity, due to the differential expression pattern of the two isoenzymes (Schmitt and van Mechelen, 1997, Plant Sci. 128: 141-150). The total lipoxygenase activity in extracts of embryos from Line G mature dry grain (M5 generation) was 0.06±0.04 U/mg protein in comparison to 0.74 ±0.44 U/mg protein in cv Vintage embryo extracts, as determined by the spectrophotometric lipoxygenase assay described in section 2 of Example 1. The residual lipoxygenase activity in mature embryos of Line G in both the M4 and M5 generations was found to be approximately 9 % of the parental line.

### Example 2

### Line G is a cv Vintage Mutant with a Low-Lipoxygenase Phenotype

The agronomic properties and mutant phenotype of Line G were analysed in material of the M5 generation. Initial analyses were conducted to confirm that the analysed M5 material was homozygous for the mutant phenotype. The low LOX-1 phenotype in Line G, detected in the M3 generation, could result from a dominant or a recessive mutation. If the Line G selected at the M3 generation was heterozygous for a dominant mutation, then subsequent generations would show segregation for the phenotype. The lipoxygenase activity in 26 individual Line G embryos from quiescent grain of the M5 generation was measured and compared to cv Vintage wild type embryos. The lipoxygenase activity in all Line G embryos was very low, with an average of 0.06 ±0.04 U lipoxygenase per mg protein, compared to 0.74±0.44 U lipoxygenase per mg protein in wild type cv Vintage embryos. These data confirmed that Line G in the M5 generation was homozygous for the low lipoxygenase trait.

### 1. Line G has a wild type plant growth physiology and grain development.

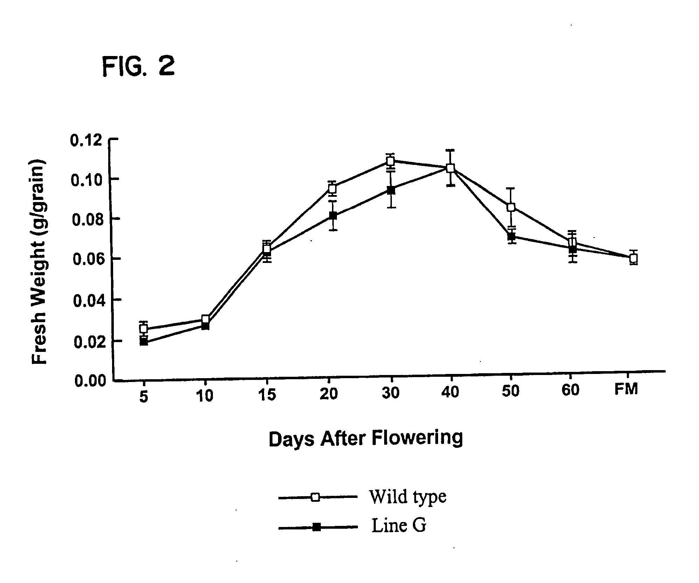

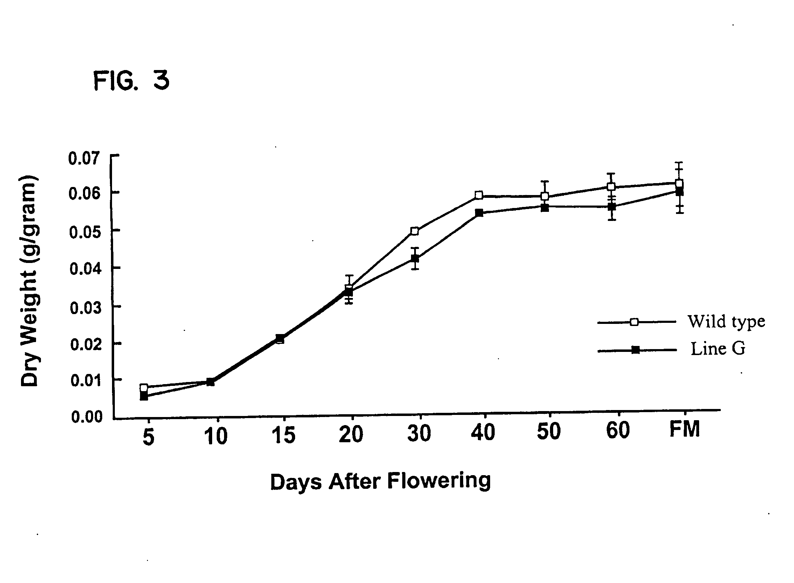

Line G and cv Vintage grain were germinated and grown in a climate chamber under 16 hours light at 15°C and 8 hours dark at 12°C at a relative humidity of 80%. The growth characteristics of Line G and cv Vintage plants were similar with regard to plant height, number of tillers per plant, the onset of flowering and number of grains per spike. The fresh weight (Figure 2) and dry weight (Figure 3) of grain of Line G and wild type cv Vintage during development from 5 days after flowering (DAF) until full maturity, approximately 90 DAF, were very similar.

### 2. Line G grain have a low-lipoxygenase 1 phenotype throughout development

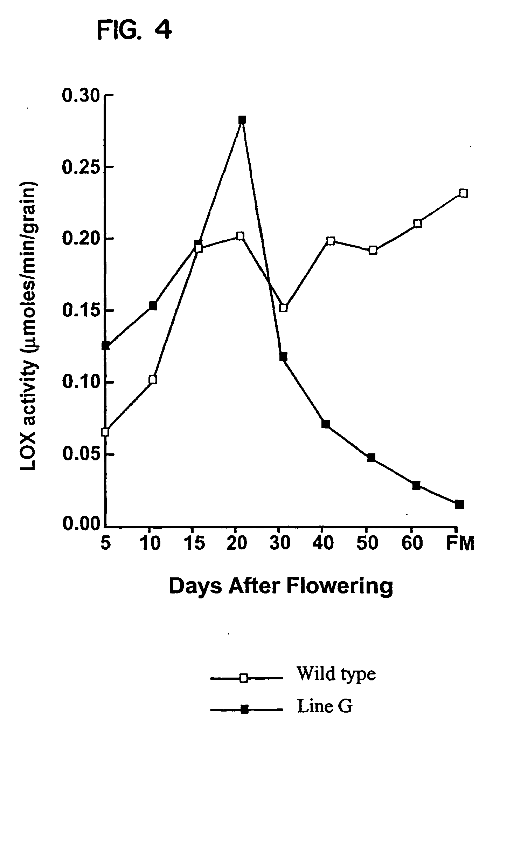

Lipoxygenase activity was measured in extracts of developing barley grain of Line G (M5 generation) and wild type cv Vintage. Grain was homogenised in ice-cold 20 mM Tris-HCl buffer pH 7.5 containing 0.1% (v/v) Nonidet P-40, a non-ionic detergent that enhances lipoxygenase extraction, and centrifuged at 15,000 *g* for 20 minutes to remove insoluble material. Lipoxygenase activity in the extracts was measured polarographically in 200 µl oxygen-saturated buffer (0.2 M boric acid, 25 mM HEPES, pH 6.5) containing 1.2 mM linoleic acid at 25°C, using a Clark-type electrode to measure oxygen consumption. Lipoxygenase activity increased during the first 20 days of grain development in both Line G and wild-type grain, but only in Line G did the activity level fall during grain maturation (Figure 4).

The relative amounts of 9-HPOD and 13-HPOD formed during linoleic acid oxidation provides a measure of the levels of LOX-1 and LOX-2 activity in the grain extracts. In this case Nonidet P-40 was omitted from the grain extraction buffer to avoid the co-extraction of hydroperoxide-consuming enzymes. The extracts (100 µl), mixed with 10 my 50 mM phosphate buffer pH 6.5 containing 200 µM linoleic acid, were incubated for 20 minutes. The reaction was terminated by adjusting the pH to 3.5, and an internal standard was added. The hydroperoxides formed in the assay were bound on an octadecyl solid phase column (Bakerbond, Baker) and eluted with methanol. The 9-HPOD and 13-HPOD were then separated by reverse phase HPLC on a C-18 column with an isocratic elution solvent (tetrahydrofuran:Methanol:H₂O:acetic acid; 25:30:44.9:0.1 (v/v) adjusted to pH 5.5 with concentrated ammonia) at a flow rate of 0.5 ml/minute as described by Aarle et al., 1991, FEBS Letters 280: 159-162. Hydroperoxides were detected at 234 nm and the HPOD peaks were corrected against the internal standard, prostaglandin B2.

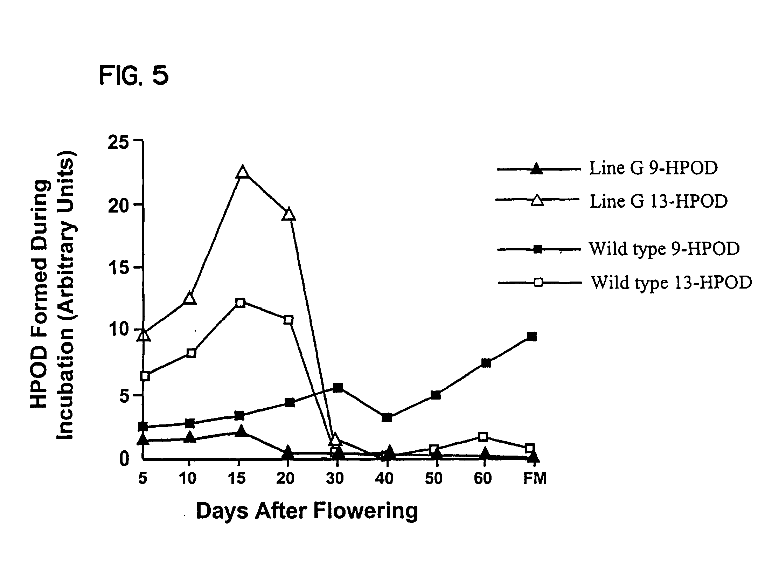

**Figure 5** shows that 13-HPOD was the major product of lipoxygenase activity present in grain during the first 20 DAF, while 9-HPOD was formed by lipoxygenases active during grain maturation. While both Line G and wild-type grain extracts shared a similar profile of 13-HPOD synthesising activity, Line G did not show the wild-type rise in 9-HPOD synthesising activity. These data are consistent with a loss of LOX-1 activity in maturing Line G barley grain.

### 3. Line G grain have a low-lipoxygenase 1 phenotype on germination

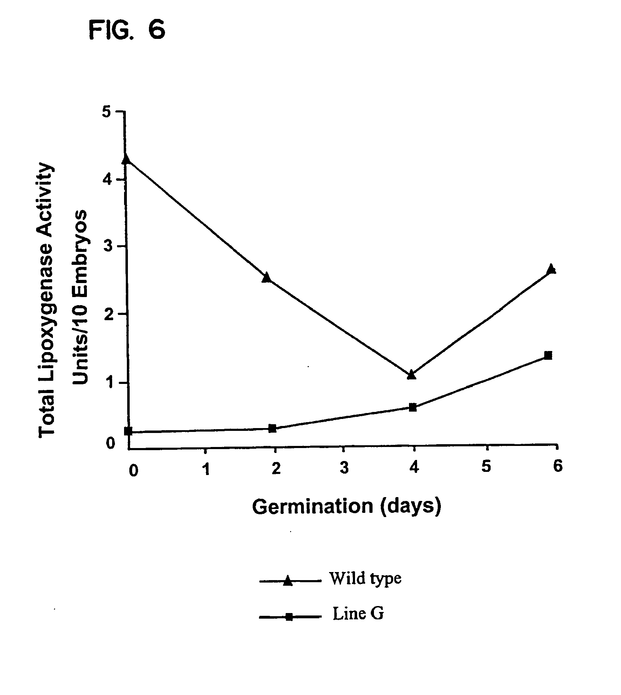

Total lipoxygenase activity in extracts of embryos of grain germinated at 15°C was assayed as described in Example 1. The lipoxygenase activity present in quiescent wild-type grain declined during the first 4 days of germination and then increased (**Figure 6**). In Line G, lipoxygenase activity in quiescent grain was very low but increased after 4 days.

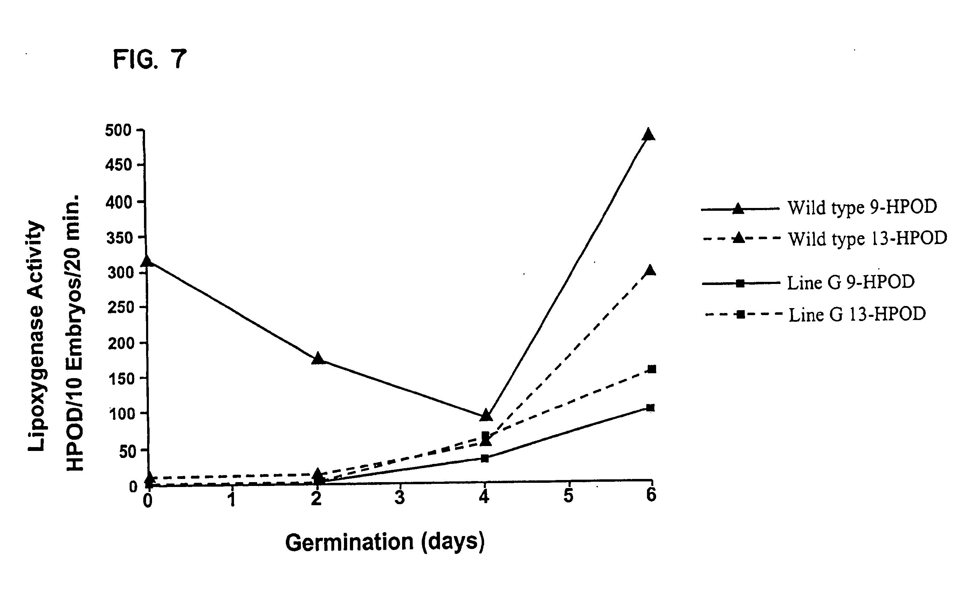

Analysis of the HPODs formed by the lipoxygenase activity in germinating embryos showed that 9-HPOD was the major product of lipoxygenases present in quiescent wild-type grain (**Figure 7**). The level of 9-HPOD formation fell with the decline in lipoxygenase activity in the extracts. The rise in lipoxygenase activity after 4 days was accompanied by the formation of both 9-HPOD and 13-HPOD. The low lipoxygenase activity in Line G quiescent grain was associated with an absence of HPOD formation, while the rise in activity after 4 days mainly produced 13-HPOD. These data provide evidence that LOX-1 activity leading to the formation of 9-HPOD is greatly reduced in the embryos of both developing, quiescent and germinating barley grain of Line G, while LOX-2 activity leading to formation of 13-HPOD is unchanged in Line G.

### Example 3

### Line G has a Mutant Lipoxygenase 1 Gene (lox-1) Causing a Low Lipoxygenase Phenotype

The molecular basis for the low-LOX-1 phenotype of Line G was investigated in order to provide a complete description of the mutant. The following analyses were performed to provide a complete characterization of the phenotype:

### 1. Lipoxygenase-1 is synthesised in the developing and germinating grain of Line G

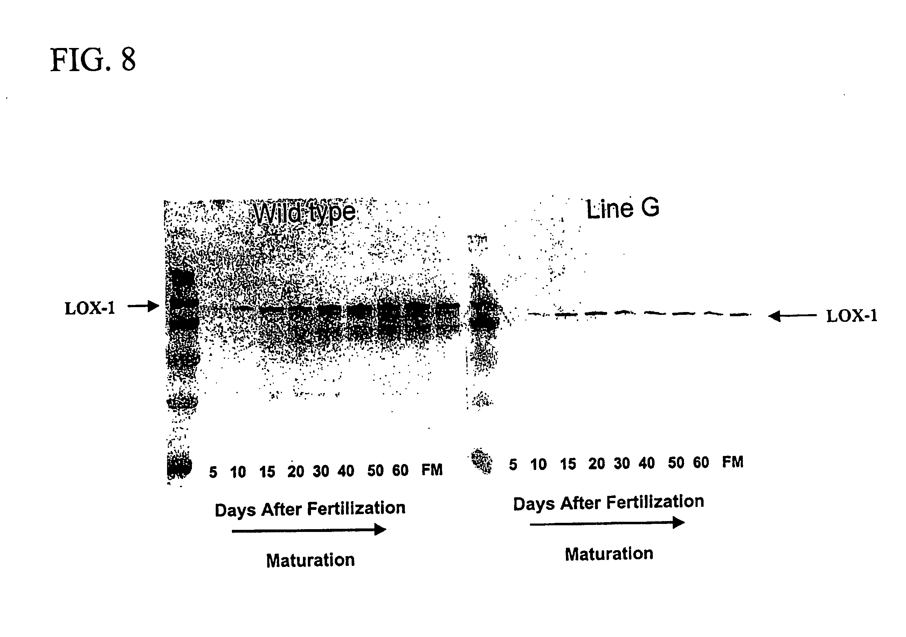

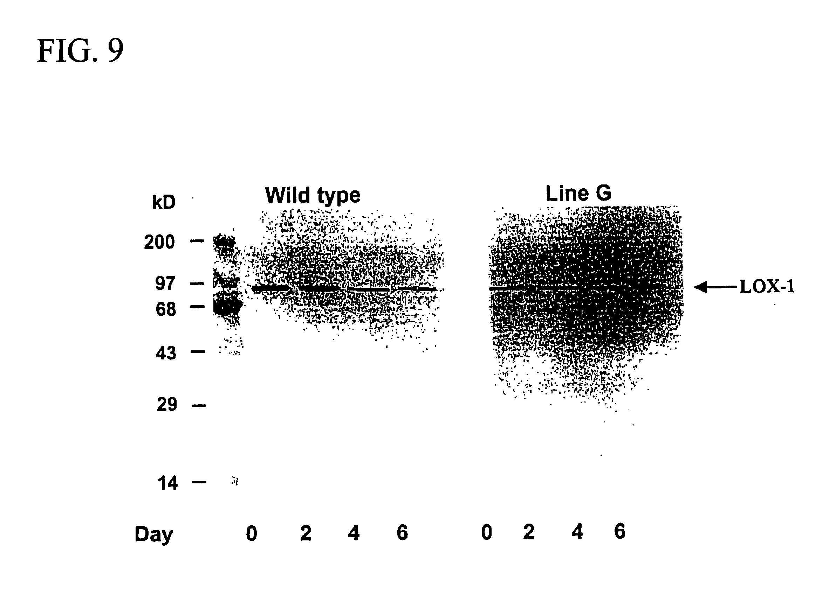

Western blot analysis of extracts of embryos from developing and germinating barley grain were performed in parallel with the measurement of lipoxygenase activity, as described in Example 2. The crude extracts were separated by sodium dodecyl sulphate-polyacrylamide gel electrophoresis (SDS-PAGE) according to Laemmli, 1970, Nature 227: 680-685. The separated proteins were transferred to nitrocellulose by semi-dry blotting, according to Towbin et al., (1979) Proc. Natl. Acad. Sci. USA 76: 4350-4354. The blot was probed with the LOX-1 specific monoclonal antibody, 5D2, as described Holtman et al., 1996, Plant Physiology 111: 569-576, at 500x dilution, followed by incubation with goat anti-mouse antibody coupled to alkaline phosphatase, and detected with the alkaline phosphatase substrates nitro blue tetrazolium and 5-bromo-4-chloro-3-indolyl-phosphate as described by Holtman et al., 1996, Plant Physiol. 111: 569-576. The Western analyses revealed that LOX-1 protein was detected in developing grain from 10 DAF in cv Vintage embryos and the level increased during grain maturation **(****Figure 8**). The protein was also present in the embryo of cv Vintage quiescent grain but declined slowly during germination (**Figure 9**). Although LOX-1 is recognised in extracts of Line G embryos and migrates in SDS-PAGE as a protein of similar size to cv Vintage LOX-1, the immunodetectable levels of the protein in Line G were slightly lower than in cv Vintage.

### 2. The lox-1 gene is expressed in the developing and germinating grain of Line G

Total RNA was isolated from embryos of developing and germinating barley grain, according to the procedure of Hensgens and van Os-Ruygrok, 1989, Rice Genet. Newslett. 6: 163-168, in parallel with the measurement of lipoxygenase activity, described in Example 2. The RNA samples (7.5 µg) were separated on denaturing agarose gels and Northern blotted as described by Sambrook et al., 1989 in Molecular Cloning, a Laboratory Manual, Cold Spring Harbour Laboratory Press, NY. The blots were hybridised with a ³²P-labelled probe generated from the barley 3' untranslated region, nucleotides 2659-2801 **[SEQ ID NO:1],** of the *lox* 1 cDNA (EMBL Accession no. L35931) as described by Holtman et al., 1996 Plant Physiol. 111: 569-576, using the Amersham Random Prime Kit.

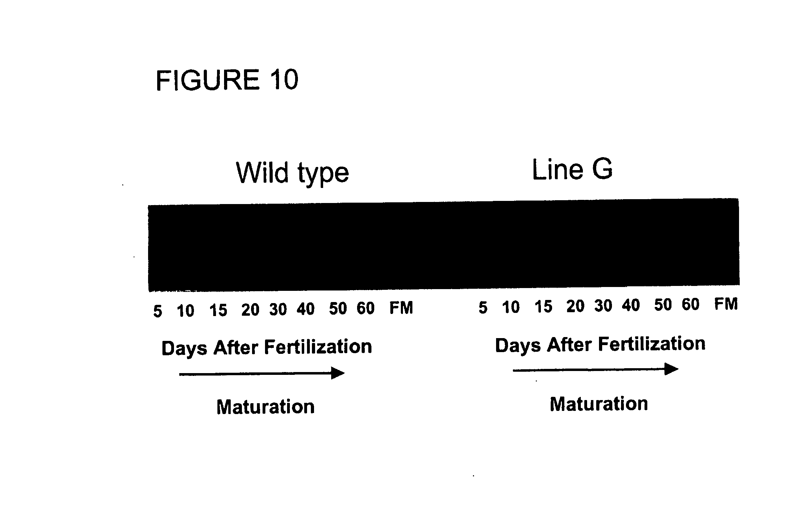

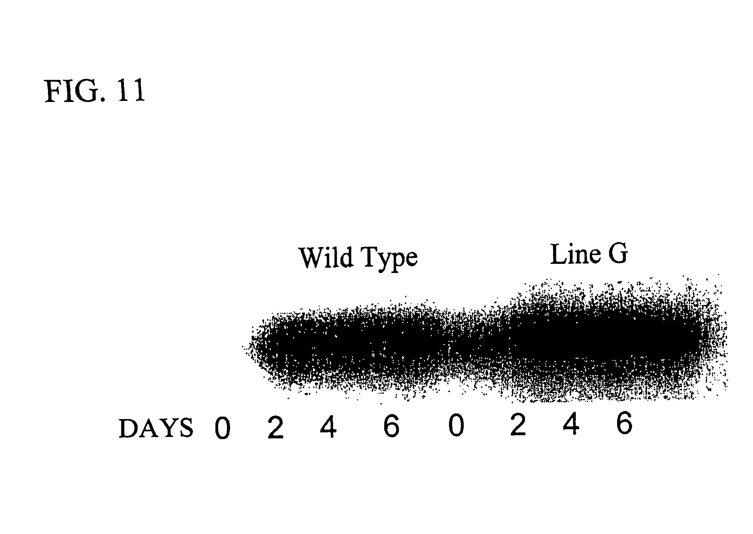

*Lox*-1 transcripts encoding LOX-1 were detected in embryos of developing and mature cv Vintage and Line G grain from 30 DAF (**Figure 10**). The level of *lox*-1 trancripts increased during germination in both cv Vintage and Line G embryos, indicating *de novo* expression of the *lox*-1 gene (**Figure 11**). Since the detectable levels of *lox*-1 transcripts were similar in Line G and cv Vintage embryos, neither reduced *lox*-1 transcription or transcript stability can account for the low-lipoxygenase phenotype of Line G.

### 3. The lox-1 gene of Line G encodes a mutant form of lipoxygenases-1

The nucleotide sequence of the *lox*-1 gene of Line G and cv Vintage were analysed and compared in order to determine the molecular basis for the low-LOX-1 phenotype of Line G, which is characterised by normal transcription of the *lox*-1 gene, but reduced accumulation and activity in the expressed lipoxygenase enzyme in grain.

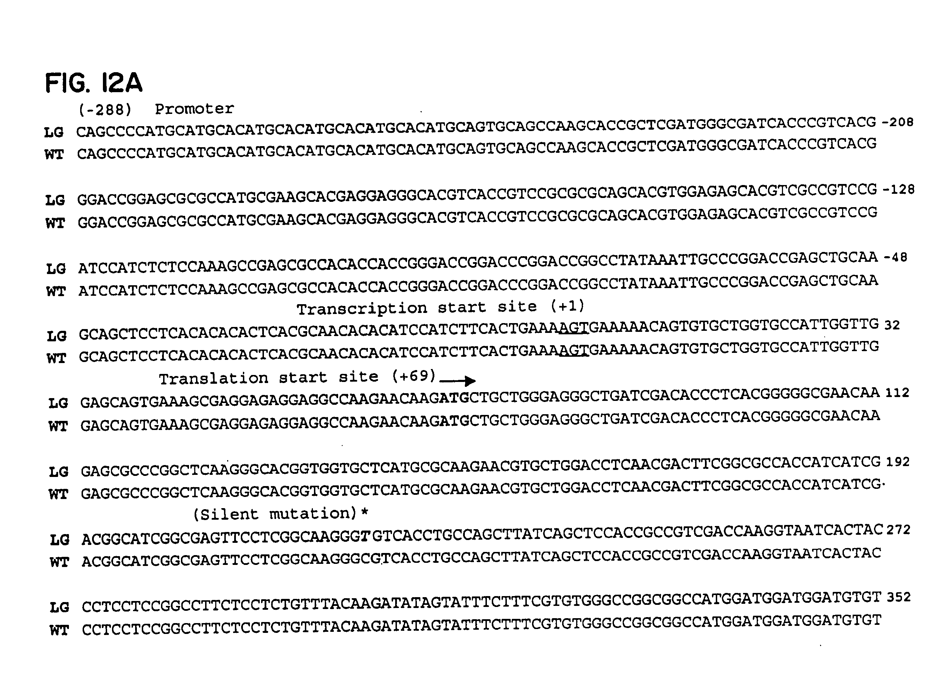

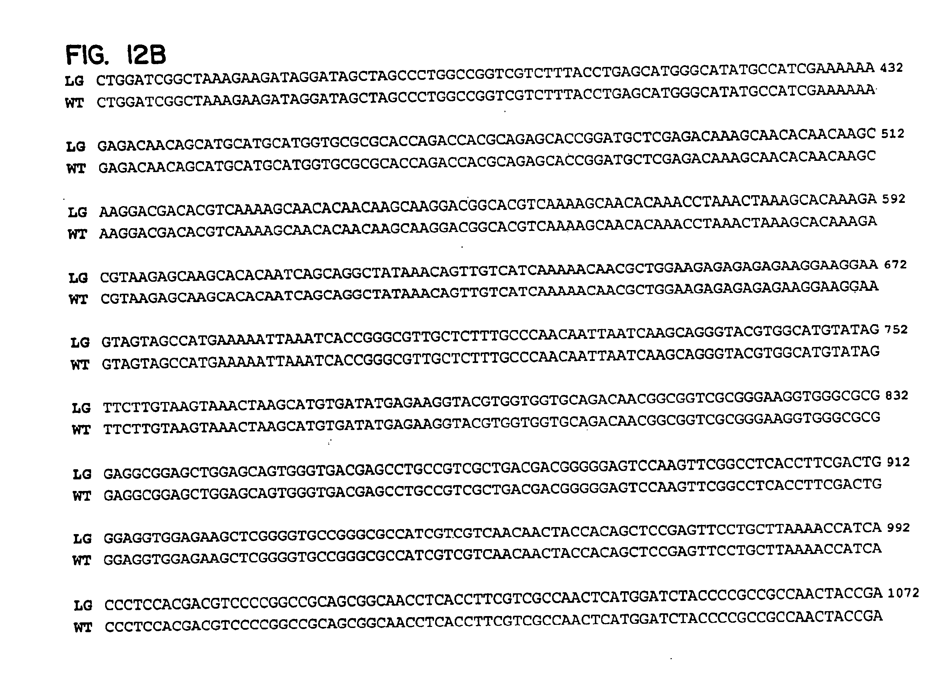

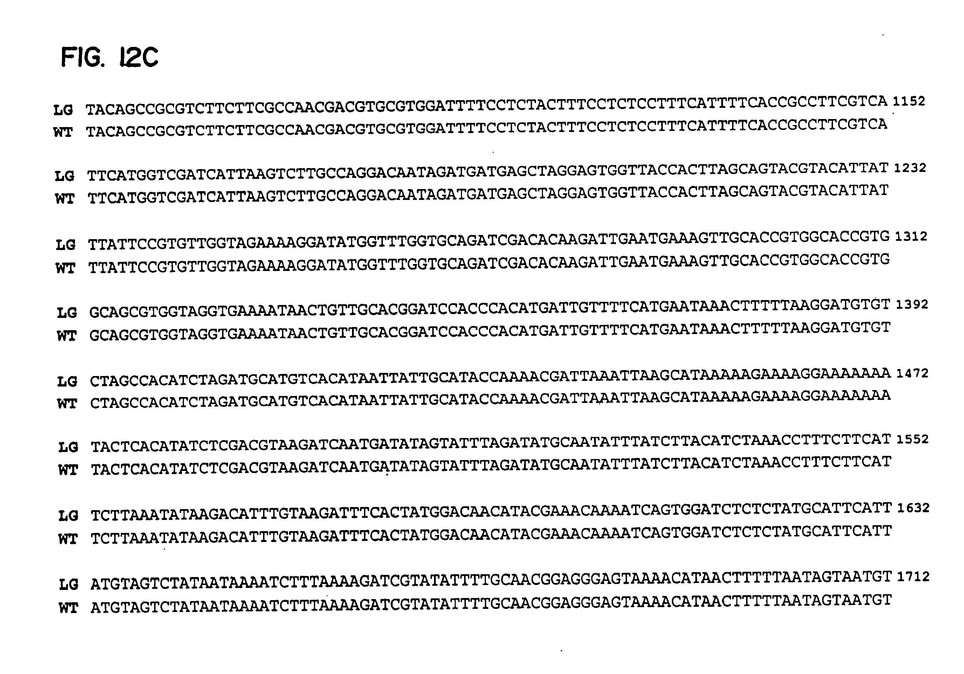

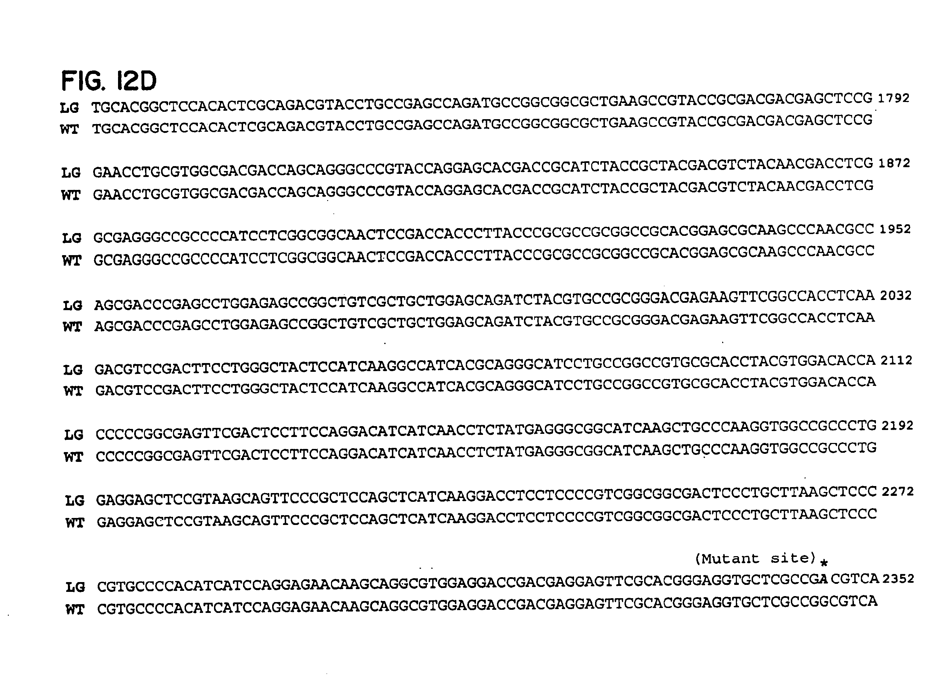

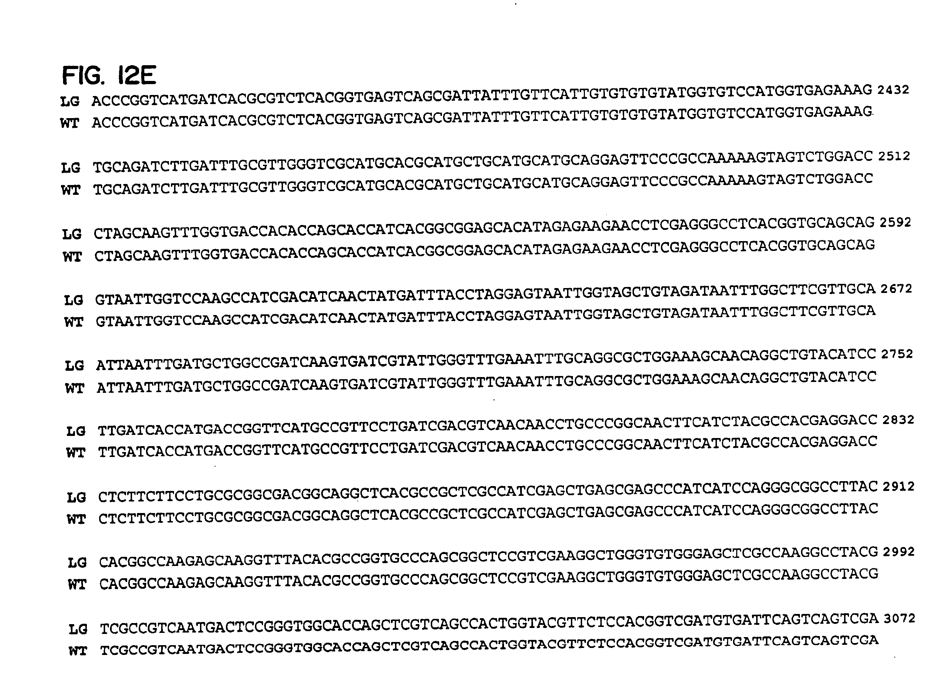

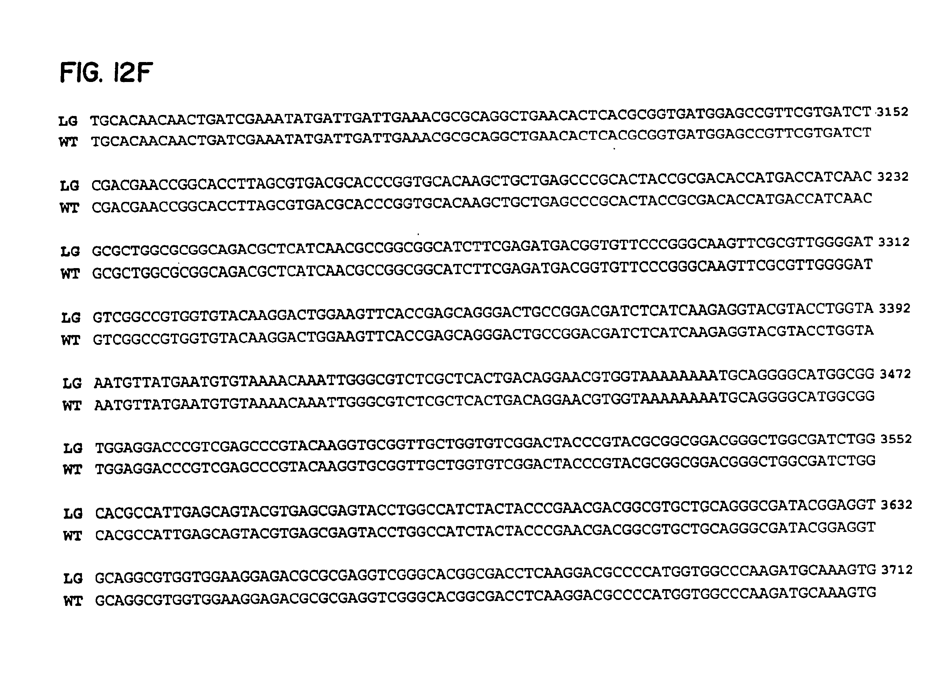

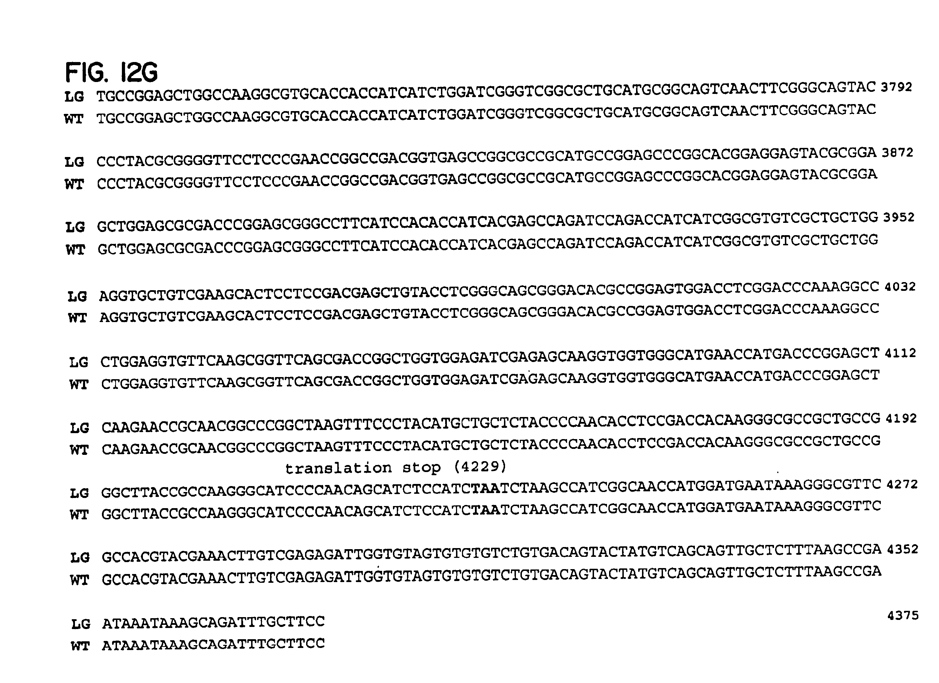

Genomic DNA from Line G and wild-type cv Vintage was isolated from seedling leaf tissue according to a method described by Pich and Schubert 1993, Nucleic Acids Res. 21: 3328. The *lox*-1 gene in the genomic DNA preparations was amplified by polymerase chain reaction (PCR) using primers based on the sequence of the barley *lox*-1 gene (van Mechelen et al. 1995, BBA 1254: 221-225; Rouster et al., 1997, Plant J. 11: 513-523). The position and sequence of the oligonucleotide primers used to amplify the *lox*-1 promoter and coding regions, indicated in **Figure 12** were as follows:

Forward primer 5'-GAA AAG CTT GGA GGT AGA CGC TGC-3' **[SEQ ID NO:2]** and reverse primer 5'-TAT AGG ATC CTT GTT CTT GGC CTC CTC TCC TCG-3' **[SEQ ID NO:3]** were used to PCR amplify the *lox*-1 promoter domain (-361 to +68) of Line G and cv Vintage *lox*-1.

Forward primer 5'-AGT GAA AAA CAG TGT GCT GGT G-3' **[SEQ ID NO:4]** and reverse primer 5'-GGC TTA AAG AGC AAC TGC TGA-3' **[SEQ ID NO:5]** were used to PCR amplify the Line G *lox*-1 coding region.

Forward primer 5'-CAA GAT GCA TAT GCT GCT GGG AG-3' **[SEQ ID NO:6]** and reverse primer 5'-CGA TGG TTT AAA TTA GAT GGA GAT GCT GT-3' **[SEQ ID NO:7]** PCR amplified the cv Vintage *lox*-1 coding region.

The PCR reactions consisted of 250 ng genomic DNA in a 50 µl volume containing 50 pmol primer and 2 U *Pfu* DNA polymerase (Promega) according to the enzyme suppliers instructions. The PCR amplifications were carried out in a Stratagene Robocycler: 1 minute at 94°C, 1 cycle; 1 minute at 94°C, 2 minutes at 62°C, and 5 minutes at 72°C, 30 cycles; 10 minutes at 72°C, 1 cycle. The PCR products were separated on 1.2 % agarose gels. DNA fragments, corresponding in length to the amplified region, were purified using Qiax II Gel extraction kit (Qiagen) and cloned into the plasmid pcDNA2.1 (Invitrogen). The nucleotide sequence of both strands of the cloned *lox*-1 promoter and coding regions was determined using the dideoxynucleotide chain termination reaction with specific oligonucleotide primers and analysed on an ABI PRISM® 310 Genetic Analyzer (PE Biosystems). Sequence comparisons were performed using the DNA STAR sequence analysis software package (DNA STAR Inc., USA).

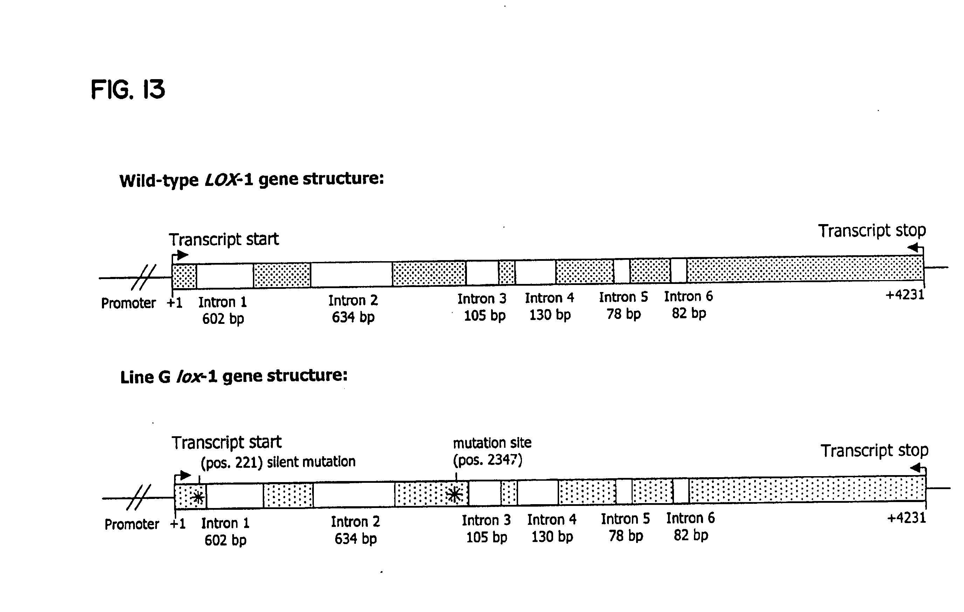

The promoter region and intron-exon structure of the barley *lox*-1 coding region are shown in **Figure 13**, and were deduced from a comparison of the nucleotide sequence of the wild-type *lox*-1 genomic and cDNA sequences **(****Figure 12****)**. The sequenced region of the *lox*-1 promoter region from -363 to +68, (numbered relative to the determined transcription start site; van Mechelen et al., 1995, BBA 1254: 221-225), is sufficient to direct embryo-specific and temporally-regulated gene expression characteristic of the native gene (Rouster et al, 1998, Plant J 15: 435-440). The promoter and transcribed region of the wild-type *lox*-1 gene **[SEQ ID NO:8]** is 4663 nt in length and contains 6 introns of between 82 nt and 634 nt in length, which are absent from the respective cDNA **[SEQ ID NO:10]** and must therefore be removed during RNA transcript splicing.

Comparison of the nucleotide sequence of *lox*-1 of Line G with that of wild-type (**Figure 12**) showed that the Line G *lox*-1 allele has two point mutations. One is a silent C→T substitution at position 221 in exon 1, and the second is a G→A substitution at position 2347 in exon 3 (**Figure 13**). The wild-type barley *lox*-1 gene encodes a protein of 862 amino acid residues **[SEQ ID NO:9],** while the mutation at position 2347 in Line G *lox*-1 allele causes an amino acid substitution of glycine to aspartic acid at residue 368 in the encoded protein.

Alignment of related plant lipoxygenases indicated that the glycine-368 in barley LOX-1, is strongly conserved. Furthermore this residue, which corresponds to glycine-353 in soybean LOX-1, is one of 51 neutral or hydrophobic residues which line the substrate cavity of the enzyme, as seen from its crystal structure (Minor et al., 1996, Biochemistry 35: 10687-10701) and is shown in (Figure 22). The insertion of a charged amino acid residue at this position is thus likely to disturb the structural and functional properties of the enzyme.

### 4. The mutated LOX-1 protein encoded by the Line G lox-1 allele has low enzymic activity and is responsible for the low lipoxygenase phenotype of Line G.

The sodium azide mutagenesis of cv Vintage grain, which induced the mutated *lox*-1 allele in Line G, may have induced additional mutations in the Line G genome. Two experimental approaches have been taken to demonstrate that the mutant *lox*-1 allele in Line G is responsible for its low lipoxygenase phenotype, rather than other mutations in the genome. The enzymic activity of the LOX-1 encoded by the mutant and wild-type *lox*-1 allele have been determined in order to prove that the glycine→aspartic acid substitution in the mutant enzyme causes reduced stability and activity. The two *lox*-1 genes were transiently expressed in aleurone protoplasts isolated from imbibed mature grain, since the level of endogenous lipoxygenase expression in these cells was expected to be below detection limits. None of the identified barley lipoxygenase genes, which are expressed in germinating barley, are detected in the aleurone tissue (van Mechelen *et al.,* 1999 *supra).* In order to direct transient expression of the *lox*-1 gene in aleurone protoplasts, their coding regions were translationally fused to a constitutive promoter known to be active in these protoplasts.

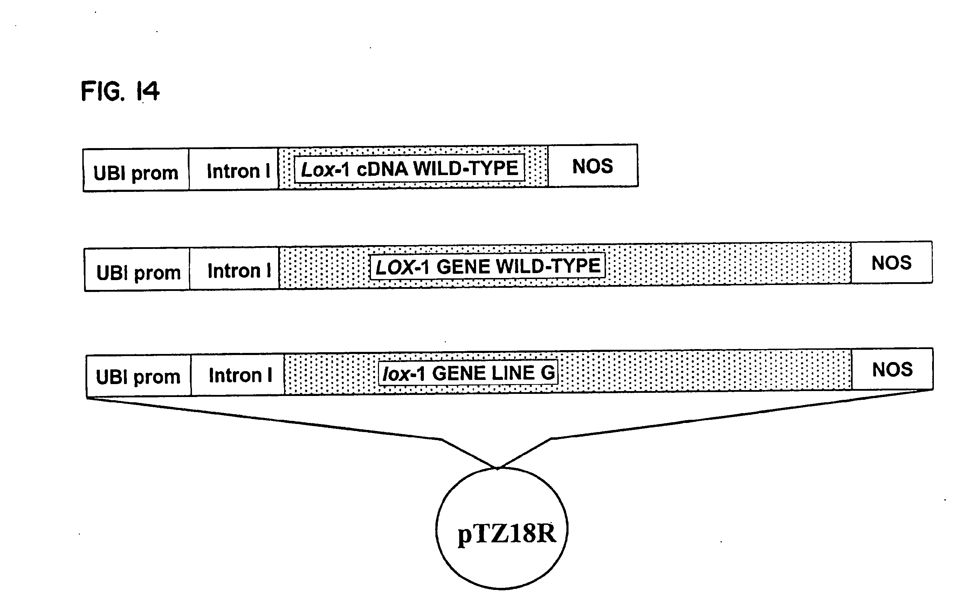

The coding regions of the mutant (sequence positions +1 to +4350) and the wild-type *lox*-1 gene (sequence positions +69 to +4230) and the wild-type *lox*-1 cDNA (sequence positions +69 to +2654) each cloned in plasmid pcDNA2.1 (see section 3), were excised by digestion of the *Kpn*I and *Eco*RV sites in vector polylinker. The coding regions were cloned in the pUBARN plasmid (Jensen et al., 1998, Hereditas 129: 215-225) between the constitutively active maize ubiquitin *Ubi* promoter (as described in U.S. patent No. 005510474A) and the *Nos* terminator, in place of the *bar* gene which encodes phosphinotricin acetyl transferase (**Figure 14**).

Protoplasts were isolated from aleurone tissue of imbibed *Hordeum vulgare* cv Himalaya according to the protocol of Skriver et al. 1991, Proc. Natl. Acad. Sci. USA 88: 7266-7270. Aliquots of 2.10⁵ protoplasts were transfected at 0°C with ∼100 µg plasmid DNA (equimolar amounts of each plasmid) by polyethylene glycol (PEG) mediated DNA uptake (Lee et al., 1997, Plant Mol. Biol. 13: 21-29), and then incubated in aleurone protoplast culture media at 25°C as described previously (Skriver *et al.,* 1991 *supra*). After 48 hours incubation, the culture medium was carefully removed and the protoplasts were resuspended and homogenised in 300 µl lipoxygenase assay buffer (0.2 mM boric acid, 25 mM HEPES, pH 6.5). The homogenates were centrifuged at 15,000 *g* for 5 minutes to pellet insoluble material, and the supernatants (10 µl) were subsequently assayed for total lipoxygenase activity using the rapid screening assay described in Example 1, section 1, but with omission of the NDGA inhibitor. The protein content of the protoplast extracts was measured by a Bradford dye-binding assay (Bradford 1976, Anal. Biochem., 72: 248) supplied by Bio-Rad Laboratories, Hercules, California, USA, and lipoxygenase activity was expressed per mg protein in the extract.

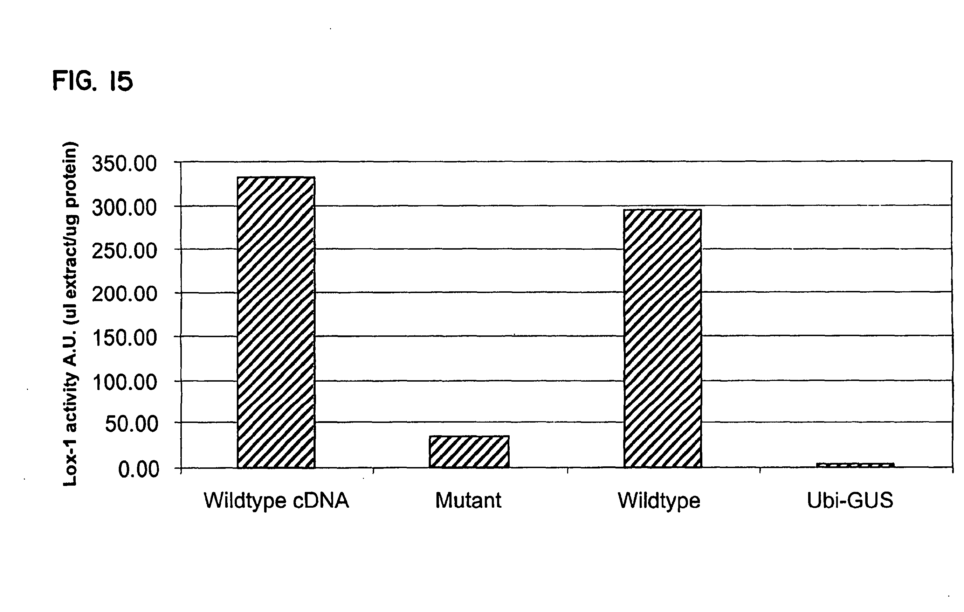

Protoplasts transfected with the control plasmid, pUBI-GUS, where the maize ubiquitin-1 promoter directs expression of the β-glucuronidase reporter gene, gave no detectable lipoxygenase activity. Transient expression of the wildtype *lox*-1 gene and cDNA in protoplasts both gave high levels of lipoxygenase activity in the protoplast extracts (**Figure 15**). The higher expression of the wild-type *lox*-1 cDNA in comparison to the genomic sequence may be due to a higher transfection frequency for the smaller *lox*-1 cDNA expression plasmid (4929 bp versus the 6505 bp *lox*-1 gene construct). Transient expression of the mutant *lox*-1 gene gave low levels of lipoxygenase activity, ∼10 % of wild-type lipoxygenase activity. These data clearly demonstrate that the mutant *lox*-1 gene in Line G encodes a lipoxygenase with greatly reduced activity, which accounts for the low lipoxygenase phenotype.

### Example 4

### PCR-Cleavage Amplified Polymorphic Site (PCR-CAPS) Assay: A Method Used for Identification of the Mutant lox-1 Gene

An analytical method allowing the identification of the Line G mutant *lox*-1 gene in any genetic background was developed based on the PCR-CAPS assay. The assay involves PCR amplification of genomic DNA fragments, followed by digestion of the amplified sequences with a specific restriction endonuclease to display a restriction fragment length polymorphism (RFLP).

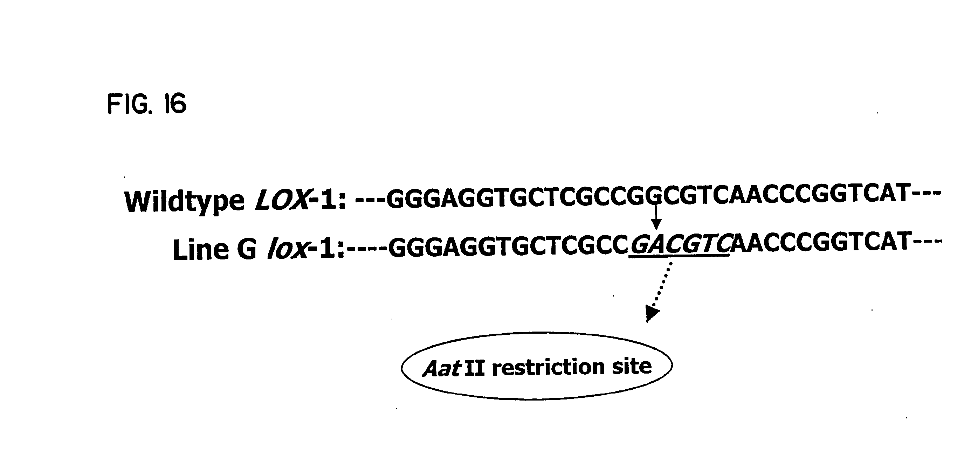

The coding sequence of the mutant *lox*-1 gene harbours two point mutations (see Example 3), where the mutation at position 2347 (**Figure 12**) introduces an additional *Aat* II restriction endonuclease cleavage site, not found in the wild-type *lox*-1 gene (**Figure 16**). The following PCR-CAPS assay, based on the polymorphism created by the presence of this restriction site in the *lox*-1 gene, is shown to descriminate between a wild-type *lox*-1 gene and a mutated *lox*-1 gene.

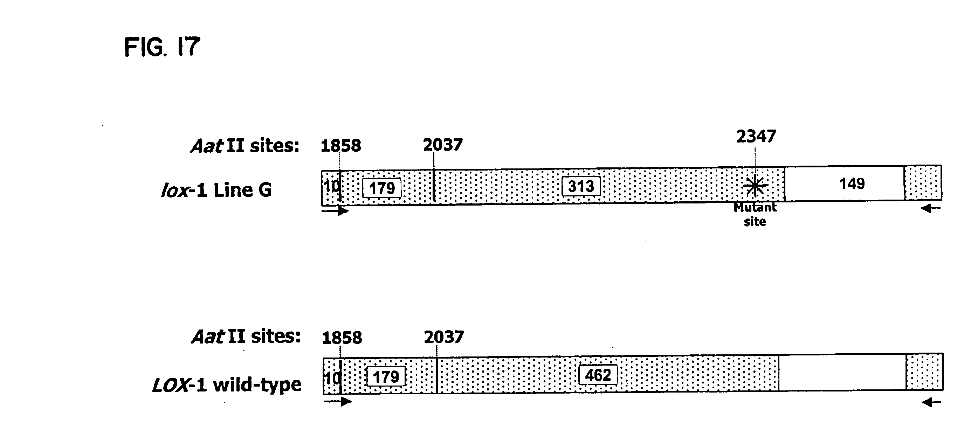

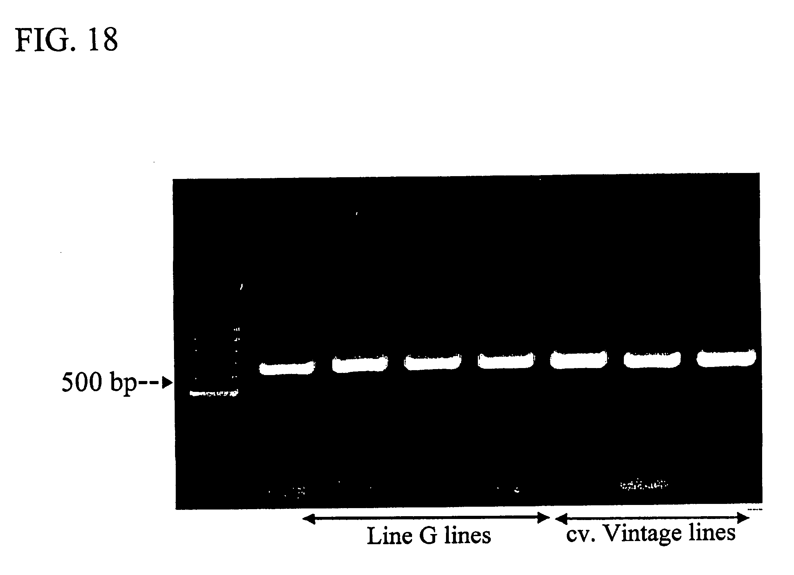

Genomic DNA was isolated from young leaves of M6 seedlings of *Hordeum vulgare,* L. cv Vintage and Line G according to the procedure of Pich and Schubert (1993, *supra*). The DNA sequence encompassing position 2347 (Line G *lox*-1 gene mutation site) was amplified by PCR, using primers specific for the *lox*-1 gene **[SEQ ID NO:8].** The DNA fragments amplified by the selected forward primer 5'-CGCTACGACGTCTACAACGA-3' **[SEQ ID NO:13]** and reverse primer 5'-CAGACTACTTTTTGGCGGGA-3' **[SEQ ID NO:14]** are shown in **Figure 17**. PCR reactions were carried out with 250 ng genomic DNA in a 50-µl volume containing 50 pmol of each primer and 1 unit Taq DNA polymerase (Promega) according to the suppliers instructions. PCR amplifications were carried out on a Stratagene Robocycler as follows: 1 minute at 94°C, 1 cycle; 1 minute at 94°C, 1.5 minutes at 60°C, and 2 minutes at 72°C, 30 cycles; 10 minutes at 72°C, 1 cycle. The amplified fragments of the mutant and wild-type *lox*-1 gene were -650 bp (**Figure 18**), corresponding to the expected size (**Figure 17**). The PCR products, purified on a spin column (Qiagen), were digested with 25 unit *Aat* II restriction endonuclease for 24 hours at 37°C and analyzed on a 1.2% agarose gel.

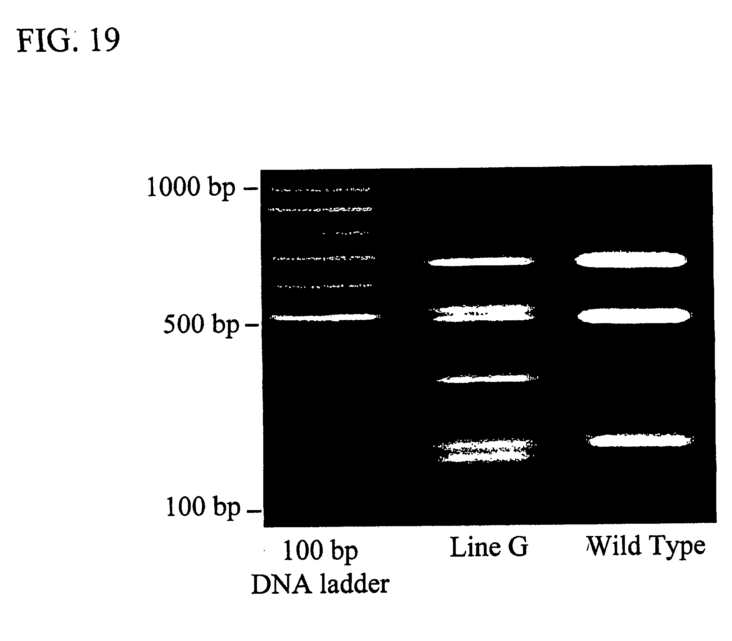

Digestion of the wild-type *lox*-1 PCR product yielded DNA fragments of 10, 179, and 462 bp, and the fragments from the mutant lox-1 PCR product were 10, 149, 179, and 313 bp, where additional DNA fragments were due to partial digestion of the *lox*-1 PCR product (**Figure 19**). The fragment pattern corresponds to the expected RFLP resulting from this mutation, where the 313 bp fragment is unique to the mutant *lox*-1. This PCR-CAPS assay provides a reproducible and specific tool for identification of the *lox*-1 allele in barley and can thus be exploited in barley breeding programs aimed at introducing this gene in new barley varieties.

### Example 5

### Back-Crossing the Low Lipoxygenase Phenotype of Line G to cv Alexis Demonstrates a Genetic Linkage to the Mutant lox-1 Gene

Repeated back-crossing was used to transfer the low-lipoxygenase phenotype from line G into a recurrent parent (in this case the cv. Alexis). The back-crossing program shown in **Figure 20**, combined with selection for the low-lipoxygenase phenotype, progressively substitutes the Line G genome by the recurrent parent genome. Furthermore, other mutations introduced into the Line G genome during the sodium azide mutagenesis treatment will be eliminated. In the first back-cross of the homozygous low-lipoxygenase Line G (denoted genotype *ll*) to cv Alexis (denoted genotype LL) the progeny lines will be heterozygous (denoted genotype *LI).* A low-lipoxygenase phenotype due to a recessive mutation will not be detectable in lines heterozygous for the mutation. The progeny are self-pollinated and will give a normal Mendelian segregating population, namely 1*LL*: 2 *LI :* 1 *ll.* The low lox homozygous *ll* genotype resulting from the first back-cross will have 50 % cv Alexis genetic background. After ten rounds of back-crossing, the recurrent parent background will be approximately 99.9 %.

*Hordeum vulgare,* L. cv Alexis and Line G were propagated in a greenhouse throughout the back-crossing program. Back-crossed progeny grains were germinated in petri dishes on filterpaper, soaked with 4 ml H₂O, for 3 days at 22°C in the dark. The low-lipoxygenase lines were screened by measuring total lipoxygenase activity in extracts of the coleoptile (top 7 mm) from the germinating seedlings, as described in Example 1. Progeny of the 3^{rd} and 4^{th} back-cross were also analysed for inheritance of the mutant *lox*-1 gene using the PCR-CAPS assay described in Example 4.

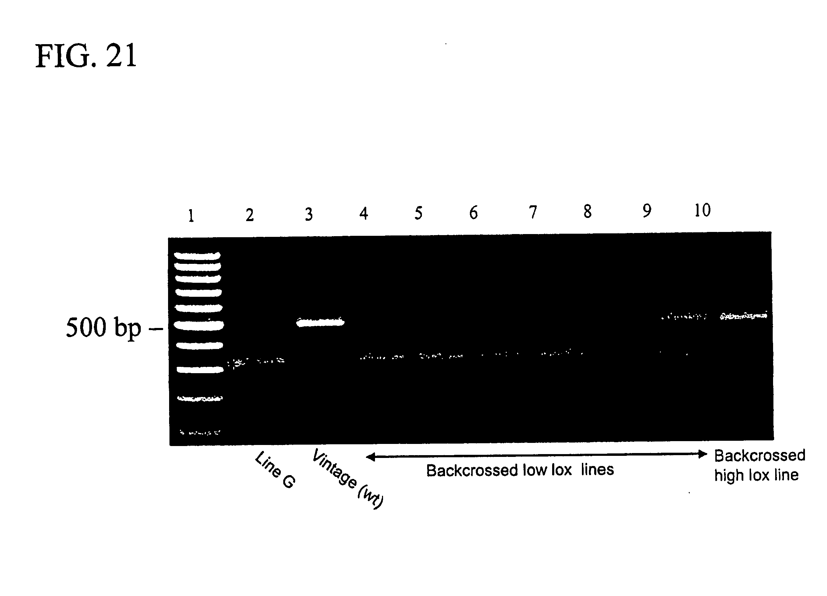

The expected frequency of the low-lipoxygenase phenotype in the segregating progeny of the four back-cross generations was 25% for a recessive mutation. The observed frequency of low-lipoxygenase activity in the progeny (24 grains) of the four back-cross generations is in agreement with the expected frequency **(****Figure 20****)**. When the 3^{rd} and 4^{th} back-cross progeny having the low lox homozygous *ll* genotype were analysed with the PCR-CAPS assay, they were all found to have the diagnostic 313 bp fragment, while progeny having wild-type lipoxygenase activity lacked this fragment **(****Figure 21****)**.

The back-crossing program demonstrates that the mutant *lox*-1 allele can be transferred to a new genetic background and is inherited in a recessive monofactorial manner following Mendelian segregation. Since the recurrent parent background is 93.8% in the 4^{th} back-cross progeny, the co-inheritance of the mutant *lox*-1 gene and the low-lipoxygenase phenotype provides confirmation of their genetic linkage.

### Example 6

### Beer Brewed From Line G Barley Malt Accumulates Less trans-2-nonenal During Storage, Giving an Improved Flavour Stability

*Hordeum vulgare* L cv Vintage and Line G were propagated in the field over several seasons in order to provide sufficient grain for industrial malting. The following industrial scale malting and brewing trials as well as analyses of the finished beer were performed to demonstrate the value of the Line G low-lipoxygenase barley for improved flavour stability.

### 1. Industrial malting and kilning of Line G and cv Vintage

Malting was performed on a 10-ton scale in an industrial malthouse in two trials as follows:

### Trial 1: Line G barley grain (1996 harvest)

Steeping conditions: 8 hours wet; 14 hours dry; 8 hours wet; 10 hours dry; 4 hours wet in 16°C steeping water. Malting conditions: 12 hours at 18°C; 24 hours at 16°C; 24 hours at 14°C; 60 hours at 12°C. Kilning conditions: 12 hours at 60°C; 3 hours at 68°C; 4 hours at 74°C; 3 hours at 80°C.

### Trial 2: cv Vintage and Line G (1996/1997 harvest)

Steeping conditions: 8 hours wet; 10 hours dry; 6 hours wet; 15 hours dry; 4 hours wet in 15°C steeping water. Malting conditions: 5 days with inlet air at 15°C and spraying to maintain moisture level. Kilning conditions: 10 hours at 50°C; 2 hours at 60°C; 2.5 hours at 80°C.

Malting analyses of 2 samples of the Line G malt from Trial 1 compared to the control malt, cv Nevada (Table 1) and from Trial 2 compared to cv Vintage (Table 2) confirmed that Line G malt was suitable for brewing trials.

**TABLE 1**

| **MALTING TRIAL 1** | | | | |
|---|---|---|---|---|
| Barley variety | | cv Nevada | LineG | LineG |
| Crop year | | 1996 | 1996 | 1996 |
| | | | | |
| Malt analyses | | | | |
| Moisture content | % | 4.7 | 4.3 | 4.4 |
| Extract fine as is. | %ai. | 76.9 | 76.1 | 75.3 |
| Extract fine d.m. | %dm. | 81.4 | 79.5 | 78.7 |
| Saccharification time | Min | <10 | <10 | < 10 |
| Diastatic power | %WK. | 252 | 373 | 365 |
| Color | EBC | 2.8 | 4.4 | 3.8 |
| pH | | 6.16 | 5.97 | 5.99 |
| Turbidity | EBC | 9.0 | 2.5 | 2.4 |
| Total protein d.m. | % | 10.35 | 10.74 | 12.03 |
| Soluble nitrogen | mg/l | 647 | 787 | 765 |
| Sol. Protein % malt d.m. | %dm. | 3.7 | 4.4 | 4.3 |
| Kolbach | | 35.3 | 40.8 | 35.4 |
| Free Amino Nitrogen | mg/l | 97 | 125 | 118 |
| Friability | % | 89.5 | 85.6 | 89.5 |
| Whole unmodified grains | % | 1.1 | 0.6 | 0.5 |
| Partly unmodified grains | % | 2.3 | 1.0 | 0.6 |
| β-glucan in wort | mg/l | 114 | 66 | 36 |
| β-glucan in malt | % w/w | 0.24 | 0.11 | 0.05 |
| S-methylmethionine/DMS eq. | µg/g | 2.4 | 6.4 | 8.4 |
| Free DMS | µg/g | 1.0 | 6.6 | 4.7 |
| NDMA | µg/kg | n.d. | 0.3/0.6 | 0.3/0.3 |

**TABLE 2**

| **MALTING TRIAL 2** | | | | |
|---|---|---|---|---|
| Barley variety | | Vintage | Line G | Line G |
| Crop year | | 1996 | 1996 | 1997 |
| | | | | |
| Moisture content | % | 4.1 | 4.1 | 4.3 |
| Extract fine as is. | %ai. | 77.0 | 75.6 | 77.5 |
| Extract fine d.m. | %dm. | 80.3 | 78.8 | 80.9 |
| Fine/coarse difference | % dm. | 0.7 | 1.6 | 1.7 |
| Saccharification time | min | - | - | <10 |
| Diastatic power | %WK | 343 | 342 | 268 |
| Color | EBC | 2.5 | 2.8 | 3.4 |
| PH | | 6.05 | 6.01 | 6.12 |
| Turbidity | EBC | 1.5 | 1.3 | 2.5 |
| Total protein d.m. | % | 10.98 | 12.22 | 9.82 |
| Soluble nitrogen | mg/l | 696 | 741 | 610 |
| Sol. Protein % malt d.m. | %dm. | 3.9 | 4.1 | 3.4 |
| Kolbach | | 35.2 | 33.7 | 34.6 |
| Free Amino Nitrogen | mg/l | 110 | 117 | 100 |
| Friability | % | 91.3 | 81.8 | 89.5 |
| Whole unmodified grains | % | 0.7 | 1.1 | 1.3 |
| Partly unmodified grains | % | 1.0 | 2.7 | 2.7 |
| β-glucan in wort | mg/l | 97 | 172 | 117 |
| Beta-glucan in malt | % w/w | - | - | 0.3 |
| S-methylmethionine/DMS eq. | µg/g | - | - | - |
| Free DMS | µg/g | - | | - |

### 2. Industrial brewing with Line G, cv Vintage malt, and control malt cv Nevada

Two brewing trials were performed, using wort prepared from Line G and control malt cv Nevada malt in Trial 1 and from Line G and control malt cv Vintage in Trial 2.

Beer was brewed on a 30-hl industrial scale with 475 kg malt according to the following scheme: Mashing in at 50°C; 30 minutes at 50°C; 30 minutes heating from 50 - 70°C; 15 minutes at 70°C. A portion of the wort was heated for 20 minutes from 70 - 100°C and 5 minutes at 100°C, while the main mash was kept at 70°C for another 25 minutes and then the two mashes was combined and kept for 10 minutes at 76°C. The brewing steps of wort boiling, whirlpool separation of spent grain, cooling, fermentation, lagering and packaging in brown glass bottles were according to standard brewing practise.

### 3. Flavor stability and T2N content of beer brewed from Line G, cv Vintage malt and control malt cv Nevada

The freshly bottled beer was stored at 5°C and analysed within 2 months of production. The flavor-stability of the fresh and stored beer was evaluated in two independent laboratories following two different types of beer storage conditions. In laboratory A the beer was subjected to a forced aging process, where the beer was stored at 37°C for a period of 7 days, while in laboratory B the beer was stored at 30°C for 6 and 12 weeks. *Trans*-2-nonenal levels in beer were determined by gas chromatography and mass spectrometric detection following derivatisation of carbonyls with O-(2,3,4,5,6-pentafluorobenzyl)-hydroxylamine, essentially as described by Grönqvist et al. 1993 Proceedings of the 24th EBC Congress, Oslo, 421-428. A trained beer taste panel evaluated the overall flavor score of the beer, which includes detection of a cardboard flavor, indicative of free *trans*-2-nonenal in the beer.

### Laboratory A: Forced-Aging Tolerance

Comparison of beer, brewed from Line G and the control malt, cv Nevada, in the first brewing trial (Table 3) demonstrated that beer from Line G had a greater flavor stability and a lower *trans*-2-nonenal content following forced-aging as compared to the controls. The second trial, comparing beer brewed from Line G malt with beer brewed from cv Vintage malt, the parental cultivar, confirmed the initial data (**Table 4**).

**TABLE 3**

| **BREWING TRIAL 1** | | |
|---|---|---|
| Barley Malt | cv Nevada | Line G |
| SO₂ Content (mg/ml) | 1 | 1 |
| T2N** (ppb) ― Fresh Beer | 0.009 | 0.005 |
| T2N**(ppb) ― Aged Beer (37°C / 7 DAY) | 0.117 | 0.025 |
| Flavor* - Fresh Beer | 5.9 | 5.3 |
| Flavor* - Aged Beer (37°C / 7 DAY) | 1.3 | 5.1 |

| | | |
|---|---|---|
| * Flavor evaluation scale 1-10 of increasing quality; ***trans*-2-nonenal. | | |

**TABLE 4**

| **BREWING TRIAL 2** | | |
|---|---|---|
| Barley Malt | cv Vintage | Line G |
| SO₂ Content (mg/ml) | 2 | 2.5 |
| T2N** (ppb) - Fresh Beer | 0.023 | 0.019 |
| T2N** (ppb) - Aged Beer (37°C / 7 Day) | 0.078 | 0.035 |
| FLAVOR* - Fresh Beer | 5.5 | 6.1 |
| FLAVOR* - Aged Beer (37°C / 7 Day) | 2.9 | 5.9 |

| | | |
|---|---|---|
| * Flavor evaluation scale 1-10 of increasing quality; ***trans*-2-nonenal. | | |

### Laboratory B: 30°C Storage Tolerance

Beer brewed from Line G malt had lower *trans*-2-nonenal levels following 6 and 12 weeks at the elevated storage temperature of 30°C, when compared to beer brewed from either of the reference malts (Table 5 and 6) and had a better flavor-stability as judged by a taste panel. The taste-threshold for *trans*-2-nonenal in these analysed beers lies close to 0.08 ppb.

**TABLE 5**

| **BREWING TRIAL 1** | | |
|---|---|---|
| Barley Malt | cv Nevada | Line G |
| *trans*-2-nonenal (ppb) - Fresh Beer | 0.050 | 0.044 |
| trans-2-nonenal (ppb) - 30°C / 6 weeks | 0.072 | 0.037 |
| *trans*-2-nonenal (ppb) - 30°C / 12 weeks | 0.095 | 0.046 |
| trans-2-nonenal flavor *― Fresh Beer | 0.6 | 0.3 |
| *trans*-2-nonenal flavor *― 30°C / 6 weeks | 3.7 | 1.4 |
| *trans*-2-nonenal flavor* ― 30°C / 12 weeks | 2.5 | 0.6 |

| | | |
|---|---|---|
| * *trans*-2-nonenal flavor detection score on a scale of 1 -10 | | |

**TABLE 6**

| **BREWING TRIAL 2** | | |
|---|---|---|
| Barley Malt | cv Vintage | Line G |
| *trans*-2-nonenal (ppb) ― Fresh Beer | 0.070 | 0.062 |
| trans-2-nonenal (ppb) ― 30°C / 6 weeks | 0.093 | 0.070 |
| *trans*-2-nonenal (ppb) ― 30°C / 12 weeks | 0.133 | 0.080 |
| *trans*-2-nonenal flavor* ― Fresh Beer | 0.3 | 0.9 |
| trans-2-nonenal flavor* ― 30°C / 6 weeks | 2.5 | 1.7 |
| *trans*-2-nonenal flavor* ― 30°C / 12 weeks | 2.2 | 1.3 |

| | | |
|---|---|---|
| * *trans*-2-nonenal flavor detection score on a scale of 1 -10 | | |

The improved flavor-stability of beer brewed from Line G malt, as measured by the levels of *trans*-2-nonenal detected in the beer following storage at 30°C from the combined brewing trial data, is shown to be statistically significant (Table 7).

**TABLE 7**

| ***TRANS-2-*NONENAL IN STRORED BEER** | | | | | |
|---|---|---|---|---|---|
| **fresh** | **Mean** | **Stdev** | **Difference** | **2-tailed p** | **Significant (p < 0.05)** |
| reference | 0.060 | 0.012 | 0.007 | 0.34 | no |
| line-G | 0.053 | 0.011 | | | |
| | | | | | |

| **6 weeks, 30 °C** | **Mean** | **Stdev** | **Difference** | **2-tailed p** | **Significant (p < 0.05)** |
|---|---|---|---|---|---|
| reference | 0.083 | 0.013 | 0.029 | 0.031 | yes |
| line-G | 0.054 | 0.021 | | | |
| | | | | | |

| **12 weeks, 30 °C** | **Mean** | **Stdev** | **Difference** | **2-tailed p** | **Significant (p < 0.05)** |
|---|---|---|---|---|---|
| reference | 0.114 | 0.023 | 0.051 | 0.003 | yes |
| line-G | 0.063 | 0.020 | | | |

Since the natural sulfite levels were low in both brewing trials, the free *trans*-2-nonenal levels in the aged beer would closely reflect the *trans*-2-nonenal potential of the different beers, namely the level of *trans*-2-nonenal adducts present in the fresh beer. Addition of sulfite can temporarily delay the staling process, by complexing free-*trans*-2-nonenal, until sulfite levels are reduced by oxidation due to gaseous exchange through the packaging.

The described brewing trials with low-LOX-1 barley malt provide the first unequivocal evidence that LOX-1 activity in barley during the malting and brewing process is a key determinant of the appearance of the off-flavor compound *trans*-2-nonenal in aged beer.

The above specification includes citations to numerous publications. Each publication is hereby incorporated by reference for all purposes, as if fully set forth.

The invention also relates to the following aspects as defined in the following numbered paragraphs:
1. A barley plant or portion thereof comprising a mutant LOX-1 protein, the plant or plant portion characterized by a reduction or absence of lipoxygenase activity as compared to a non-mutated control.
2. The barley plant or portion thereof of paragraph 1, wherein the lipoxygenase activity comprises catalysis of oxidation of free and esterified polyunsaturated fatty acids and polyunsaturated octadecanoic fatty acids to form 9-hydroperoxy fatty acid derivatives.
3. The barley plant or portion thereof of paragraph 1, wherein the LOX-1 protein is encoded by a mutated *lox*-1 nucleic acid sequence having one or more mutation.
4. The barley plant or portion of paragraph 3, wherein said one or more mutation is induced by chemical mutagenesis or radiation.
5. The barley plant or portion of paragraph 3, wherein said one or more mutation is induced by site-directed mutagenesis.
6. The barley plant or portion thereof of paragraph 3, wherein said one or more mutation is positioned within the promoter or transcribed region of the *lox*-1 nucleic acid sequence.
7. The barley plant or portion of paragraph 1, wherein said mutant LOX-1 protein comprises an acidic, basic, or polar amino acid substitution at one or more conserved neutral or hydrophobic amino acid residue lining the substrate cavity of wild-type barley LOX-1.
8. The barley plant or portion of paragraph 7, wherein said mutant LOX-1 protein comprises the amino acid sequence of SEQ ID NO: 12, wherein Xaa is an acidic, basic, or polar amino acid.
9. The barley plant or portion of paragraph 8, wherein Xaa is glutamic acid or aspartic acid.
10. The barley plant or portion of paragraph 9, wherein Xaa is aspartic acid.
11. A transgenic barley plant or portion thereof comprising a heterologous nucleic acid sequence expressing an antisense sequence to at least a portion of a transcribed region of the barley *lox*-1 gene, the heterologous sequence operably linked to a gene promoter sequence and a transcription terminator sequence.
12. The transgenic barley plant or portion of paragraph 11, wherein said gene promoter directs constitutive expression of the antisense sequence.
13. The transgenic barley plant or portion of paragraph 11, wherein said gene promoter directs tissue-specific or temporally-regulated expression of the operably linked antisense sequence in tissues of the plant.
14. The transgenic barley plant or portion of paragraph 11, wherein said tissue is developing, germinating, or mature embryonic tissue.
15. The transgenic barley plant or portion of paragraph 11, wherein said gene promoter sequence is a barley *lox*-1 gene promoter or a portion thereof.
16. Grain or plant progeny produced from the barley plant or plant portion of any of paragraphs 1-15.
17. A plant product produced from the barley plant or plant portion of any of paragraphs 1-15 or from the grain or plant progeny of paragraph 16.
18. The plant product of paragraph 17, wherein the product is malt.
19. A beverage manufactured using the plant product of any of paragraphs 17-18.
20. Beer manufactured using the plant product of any of paragraphs 17-18.
21. The use of a plant product according to any of paragraphs 17-18 for the preparation of a beverage presenting organoleptic qualities that remain stable over a measured period of time or at elevated storage temperatures as compared to a beverage prepared from a plant product of a non-mutated control.
22. Use of a barley plant or portion thereof, grain or plant progeny, or plant product according to any of paragraphs 1-18 for the manufacture of a beverage.
23. Use of a barley plant or portion thereof, grain or plant progeny, or plant product according to any of paragraphs 1-18 for the manufacture of a malt or beer.
24. Use of a barley plant or portion thereof, grain or plant progeny, or plant product according to any of paragraphs 1-18, for the stabilization of organoleptic properties of a brewed product over a measured period of time as compared to a control brewed product produced using a non-mutated barley plant or portion thereof, grain or plant progeny, or plant product.
25. Use of a barley plant or portion thereof, grain or plant progeny, or plant product according to any of paragraphs 1-18 for the manufacture of a brewed product having reduced levels of free *trans*-2-nonenal over a measured period of time or under conditions of elevated storage temperature, as compared to a control brewed product produced using a non-mutated barley plant or portion thereof, grain or plant progeny, or plant product.
26. A beverage produced from a barley plant or portion thereof, grain or plant progeny, or plant product according to any of paragraphs 1-18, the beverage having a reduced *trans*-2-nonenal content over a measured period of time or under conditions of elevated storage temperature as, compared to a control beverage produced from a non-mutated barley plant or portion thereof, grain or plant progeny, or plant product.
27. The beverage of paragraph 26, wherein the beverage is beer.

## Claims

1. A beverage manufactured from a barley plant or portion thereof, wherein the said barley plant or portion thereof comprises a mutant LOX-1 protein encoded by a mutated *lox*-1 nucleic acid sequence having one or more mutations, wherein the LOX-1 activity of said plant or plant portion is reduced as compared to a non-mutated control.

2. The beverage according to claim 1, wherein the beverage is beer.

3. The beverage according to any one of claims 1 and 2, wherein the beverage has a reduced trans-2-nonenal content over a measured period of time or under conditions of elevated storage temperature as compared to a control beverage produced from a non-mutated barley plant or portion thereof.

4. The beverage according to any one of claims 1 to 3, wherein said mutated LOX-1 protein comprises the amino acid sequence shown as SEQ ID NO: 12, wherein Xaa is aspartic acid, glutamic acid, histidine, lysine, arginine, threonine, serine, tyrosine, tryptophan, asparagine or glutamine.

5. A barley plant or portion thereof comprising a mutant LOX-1 protein encoded by a mutated *lox*-1 nucleic acid sequence having one or more mutations, wherein the LOX-1 activity of said plant or plant portion is reduced as compared to a non-mutated control.

6. The barley plant or portion thereof according to claim 5, wherein said mutant LOX-1 protein comprises an acidic, basic or polar amino acid residue linint the substrate cavity of wild-type barley LOX-1.

7. A method for producing a beverage according to any one of claims 1 to 5 from a barley plant or portion thereof, wherein the said barley plant or portion thereof comprises a mutant LOX-1 protein encoded by a mutated *lox*-1 nucleic acid sequence having one or more mutations, wherein the LOX-1 activity of said plant or plant portion is reduced as compared to a non-mutated control.
